(19) Europäisches Patentamt
European Patent Office
Office européen des brevets

(11) EP 2 091 517 B1

(12) **EUROPEAN PATENT SPECIFICATION**

(45) Date of publication and mention of the grant of the patent:
**29.05.2013 Bulletin 2013/22**

(21) Application number: **07862076.2**

(22) Date of filing: **16.11.2007**

(51) Int Cl.:
***A61K 9/127*** *(2006.01)* ***A61K 9/14*** *(2006.01)*
***C08G 18/10*** *(2006.01)* ***C08G 18/32*** *(2006.01)*
***C08G 79/02*** *(2006.01)* ***A61K 31/70*** *(2006.01)*
***A01K 61/00*** *(2006.01)*

(86) International application number:
**PCT/US2007/024075**

(87) International publication number:
**WO 2008/063562 (29.05.2008 Gazette 2008/22)**

(54) **MATERIALS AND METHODS OF INTRODUCING GENETIC MATERIAL INTO LIVING CELLS**

MATERIALIEN UND VERFAHREN ZUR EINFÜGUNG VON GENETISCHEM MATERIAL IN LEBENDE ZELLEN

MATERIAUX ET PROCEDES DESTINES A INTRODUIRE UN MATERIAU GENETIQUE DANS DES CELLULES VIVANTES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **16.11.2006 US 859436 P**
**16.07.2007 US 959731 P**

(43) Date of publication of application:
**26.08.2009 Bulletin 2009/35**

(73) Proprietor: **The University of Akron**
**Akron, OH 44325 (US)**

(72) Inventors:
- **YUN, Yang H.**
  **Uniontown, Ohio 44685 (US)**
- **LOPINA, Stephanie**
  **North Canton, Ohio 44720 (US)**
- **GUPTA, Anirban Sen**
  **Calcutta, West Bengal 700047 (IN)**
- **DITTO, Andrew**
  **Uniontown, Ohio 44685 (US)**
- **SARKAR, Debanjan**
  **Watertown, Massachusetts 02472 (US)**
- **SHAH, Parth**
  **Ellisbridge, Ahmedabad, Gujarat 380006 (IN)**

(74) Representative: **von Kreisler Selting Werner**
**Deichmannhaus am Dom**
**Bahnhofsvorplatz 1**
**50667 Köln (DE)**

(56) References cited:
**US-A- 6 008 318** **US-A1- 2004 156 904**
**US-A1- 2005 037 075** **US-B1- 6 221 997**

- **DES RIEUX A ET AL: "Nanoparticles as potential oral delivery systems of proteins and vaccines: A mechanistic approach" JOURNAL OF CONTROLLED RELEASE, ELSEVIER, AMSTERDAM, NL LNKD- DOI: 10.1016/J.JCONREL.2006.08.013, vol. 116, no. 1, 10 November 2006 (2006-11-10), pages 1-27, XP024957681 ISSN: 0168-3659 [retrieved on 2006-11-10]**
- **GUPTA A S ET AL: "Synthesis and characterization of l-tyrosine based novel polyphosphates for potential biomaterial applications" POLYMER, ELSEVIER SCIENCE PUBLISHERS B.V, GB LNKD- DOI: 10.1016/J.POLYMER.2004.04.052, vol. 45, no. 14, 1 June 2004 (2004-06-01), pages 4653-4662, XP004515392 ISSN: 0032-3861**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 091 517 B1

Printed by Jouve, 75001 PARIS (FR)

(Cont. next page)

- SEN GUPTA A ET AL: "Properties of l-tyrosine based polyphosphates pertinent to potential biomaterial applications" POLYMER, ELSEVIER SCIENCE PUBLISHERS B.V, GB LNKD- DOI: 10.1016/J.POLYMER.2005.05.098, vol. 46, no. 7, 10 March 2005 (2005-03-10) , pages 2133-2140, XP004774422 ISSN: 0032-3861
- Shah, P.: "Blends of L-Tyrosine Based Polyurethanes and Polyphosphate for Biomedical Applications" 14 November 2006 (2006-11-14), XP002600544 Retrieved from the Internet: URL:http://aiche.confex.com/aiche/2006/pre liminaryprogram/abstract_62130.htm [retrieved on 2010-09-14]
- Sarkar, D: "L-Tyrosine Based Polyurethane Blends for Tissue Engineering Applications" 2 March 2005 (2005-03-02), XP002600545 Retrieved from the Internet: URL:http://aiche.confex.com/aiche/2005/pre liminaryprogram/abstract_23097.htm [retrieved on 2010-09-14]

## Description

### FIELD OF THE INVENTION

The present invention generally relates to introducing genetic material to living cells. In some embodiments, the present invention relates to compositions of matter for targeted delivery of nucleic acids to cells.

### BACKGROUND OF THE INVENTION

Previously, non-viral vectors have b>een unable to reach the transfection efficiencies of viruses. Several non-viral vectors have incorporated inactivated virus particles, or fusogenic viral peptides, that lead to improved transfection efficiencies. Unfortunately, immunogenicity is still problematic. Therefore it is also desirable to have non-viral vector that is capable of carrying effective amounts of genetic material, and efficiently transfecting cells while avoiding deleterious side affects such as immune response.

United States Published Patent Application No. 2005/0025820 (hereinafter the ’820 application) is directed to a method and system for systemic delivery of growth arresting, lipid-derived bioactive compounds. More specifically, the ’820 application discloses delivering "gene therapy agents" using a variety of means including microspheres, and nanoparticles. The delivery means set forth in the ’820 application include PEGylated liposomes, and inorganic nanoparticle shells. Furthermore, the ’820 application discloses that the delivery means can be "targeted" to particular kinds of cells by coupling it with targeting moieties. In contrast, the present invention is directed to micro- and/or nano-capsules comprising poly(lactide-co-glycolide), L-tyrosine phosphate, or any combination thereof.

United States Published Patent Application No. 2002/0131995 (hereinafter the ’995 application) is directed to targeted drug delivery with CD44 receptor ligand. More particularly, the ’995 application discloses using a variety of delivery vehicles, including liposomes and microspheres, for delivering drugs to targeted cells and/or tissues. The ’995 application also discloses using such vehicles for delivering a variety of drugs including DNA. Additionally, the ’995 application discloses using hyaluronan or other glucosaminoglycans having an affinity for the CD44 receptor as targeting agents. Although the ’995 application mentions that the delivery vehicle can be a microsphere, it does not disclose how to make such a microsphere, nor does it suggest what materials such a microsphere would comprise. Furthermore, the ’995 application states that liposome embodiments are preferred, and it goes into substantially greater detail regarding how to make and use delivery vehicles made from liposomes.

In contrast, the present invention teaches micro- and/or nano-capsules comprising poly(lactide-co-glycolide), and/or L-tyrosine phosphate. Furthermore, the present invention also includes targeting agents other than hyaluronan as well as optional additives related to biocompatibility and nucleic acid transport, each of which further distinguishes the present invention from the ’995 application.

United States Published Patent Application No. 2005/0037075 (hereinafter the ’075 application) is directed to targeted delivery of controlled release polymer systems. More particularly, the ’075 application discloses polymer systems such as micro and/or nano-spheres made from a variety of polymers. For example, Paragraph [0033] states:

> Examples of suitable polymers for controlled release polymer systems include, but are not limited to... poly(lactic-co-glycolic acid), derivatives of poly(lactic-co-glycolic acid), PEGylated poly(lactic-co-glycolic acid)... poly(ethylene imine), derivatives of poly(ethylene imine), PEGylated poly(ethylene imine)...and combinations thereof. In a preferred embodiment, the controlled release polymer system is a microsphere or a nanosphere.

Additionally, the ’075 application discusses using nucleic acid ligands to target particular kinds of cells. Furthermore, the ’075 application discusses using such targeted micro/nano-spheres for delivering nucleic acids to cells. The present invention includes micro- and/or nano-capsules comprising poly(lactide-co-glycolide) and/or L-tyrosine phosphate, at least one targeting agent, and optionally including PEG-g-chitosan, and/or polyethylenimine. Although the ’075 application discloses using poly(lactide-co-glycolide) as micro and/or nanosphere delivery vehicles, it does not disclose poly(lactide-co-glycolide) in combination with PEG-g-chitosan and/or polyethylenimine. Furthermore, the ’075 application is limited to nucleic acid targeting agents, whereas the present invention includes non-nucleic acid targeting agents. Furthermore, the present invention includes micro- and/or nano-capsules comprising L-tyrosine phosphate, which is not disclosed by the ’075 application.

Thus, there is a need in the art for a non-viral vector that is capable of targeted delivery to selected cells and/or cell types in an organism, efficiently transfecting such cells, and avoiding deleterious side effects. Advantageously, a non-viral vector can mimic a virus’s ability to enter and transfect a cell, and do so without eliciting an immune response or causing the replication of competent viruses. Also advantageously, a non-viral vector can be biodegradable, nontoxic, protect genetic material disposed therein from enzyme degradation, and/or be able to avoid endosome encapsulation. Furthermore, it is desirable to have a vector that does not trigger an immune, coagulation, and/or inflammatory response.

## SUMMARY OF THE INVENTION

The present invention generally relates to introducing genetic material to living cells. In some embodiments, the present invention relates to compositions of matter for targeted delivery of nucleic acids to cells.

In one embodiment, the present invention relates to a composition for targeted delivery of nucleic acids to cells comprising: at least one synthetic polymeric micro- or nano-capsule, wherein the capsule comprises one or more materials selected from poly(lactide-co-glycolide), L-tyrosine polyphosphate, L-tyrosine polyurethane, or any combination thereof; at least one targeting moiety disposed on the surface of the capsule and available for binding to target molecules, wherein the targeting moiety comprises any moiety capable of specifically binding with target molecules such as antibodies, antibody fragments, antigens, transmembrane proteins, glycoproteins, and any combination thereof; an additive for enhancing biocompatibility selected from one or more of PEG-g-chitosan and amphiphilic PEG species; and an additive for assisting in DNA transport across a cell membrane selected from one or more of linear polyethylenimine, PEG-g-chitosan, and any combination thereof.

Preferred embodiments are apparent from the dependent claims.

## BRIEF DESCRIPTION OF THE DRAWINGS

Figure 1 is an illustration of nanospheres formed by sonication emulsion and a hypothesized nanosphere composition;
Figure 2 is an illustration of a hypothesized pDNA-LPEI escape from endosomes;
Figure 3 is a gel electrophoresis of (A) 1 kb DNA ladder; (B) stock pDNA; (C) 1 minute sonicated pDNA-LPEI; and (F through H) stock pDNAZ-LPEI;
Figure 4 is a graph of the mean number of cells in 24 well culture plates transfected with various pDNA-LPEI, BPEI, and PEG-g-CHN complex mass ratios after 3 days;
Figure 5 is a graph of the transfection percentage of human fibroblast cells with pDNA complexed with various mass ratios of polymers;
Figure 6 are photos of transfection of human fibroblast cells from (A) blank cells; (B) 1 minute sonicated pDNA; (C) pDNA with FuGENE 6; (D) PDNA-LPEI; (E) 30 seconds sonicated pDNA-LPEI; (F) 1 minute sonicated pDNA-LPEI;
Figure 7 is a scanning electron microscopy (SEM) of 1% complexed pDNA nanospheres (5000x magnification);
Figure 8 is a scanning electron microscopy (SEM) of 1% complexed pDNA nanospheres (30000x magnification);
Figure 9 is a scanning electron microscopy (SEM) of blank nanospheres (30000x magnification);
Figure 10 is a scanning electron microscopy (SEM) of impeller complexed pDNA nanospheres (30000x magnification);
Figure 11 is a scanning electron microscopy (SEM) of 10% complexed pDNA nanospheres (30000x magnification);
Figure 12 is a graph illustrating representative size distribution of blank pDNA nanospheres determined using regularized non-negatively constrained least squares method;
Figure 13 is a graph illustrating representative size distribution of 1% complexed pDNA nanospheres determined using regularized non-negatively constrained least squares method;
Figure 14 is a graph illustrating representative size distribution of impeller formed 1% complexed pDNA nanospheres determined using regularized non-negatively constrained least squares method;
Figure 15 is a graph illustrating representative size distribution of 10% complexed pDNA nanospheres determined using regularized non-negatively constrained least squares method;
Figure 16 is a degradation of blank nanospheres mean diameter using a regularized non-negatively constrained least squares method;
Figure 17 is a degradation of 1% complexed pDNA nanospheres mean diameter using a regularized non-negatively constrained least squares method;
Figure 18 is a degradation of impeller formed 1% complexed pDNA nanospheres mean diameter using a regularized non-negatively constrained least squares method;
Figure 19 is a degradation of 10% complexed pDNA nanospheres mean diameter using a regularized non-negatively constrained least squares method;
Figure 20 is a set of images of human dermal fibroblasts exposed to 200 $\mu$L of buffers with (A) showing dead cells; (B) showing live and metabolically active cells; and (C) combined fluorescence channels;
Figure 21 is a set of images of human dermal fibroblasts exposed to 4 $\mu$g of pDNA with (A) showing dead cells; (B) showing live and metabolically active cells; and (C) combined fluorescence channels with a dead cell being circled;
Figure 22 is a set of images of human dermal fibroblasts exposed to 2 mM $H_2O_2$ with (A) showing dead cells; (B) showing live and metabolically active cells; and (C) combined fluorescence channels;
Figure 23 is a set of images of human dermal fibroblasts exposed to 4 $\mu$g of pDNA with 12 $\mu$L of FuGENE 6 with

(A) showing dead cells; (B) showing live and metabolically active cells; and (C) combined fluorescence channels with dead cells being circled;

Figure 24 is a set of images of human dermal fibroblasts exposed to 4 μg of pDNA complexed with 4 μg of LPEI with (A) showing dead cells; (B) showing live and metabolically active cells; and (C) combined fluorescence channels with dead cells being circled;

Figure 25 is a set of images of human dermal fibroblasts exposed to 400 μg of 1% complexed pDNA nanospheres with (A) showing dead cells; (B) showing live and metabolically active cells; and (C) combined fluorescence channels with dead cells being circled;

Figure 26 is a set of images of human dermal fibroblasts exposed to 400 μg of PLGA nanospheres with (A) showing dead cells; (B) showing live and metabolically active cells; and (C) combined fluorescence channels with dead cells being circled;

Figure 27 is a set of images of human dermal fibroblasts exposed to 400 μg of impeller formed 1% complexed pDNA nanospheres with (A) showing dead cells; (B) showing live and metabolically active cells; and (C) combined fluorescence channels with dead cells being circled;

Figure 28 is a set of images of human dermal fibroblasts exposed to 100 μg of 10% complexed pDNA nanospheres with (A) showing dead cells; (B) showing live and metabolically active cells; and (C) combined fluorescence channels;

Figure 29 is a graph illustrating cell viability of various green vectors determined by LIVE/DEAD cell assay;

Figure 30 is a graph illustrating the loading efficiency of various nanospheres formulations;

Figure 31 is an image of gel electrophoresis of (A) 1 kb DNA ladder, blank nanosphere release after (B) 1 day; (C) 2 days; (C) 3 days (E) 4 days; (F) 5 days; (G) 6 days; and (H) 7 days;

Figure 32 is an image of gel electrophoresis of (A) 1 kb DNA ladder; (B) stock pDNA; (C) stock pDNA-LPEI, 1% complexed pDNA nanosphere release after (D) 0.5 hours; (E) 1.5 hours; (F) 3.0 hours; (G) 6.0 hours; (H) 12.0 hours; (I) 24 hours; (J) 2 days; (K) 3 days; (L) 4 days; (M) 5 days; (N) 6 days; and (O) 7 days;

Figure 33 is an image of gel electrophoresis of (A) 1 kb DNA ladder; (B) stock pDNA; (C) stock pDNA-LPEI, 10% complexed pDNA nanosphere release after (D) 0.5 hours; (E) 1.5 hours; (F) 6.0 hours; (G) 12.0 hours; (H) 24.0 hours; (I) 2 days; (J) 3 days; (K) 4 days; (L) 6 days: (M) 7 days;

Figure 34 is an image of gel electrophoresis of (A) 1 kb DNA ladder; (B) stock pDNA; (C) stock pDNA-LPEI, impeller formed 1% complexed pDNA nanosphere release after (D) 0.5 hours; (E) 1.5 hours; (F) 3.0 hours; (G) 6.0 hours; (H) 12.0 hours; (I) 24 hours; (J) 2 days; (K) 3 days; (L) 4 days; (M) 5 days; (N) 6 days: (O) 7 days;

Figure 35 is an image of gel electrophoresis of (A) 1 kb DNA ladder: (B) stock pDNA, non-complexed pDNA nanosphere release after (C) day 1; (D) day 2; (E) day 3;

Figure 36 are images of representative transfection of primary human dermal fibroblasts obtained by day 2 release from (A) blank nanospheres; (B) 1% DNA nanospheres; (C) 10% pDNA nanospheres; (D) Impeller formed 1% pDNA nanospheres;

Figure 37 is a graph illustrating the cumulative release of pDNA-LPEI based on transfection percentage of primary human dermal fibroblasts;

Figure 38 is a set of images of (A) FITC labeled nanospheres; (B) DAPI stained nuclei; (C) rhodamine phalloidin stained cytoskeleton; and (D) suggested human fibroblast cellular uptake of FITC labeled nanospheres;

Figure 39 is a set of images of (A) FITC fluorescence filter on human fibroblasts; (B) DAPI stained nuclei; (C) rhodamine phalloidin stained cytoskeleton; (D) human fibroblasts without addition of FITC labeled nanospheres;

Figure 40 is a set of images from a confocal microscopy of fibroblast uptake of FITC labeled nanospheres;

Figure 41 is a set of images from day 3 of human dermal fibroblasts transfected with: (A) 4 μg pDNA; (B) 4 μg of pDNA with 12 μL of FuGENE 6 transfection reagent; (C) 4 μg of pDNA complexed with 4 μg of LPEI; (D) 400 μg of 1% complexed pDNA nanospheres;

Figure 42 is a set of images from day 5 of human dermal fibroblasts transfected with: (A) 4 μg pDNA; (B) 4 μg of pDNA with 12 μL of FuGENE 6 transfection reagent; (C) 4 μg of pDNA complexed with 4 μg of LPEI; (D) 400 μg of 1% complexed pDNA nanospheres;

Figure- 43 is a set of images from day 7 of human dermal fibroblasts transfected with: (A) 4 μg pDNA; (B) 4 μg of pDNA with 12 μL of FuGENE 6 transfection reagent; (C) 4 μg of pDNA complexed with 4 μg of LPEI; (D) 400 μg of 1% complexed pDNA nanospheres;

Figure 44 is a set of images from day 9 of human dermal fibroblasts transfected with: (A) 4 μg pDNA; (B) 4 μg of pDNA with 12 μL of FuGENE 6 transfection reagent; (C) 4 μg of pDNA complexed with 4 μg of LPEI; (D) 400 μg of 1% complexed pDNA nanospheres;

Figure 45 is a set of images from day 11 of human dermal fibroblasts transfected with: (A) 4 μg pDNA; (B) 4 μg of pDNA with 12 μL of FuGENE 6 transfection reagent; (C) 4 μg of pDNA complexed with 4 μg of LPEI; (D) 400 μg of 1 % complexed pDNA nanospheres;

Figure 46 is a graph illustrating the comparative transfection efficiency of pDNA-LPEI versus 1% pDNA-LPEI nanospheres over 11 days;

Figure 47 is a graph illustrating the comparative transfection efficiency of FuGENE 6 versus 1% pDNA-LPEI nanospheres over 11 days;
Figure 48 is a graph illustrating the transfection efficiency profile of 1% pDNA-LPEI nanospheres versus pDNA-LPEI and pDNA-FuGENE 6 over 11 days;
Figure 49 is a graph illustrating the cumulative transfection efficiency profile of 1% pDNA-LPEI nanospheres versus pDNA-LPEI and pDNA-FuGENE 6 over 11 days;
Figure 50 is a bright field image of human dermal fibroblast expressing lacZ gene;
Figure 51 is a $^{1}H$ NMR image of PEG-HDI-DTH;
Figure 52 is a $^{1}H$ NMR image of PCL-HDI-DTH;
Figure 53 is a $^{13}C$ NMR image of PEG-HDI-DTH;
Figure 54 is a $^{13}C$ NMR image of PCL-HDI-DTH;
Figure 55 is a set of FT-IR traces of L-tyrosine-based polyurethanes formed in accordance with the present invention;
Figure 56 is a set of FT-IR traces of the components, pre-polymer and polyurethanes in accordance with the present invention;
Figure 57 is a set of DSC heating curves of L-tyrosine-based polyurethanes formed in accordance with the present invention;
Figure 58 is a TGA analysis of L-tyrosine-based polyurethanes formed in accordance with the present invention; and
Figure 59 is a stress-strain curve for L-tyrosine-based polyurethanes formed in accordance with the present invention.

## DETAILED DESCRIPTION OF THE INVENTION

The present invention generally relates to introducing genetic material to living cells. In some embodiments, the present invention relates to compositions of matter for targeted delivery of nucleic acids to cells.

The present disclosure generally relates to methods and materials for targeted delivery of genetic material to eukaryotic cells. Some embodiments of the present invention include nanospheres formulated from a polymer blend of L-tyrosine polyphosphate (LTP), polyethylene glycol grafted to chitosan (PEG-g-CHN), and plasmid DNA (pDNA) complexed with linear 2 polyethylenimine (LPEI) as non-viral gene delivery vector. Thus, some embodiments are capable of mimicking a virus by being taken up by, for example, mammalian cells, escaping endosome entrapment, protecting genetic material disposed therein from enzyme degradation, and/or efficiently transfecting cells *in vitro.*

Sustained Release from Non-Viral Vectors:

Non-viral vectors need to exhibit a sustained and controlled release in order to decrease the number of doses and maintain optimum dosage levels. The rate at which the vector degrades determines the release kinetics and duration of the release. Sustained DNA release can prolong exogenous gene expression, thereby reducing the need for repeated dosing, which is a significant advantage for long-term gene therapy. Previous studies used hydrogels, polymer matrices, and microspheres to obtain a sustained controlled release. Microspheres formulated from hyaluronan exhibit a sustained release of pDNA over a couple of months, which is desirable for long term therapies. However, aggressive short term gene deliveries may be necessary for suicide gene therapy related to cancer treatment. Thus, more aggressive therapies would benefit from a polymer vector that degrades and produces a sustained release over several days.

L-tyrosine Polyphosphate Degradation:

The formulation of a non-viral vector with L-tyrosine polyphosphate (LTP) results in a vector that exhibits a sustained release over 7 days. LTP would be an ideal gene-vector for short term therapies. LTP is a biodegradable peptide polyphosphate that is synthesized from the natural amino acid L-tyrosine. LTP hydrolytically biodegrades at the phosphoester linkage and enzymatically degrades at the peptide linkage in the polymer backbone into L-tyrosine based derivatives and hence is suitable for biomaterial applications. The degradation products are nontoxic phosphates and L-tyrosine. Furthermore, the degradation products of LTP have negligible effect on local pH, which is unlike other biomaterials such as poly[DL-lactide-co-glycolide] (PLGA). L-tyrosine polyphosphate is soluble in a variety of common organic solvents and thus can therefore be processed into microsphere or nanosphere formulations.

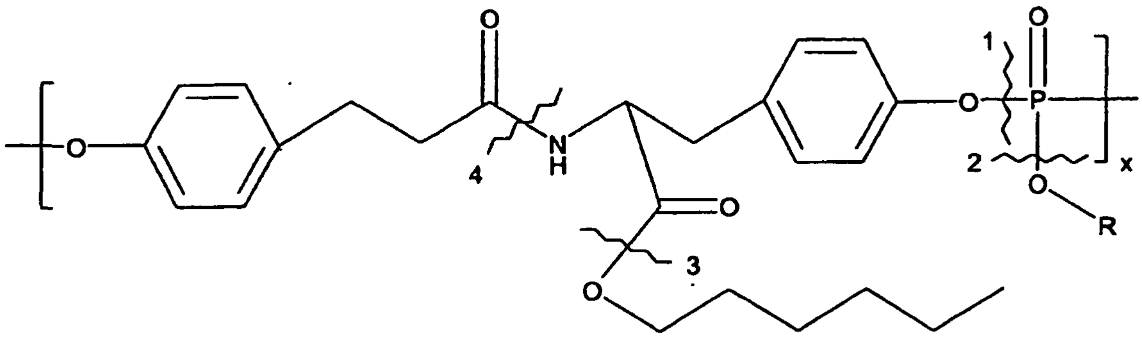

wherein the above formula represents the chemical structure of L-tyrosine polyphosphate and where (1) through (4) represent the degradation sites thereof, where (1) is the backbone phosphoester bond - hydrolysis; (2) is the pendant phosphoester bond - hydrolysis; (3) is the pendant alkyl (hexyl) ester bond - hydrolysis; and (4) is the backbone amide (peptide) bond - enzymolysis and where x is an integer in the range of 10 to 80, or an integer from 15 to 75, or an integer from 20 to 70, or an integer from 25 to 60, or an integer from 30 to 50, or even an integer from 35 to 45. Here, as well elsewhere in the specification and claims, individual range limits can be combined to form additional non-disclosed ranges.

In another embodiment, the number of repeating units, x, in the above formula are selected so that the molecular weigh of the above L-tyrosine polyphosphate before degradation is approximately in the range of 5,000 Daltons (Da) to 40,000 Da, or from 7,500 Da to 30,000 Da, or from 10,000 Da to 25,000 Da, or even from 15,000 Da to 20,000 Da. Here, as well elsewhere in the specification and claims, individual range limits can be combined to form additional non-disclosed ranges. In another embodiment, the number of repeating units, x, is selected so that the molecular weight of the above L-tyrosine polyphosphate is 11,000 Da.

**Nanospheres as Non-Viral Vectors:**

Biodegradable nanospheres hold a unique advantage over the other non-viral vectors, since they are comparable in scale to viruses. Viruses range in size from tens to hundreds of nanometers. Previous studies show that particles with radii smaller than 50 nm exhibit significantly greater uptake by endocytosis or pinocytosis compared to particles larger than 50 nm with an optimal size around 25 nm. Some cell receptors can facilitate vector uptake into the cytoplasm directly across the plasma membrane, but the most common route for receptor-mediated uptake of macromolecular moieties is by endocytosis. Thus, endocytosis is an attractive mechanism for targeted gene delivery, which can be achieved by nanospheres. Also similar to viruses, once nanospheres are endocytosed they can provide an intracellular pDNA delivery that will avoid enzyme degradation in the circulation.

Nanospheres Encapsulating pDNA:

The most common method for encapsulating proteins or plasmids in nanospheres is an emulsion created by sonication (Figure 1). During the emulsification, thermodynamics favor hydrophilic pDNA being entrapped in an inner water phase inside an oil phase. The resulting nanospheres are formed after extracting the solvent by evaporation. Several problems such as poor loading, burst release, emulsion instability, and loss of bioactivity arise when formulating nanospheres for pDNA delivery. Poor entrapment efficiency can occur due to an instable emulsion, or when large amount of the pDNA collects in the outer water phase of the emulsion. Likewise, burst release can result when the pDNA in the outer water phase adheres to the surface of the nanospheres during solvent evaporation. Sonication can shear most of the pDNA such that it is no longer bioactive. These problems can be alleviated by blending cationic polymers complexed to pDNA with amphiphilic polymers that prevent shearing and stabilize the emulsion.

Role of Chitosan Grafted with Polyethylene Glycol in Nanospheres:

Blending chitosan grafted with polyethylene glycol (PEG-g-CHN) into the nanospheres not only stabilizes the emulsion in order to increase nanosphere yield, but also enhances the biocompatibility. Several studies show PEG-g-CHN forms complexes with DNA and can be used a gene vector. Chitosan itself is shown to form complexes with pDNA and improve transfection efficiency. Furthermore, chitosan is approved by the FDA as a food additive and considered to be nontoxic. However, chitosan is limited as a gene vector since the crystalline structure of chitosan with its intra- and inter-molecular hydrogen bonds inhibits its solubility in organic solvents or aqueous solutions at physiological pH.. By grafting polyethylene glycol (PEG) to chitosan through hydrogen bonding, an amphiphilic polymer is formed that is soluble

in both dimethyl sulphoxide (DMSO) and acidic aqueous solutions. When formulating nanospheres with PEG-g-CHN (Figure 1) by the emulsion method, thermodynamics favor hydrophilic PEG enrichment between the oil and outer water phase. Thus, during solvent evaporation, PEG is hypothesized to become enriched at the surface of the nanospheres. PEG on the surface on the nanospheres is ideal, since PEG prevents plasma protein adsorption, platelet adhesion, and thrombus formation by the steric repulsion mechanism. Nanospheres with PEG at the surface could be stealth to the coagulation, immune, and inflammatory systems. Thermodynamics also favor chitosan to be enriched with LTP in the oil phase.

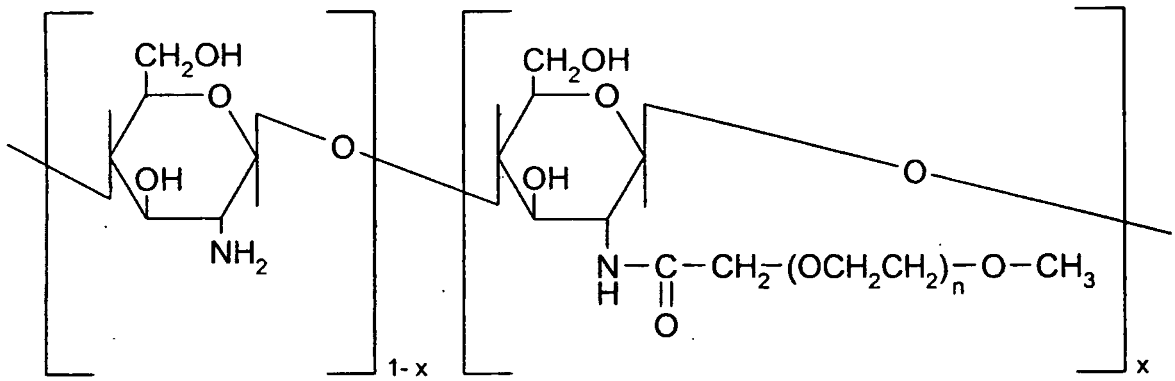

Chemical Structure of PEG-g-CHN

The PEG-g-CHN, the generic structure of which is illustrated above, was purchased from CarboMer, Inc (Catalog No. 7-00105).

Role of Linear Polyethylenimine in Nanospheres:

Incorporating the cationic polymer, linear polyethylenimine (LPEI), in the nanosphere formulation serves two major purposes. First, LPEI condenses pDNA, which prevents shearing of the pDNA during sonication. This prevention of shearing has been verified by preliminary research that revealed sonicated complexed pDNA with LPEI to be bioactive and intact. Condensation occurs due to the charge attractions between the positively charged LPEI and negatively charged pDNA. The N/P ratio is a measure of the ionic balance of the pDNA-LPEI complexes. The positive charge of LPEI originates from the nitrogen of the repeat unit of LPEI, $NHCH_2CH_2$, which has a molecular weight of 43 g/mol. The negative charge in the plasmid DNA backbone arises from the phosphate group of the deoxyribose nucleotides. The average molecular weight of the nucleotides is assumed to be 330 g/mol. Hence, a complex of a 1 mg of pDNA to 1 mg of LPEI is an N/P ratio of 7.7. Fortunately, pDNA-LPEI complexes are hydrophilic, so thermodynamics will favor their encapsulation in the inner water phase of the nanospheres during emulsification (Figure 1). Therefore, as the LTP of the nanosphere degrades, the pDNA-LPEI complexes can be released.

The second purpose of LPEI is its dramatic increase of vector transfection efficiency. Direct release of non-complexed pDNA in extracellular fluid or cytoplasm has poor transfection efficiency due to its inability to bind and pass through cellular membranes, escape entrapment in endosomes, and lysosomal degradation. The cellular membrane, like pDNA, has a negative charge. Thus, pDNA will repel from cellular membranes and is not likely to be endocytosed. Complexing the pDNA with positive cationic polymers like branched polyethylenimine (BPEI) and LPEI will help neutralize its charge. Both BPEI and LPEI condense DNA into complexes that are 50 to 100 nm in diameter, which is a particle size that can be endocytosed. These complexes are formed in low ionic strength solutions in the attempt to control the overall size of the complexes. Smaller complexes appear to have higher transfection efficiency and less toxicity than larger complexes. Numerous studies reveal that LPEI 25 kDa has a higher transfection efficiency and lower cytotoxicity than BPEI.

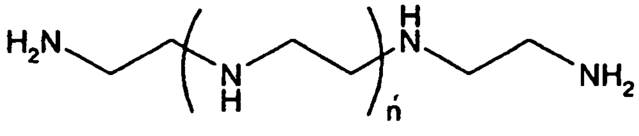

Chemical Structure of Linear Polyethylenimine

In the above formula, the number of repeating units, n, is selected so that the molecular weight of the above linear polyethylenimine composition is in the range of 5,000 Da to 50,000 Da, or from 10,000 Da to 40,000 Da, or from 15,000 Da to 30,000 Da, or even from 20,000 Da to 25,000 Da. Here, as well elsewhere in the specification and claims, individual range limits can be combined to form additional non-disclosed ranges. In another embodiment, n is selected so that the molecular weight of the above linear polyethylenimine composition is 25,000 Da.

The exact mechanism of transfection enhancement by condensing pDNA with LPEI is unknown. However, LPEI is suspected to be able to escape entrapment by endosomes and eventual lysosome degradation. On the other hand, pDNA-LPEI is hypothesized to escape from endosomal entrapment by the "proton sponge theory" (Figure 2). In the proton sponge theory, the integrated amino groups in the backbone structure of LPEI possess a low pKa that shows a buffering property below physiological pH. Thus, LPEI in the endosome interferes with pH lowering of the compartment, and induces an increased ion osmotic pressure to cause endosomal swelling and subsequent disruption. Consequently, the endocytosed pDNA-LPEI complex can be efficiently delivered to the nucleus. The mechanism by which the pDNA-LPEI complex enters the nucleus and is expressed is unknown. LPEI and pDNA are believed to dissociate in order to achieve transfection. Other theories believe that vector complexes can only enter the nucleus during mitosis. Others postulate that complexes or pDNA diffuse their way through nuclear pores that have a mean diameter of 25 nm.

Methods:

Successful gene therapy with a non-viral vector can be accomplished by overcoming the barriers such as: passing through the cell membrane, escaping from endosomes, protecting the DNA from enzyme degradation and shearing, and efficient transfection. These barriers will be overcome by nanospheres formulated from a polymer blend of L-tyrosine polyphosphate (LTP), polyethylene glycol grafted to chitosan (PEG-g-CHN), and plasmid DNA (pDNA) complexed with linear polyethylenimine (LPEI).

Agarose Gel Electrophoresis Assay of Sonicated pDNA-LPEI Complex:

Sonication during the emulsion step of nanosphere synthesis was known to shear and destroy pDNA. Therefore, plasmid DNA (pDNA) needed to be condensed with PEG-g-CHN, LPEI, or BPEI. To determine whether complexing pDNA with LPEI protected the pDNA from shearing during the sonication of nanosphere synthesis, an agrose gel electrophoresis assay was performed. First, all water used in the following experiments was distilled and de-ionized (Barnstead NanoPure II) and autoclaved (American Standard 25X-1) to inactivate DNAase. Next, PEF1-V5 (Invitrogen) plasmid DNA was propagated using a QIAGEN plasmid purification kit. LPEI (PolyScience Inc.) with a molecular weight of 25,000 Daltons was dissolved in $dH_2O$ at 70°C at a concentration of 1 mg/ml. Then, 1:1 mass ratio of pDNA-LPEI (20 μg/ml pDNA and 20 μg/ml LPEI) samples were condensed for 45 minutes at 37°C in 500 μL of autoclaved distilled and de-ionized $H_2O$ ($dH_2O$). These samples were prepared in triplicate for both 30 second and 1 minute sonication times. Next, the sonicator tip (Branson 102C CE) was placed in the pDNA-LPEI samples and sonicated for either 30 seconds or 1 minute. Afterwards, 30 μL was taken from each sonicated sample and mixed with 6 μL of 6x dye (Sigma-Aldrich) then loaded into a 0.7% agarose gel containing ethidium bromide (0.5 μg/ml, Fisher Scientific).

Transfection Efficiency and Cytotoxicity of pDNA-Polymer Complexes:

Complexing pDNA with LPEI and BPEI have been shown to greatly enhance cellular transfection; however they were toxic at high dosages. Previous studies have shown that transfection increased with increasing the N/P ratio between pDNA and BPEI or LPEI. Therefore, the optimization of transfection efficiency with low toxicity was necessary. In order to compare transfection efficiency and cell viability of various sonicated and un-sonicated pDNA-polymer complexes, an X-Gal transfection assay was performed. First, PEG-g-CHN (CarboMer Inc.) with 80% acetylation was dissolved at a concentration of 3.33 mg/ml in 0.1 N acetic acid for 48 hours at 37°C under rotation. LPEI (PolyScience Inc.) with a molecular weight of 25,000 Daltons was dissolved in $dH_2O$ at 70°C for 15 minutes at a concentration of 1 mg/ml. BPEI (PolyScience Inc.) with a molecular weight of 50,000 Daltons was prepared as a 30% aqueous solution. Then, pDNA-LPEI mass ratios of 1:1, 1:2, 1:4 and 1:8; pDNA-BPEI mass ratios of 1:1, 1:2, and 1:8; pDNA-PEG-g-CHN mass ratios of 1:1 and 1:10 were condensed for 45 minutes at 37°C in 500 μL of $dH_2O$ with a pDNA concentration of 20 μg/ml. Next, primary human dermal fibroblasts (a gift from Judy Fulton at the Kenneth Calhoun Research Center, Akron General Medical Center) were seeded onto well tissue culture plates at a density of 25,333 cells/well and maintained overnight at 37°C with fibroblast feeding media (90% Dulbecco's Modified Eagle Medium and 10% Fetal Calf Serum containing 1% Antimycotic). The next day, the fibroblast feeding media was replaced.

Next, 200 μL samples (4 μg of pDNA per sample) of pDNA-LPEI complex and of sonicated pDNA-LPEI complex were added to each well. Stock pDNA and TE buffer were used as negative controls. The positive controls were pDNA conjugated FuGENE 6 (Roche, Indianapolis, IN), a commercial transfection reagent. FuGENE 6 was prepared by incu-

bating 4 μg of pDNA (40 μL) with 12 μL of FuGENE 6 and 148 μL of DMEM for 15 minutes. The cells were incubated for 72 hours at 37°C. Next, the cells were washed with phosphate buffer saline (PBS), fixed with 1% formaldehyde and an X-Gal staining assay was used to determine transfection. Five random photos were taken. The cells that appeared blue were successfully transfected and were expressing the β-gal enzyme. Transfection percentage was calculated by dividing the number of transfected (blue) cells by the total number of cells.

Nanosphere Synthesis:

Nanospheres formulated with LTP, PEG-g-CHN, and pDNA-LPEI were prepared using an emulsion of water and oil by sonication and solvent evaporation technique (See Table 1 below). For each nanosphere formulation, PEG-g-CHN was dissolved at a 3.33 mg/ml concentration in 0.1 N acetic acid for 48 hours at 37°C. LTP was synthesized according to the protocol established by Gupta and Lopina. LTP was dissolved in chloroform at a concentration of 100 mg/ml. Next, a 5% polyvinyl pyrrolidone (PVP) in $dH_2O$ was prepared. LPEI was dissolved in $dH_2O$ for 15 minutes at 70°C at 3, 10 or 15 mg/ml. Then, for pDNA-LPEI complex loaded nanospheres, the pDNA and LPEI were complexed in $dH_2O$ for 45 minutes at concentrations of 0.3, 1.0, or 3.0 mg/ml each. Next, nanosphere formulations shown in below were emulsified by a sonicator (Branson 102C CE) for 1 minute. Nanosphere synthesis was performed in 6 replicates. A nanosphere batch with 10% loaded pDNA complexed with an equal amount of LPEI was also formulated (Table 2). Blank nanospheres and blank PLGA nanospheres were formulated as negative controls (Table 2). An additional batch of nanospheres was produced using a water-in-oil-in-water emulsion formed by an impeller (Yamato Lab-Stirrer LR400D).

Table 1 - Complexes of pDNA and LPEI for Various Nanosphere Formulations

| Polymer | Initial Concentration (mg/mL) | Volume (mL) | Mass (mg) | Final Concentration (mg/mL) |
|---|---|---|---|---|
| Sonicator Formed 1% Complexed pDNA Nanospheres | | | | |
| pDNA | 3.0 | 1.00 | 3.0 | 0.3 |
| LPEI | 3.0 | 1.00 | 3.0 | 0.3 |
| $dH_2O$ | - | 8.00 | - | - |
| Total | - | 10.00 | - | - |
| Impeller Formed 1% Complexed pDNA Nanospheres | | | | |
| pDNA | 15.0 | 0.20 | 3.0 | 3.0 |
| LPEI | 15.0 | 0.20 | 3.0 | 3.0 |
| $dH_2O$ | - | 0.60 | - | - |
| Total | - | 1.00 | - | - |
| Sonicator Formed 10% Complexed pDNA Nanospheres | | | | |
| pDNA | 10.0 | 1.00 | 10.0 | 1.0 |
| LPEI | 10.0 | 1.00 | 10.0 | 1.0 |
| $dH_2O$ | - | 8.00 | - | - |
| Total | - | 10.00 | - | - |

Table 2 - Nanosphere Formulations

| Polymer | Concentration (mg/mL) | Volume (mL) | Mass (mg) | Mass (%) | Volume (%) |
|---|---|---|---|---|---|
| Sonicator Formed 1% Complexed pDNA Nanospheres | | | | | |
| LTP | 100.0 | 2.91 | 291.0 | 97.0 | 2.8 |
| pDNA-LPEI | 0.6 | 10.00 | 6.0 | 2.0 | 9.6 |
| PEG-g-CHN | 3.3 | 0.90 | 3.0 | 1.0 | 0.9 |
| 5% PVP | - | 90.00 | - | - | 86.7 |
| Total | | 103.81 | 300.0 | | |

(continued)

| | | | | | |
|---|---|---|---|---|---|
| Impeller Formed 1% Complexed pDNA Nanospheres | | | | | |
| LTP | 100.0 | 2.91 | 291.0 | 97.0 | 2.8 |
| pDNA-LPEI | 6.0 | 1.00 | 6.0 | 2.0 | 0.9 |
| PEG-g-CHN | 3.3 | 0.90 | 3.0 | 1.0 | 0.9 |
| 5% PVP | - | 100.00 | - | | 95.4 |
| Total | | 104.81 | 300.0 | | |
| Sonicator Formed 10% Complexed pDNA Nanospheres | | | | | |
| LTP | 100.0 | 0.79 | 79.0 | 79.0 | 0.8 |
| pDNA-LPEI | 2.0 | 10.00 | 20.0 | 20.0 | 9.9 |
| PEG-g-CHN | 3.3 | 0.30 | 1.0 | 1.0 | 0.3 |
| 5% PVP | - | 90.00 | - | | 89.0 |
| Total | | 101.09 | 100.0 | | - |
| Sonicator Formed 1% Non-Complexed pDNA Nanospheres | | | | | |
| LTP | 100.0 | 2.91 | 291.0 | 97.0 | 2.8 |
| pDNA | 3.7 | 0.80 | 3.0 | 1.0 | 0.8 |
| LPEI | 15.0 | 0.20 | 3.0 | 1.0 | 0.2 |
| PEG-g-CHN | 3.3 | 0.90 | 3.0 | 1.0 | 0.8 |
| 5% PVP | - | 100.00 | - | - | 95.4 |
| Total | | 104.81 | 300.0 | | |
| Sonicator Formed Blank Nanospheres | | | | | |
| LTP | 100.0 | 2.94 | 294.0 | 97.0 | 2.8 |
| LPEI | 3.0 | 1.00 | 3.0 | 2.0 | 0.9 |
| PEG-g-CHN | 3.3 | 0.90 | 3.0 | 1.0 | 0.9 |
| 5% PVP | - | 100.00 | - | - | 95.4 |
| Total | | 104.84 | 300.0 | | |

Next, the chloroform was allowed to evaporate for 5 hours while the emulsion was gently stirred. The nanospheres were then collected by centrifugation at 15,000xg for 15 minutes. Afterward, the nanospheres were washed 3 times by centrifugation at 15,000xg for 15 minutes with autoclaved $dH_2O$. The nanospheres were then shell frozen in 10 ml of $dH_2O$, and were placed in a lyophilizer (Labconco Freezone 4.5) for 72 hours. Finally, the lyophilized nanospheres were stored in a desiccator.

Characterize the Size, Shape, Morphology, Degradation, and Cytotoxicity of pDNA-LPEI Loaded Nanospheres:

Rational: Intracellular delivery of pDNA to the nucleus should increase transfection efficiency by avoiding enzyme degradation in the circulation. The nanospheres must be endocytosed to accomplish intracellular delivery. In order to be endocytosed, the nanospheres must mimic the nanoscale of a virus. Therefore, verification was necessary to show that 1% loading of complexed pDNA in the nanospheres did not affect their size, shape, and morphology compared to blank nanospheres in the preliminary research. Furthermore, measuring nanosphere diameter as it degrades, characterized the degradation profile. In addition, fibroblasts exposed to nanospheres must have comparable cell viability to fibroblasts exposed to PLGA nanospheres and unexposed fibroblasts. The hypothesis was that SEM and laser light scattering would show that 1% complexed pDNA loaded nanospheres were comparable in size, shape, and morphology to blank nanospheres in the preliminary research. Furthermore, laser light scattering would show that nanospheres were completely degraded after 7 days based on previous research. Live/dead cell assay would show that fibroblasts exposed to 1% complexed pDNA nanospheres were comparable cell viability to unexposed fibroblasts.

Scanning Electron Microscopy of Nanospheres:

Scanning electron microscopy (SEM, Hitachi S2150) was used in order to qualitatively compare the size, shape,

and morphology of 1% complexed pDNA loaded nanospheres to the blank and PLGA nanospheres. First, 1 mg of nanospheres was suspended in 1 ml of distilled and de-ionized $H_2O$. Then, 200 μL of the suspended microspheres were pipetted onto a stub, dehydrated, sputter coated with silver/palladium, and examined. Blank nanospheres, 1% non-complexed nanospheres, 10% complexed pDNA nanospheres, impeller formed 1% complexed pDNA nanospheres, and PLGA nanospheres were used as controls.

Laser Light Scattering of Nanospheres:

Dynamic laser light scattering was used as an additional method for comparing the size of the 1% complexed pDNA loaded nanospheres to the blank nanospheres. The nanosphere sample was prepared by suspending 1 mg of nanospheres in 10 ml of PBS that had been passed through a 0.2 μm filter. The suspended nanospheres were centrifuged for 10 seconds at 1000xg to remove any large aggregates. Then, the sample was decanted into a glass scintillation vial. A dynamic laser light scattering system (Brookhaven Instruments BI- 200SM) calculated the nanosphere diameter by the Regularized Non-negatively Constrained Least Squares (CONTIN) method. The range of nanosphere size was reported as differential distribution values. The differential distribution value varied from 0 to 100, not percent, just 100. The highest peak or modal value was assigned to the number 100. For example, if the diameter of 150 nm had the differential distribution value of 37 and at the diameter of 200 nm the differential distribution value was 74, then, the distribution had twice the amount at 200 nm than it did at 150 nm. The differential distribution values were the relative amount at the corresponding diameter. Blank nanospheres, 1% non-complexed nanospheres, 10% complexed pDNA nanospheres, impeller formed 1% and blank PLGA nanospheres will be used controls.

Degradation of Nanospheres:

To quantify the release duration and the degradation of the nanospheres in vitro, laser light scattering was utilized. Light scattering samples and the procedure was performed according to the procedure described herein. Then, the nanospheres were incubated at 37°C and slightly shaken for 11 days. On days 0, 1, 2, 3, 4, 7, and 11, laser light scattering was performed in order to measure nanosphere diameter. The mean diameter of nanospheres was calculated by the Brookhaven software and reported for each day. Blank nanospheres, 1% non-complexed nanospheres, and blank PLGA nanospheres were used controls.

Cell Viability after Exposure to Nanospheres:

The cell viability of primary human dermal fibroblasts after exposure to 1% complexed pDNA nanospheres was determined using a live/dead cell assay (Invitrogen). First, primary human dermal fibroblasts (a gift from Judy Fulton at the Kenneth Calhoun Research Center, Akron General Medical Center) were seeded onto 24 well tissue culture plates at a density of 25,333 cells/well and maintained overnight at 37°C with fibroblast feeding media (90% Dulbecco's Modified Eagle Medium and 10% Fetal Calf Serum containing 1% Antimycotic). The next day, the fibroblast feeding media was replaced. Fibroblasts were exposed to 400 μg of nanospheres. After 1, 3, 7, and 11 days a live/dead cell assay was performed according to the manufacturer's instructions. Blank nanospheres, pDNA-LPEI, pDNA-FuGENE 6, 10% pDNA nanospheres, impeller formed 1% complexed pDNA nanospheres, and blank PLGA nanospheres were used controls.

Quantify and Characterize Nanosphere Loading and Release of pDNA-LPEI:

Rationale: A sustained release of complexed pDNA would decrease the number of dosages. In order to treat short term gene therapies, a sustained release of intact complexed pDNA for approximately 7 days was desired. Therefore, the release of complexed pDNA over 7 days was characterized, quantified, and structurally examined. The hypothesis was that a sustained release would be observed over a course of at least 3 days based off of preliminary research. Furthermore, a significant difference was expected between the transfection efficiency of the release samples and of the negative controls based on preliminary results. AFM was hypothesized to reveal intact pDNA-LPEI complexes released from the 1% complexed pDNA nanospheres. The released pDNA-LPEI complexes would not be significantly different in size to the stock pDNA-LPEI complex.

Loading Efficiency of pDNA in Nanospheres:

The loading efficiency of the various nanosphere formulations was performed using a PicoGreen® (Molecular Probes) fluorescence assay. The loading of all formulations of pDNA nanospheres was determined by dissolving 2 mg of nanospheres in 0.2 ml of chloroform for 1 hour at 37°C. Then, an equal volume of autoclaved TE buffer was added and lightly shaken for 2 minutes. The phase separation between the chloroform and the TE was allowed to form after

30 minutes. This mixture was then centrifuged for 5 seconds at 10,000xg. Next, 200 μL of TE supernatant was sampled. The amount of pDNA was determined with a PicoGreen® (Molecular Probes) fluorescence assay according to manufacture's instructions.

Agrose Gel Electrophoresis of pDNA Released from Nanospheres:

The release of pDNA-LPEI from 1% complexed pDNA loaded nanospheres was characterized. One batch each of impeller formed 1% complexed pDNA nanospheres and 10% complexed pDNA nanospheres was also analyzed. Blank and 1% non-complexed nanospheres were used as controls. First, 2 mg of each nanosphere formulation was suspended in 500 μL of TE buffer and incubated at 37°C under constant rotation. Next, after 30 minutes, 1, 3, 6, 12 hours, 1, 2, 3, 4, 5, 6, 7 days, the nanosphere suspensions were centrifuged at 10,000x, 450 μL of the supernatant was collected and replaced with an equal volume of fresh TE buffer. Furthermore, the release samples were lyophilized and re-suspended in 100 μL of TE buffer. The structural integrity of the pDNA and pDNA-LPEI released from the 1% non-complexed pDNA loaded nanospheres were analyzed by agrose gel electrophoresis. First, 30 μL samples from each release time point and nanosphere formulation were mixed with 6 μL of 6x loading dye and loaded into a 0.8% agrose gel containing ethidium bromide.

Release Profile of Nanospheres Based from Transfection:

The quantitation of the bioactivity of pDNA-LPEI released from the nanospheres was determined with an X-Gal transfection assay using primary human dermal fibroblasts. The cells were maintained according to the protocol in Section 3.2.4 of this thesis. Next, 40 μL release samples was added to the feeding medium. Control fibroblasts were transfected with stock pDNA using a mixture of 200 ng (2 μL), 1.9 μL of FuGENE 6, and 96.1 μL of DMEM. Stock pDNA, release from blank nanospheres, 1% non-complexed pDNA loaded nanospheres, and TE buffer will be used as negative controls. Next, the cells were washed with phosphate buffer saline (PBS), fixed with 1% formaldehyde and an X-Gal staining assay was used to determine transfection. Five random photos were taken. The cells that appeared blue were successfully transfected and were expressing the β-gal enzyme. Transfection percentage was calculated by dividing the number of transfected (blue) cells by the total number of cells.

Atomic Force Microscopy of Released pDNA-LPEI:

The physical structure of the released pDNA-LPEI complexes was characterized using atomic force microscopy (AFM, Vecco Nanoscope III). First, stock samples of pDNA and pDNA-LPEI at concentrations of 20 μg/ml were analyzed in order to obtain a benchmark visual. Samples were prepared by pipetting 20 μL of the release onto a silicon wafer (donated by Bi-min Zhang Newby, Department of Chemical Engineering, University of Akron), dehydrated, and visualized using AFM. Blank nanospheres, stock pDNA, and stock pDNALPEI complex will be used controls. The structure of the released pDNA-LPEI complex was compared to the structure of stock pDNA-LPEI complex.

Verify Nanospheres are Taken up by Cells and Achieve Higher Transfection Efficiency as Compared to pDNA Alone:

Rationale: An overall enhancement of transfection through a sustained release of pDNA-LPEI complexes from endocytosed nanospheres as compared to pDNA alone needs to be verified. Therefore, endocytosis of nanospheres and the increase in transfection efficiency was verified. The hypothesis was that cellular uptake of the FITC labeled nanospheres will be visualized and verified using confocal microscopy. In addition, a significant difference in the transfection efficiency was hypothesized to exist between the control cells exposed to 4 μg of pDNA and the cells exposed to 400 μg of either 1% or 10% complexed pDNA loaded nanospheres.

Cellular Uptake of Nanospheres:

In order to determine if the LTP/PEG-g-CHN/LPEI nanospheres were taken up by endocytosis, primary human fibroblasts were exposed to FITC labeled nanospheres and visualized with confocal microscopy (Olympus Fluoview). First, FITC loaded (1%) nanospheres were produced according to the aforementioned nanosphere synthesis procedure in Section 3.2, except 3 mg/ml FITC solution in 1 ml of DMSO was used in place of 1 ml of $dH_2O$. Next, human dermal fibroblasts were seeded onto sterile German glass cell culture cover slips (Fisher) in 24 well tissue culture plates at a density of 25,333 cells/well and maintained overnight at 37°C with fibroblast feeding media. The next day, the fibroblast feeding media was replaced. Then, 1 mg of FITC labeled nanospheres was suspended in 1 ml of $dH_2O$. Next, 80 μL of the suspended nanospheres were added to the feeding medium of each well. Human dermal fibroblast cells without any exposure to FITC loaded nanospheres were seeded onto German glass cover slips were used as negative controls.

After 24 hours of incubation, the fibroblast cells were washed with PBS and fixed with 1% formaldehyde in PBS for 10 minutes. Next, the cells were washed with PBS, and then 2.5 μL of 6.6 μM Rhodamine Phalloidin stock solution (Molecular Probes) was diluted to 100 μL in PBS and added onto each cover slip. After 20 minute incubation at room temperature, the cells were washed with PBS. The cover slips were then mounted to glass slides with Vectashield mounting media containing DAPI (Vector laboratories). Excess mounting media was removed; the cover slips were sealed and stored at 4°C. Next, FITC labeled nanosphere cellular uptake was visualized first using fluorescent microscopy (Axiovert 200, Carl Zeiss), and then with confocal microscopy (Olympus Fluoview) at NEOUCOM with 2 channels of fluorescence (FITC and Rhodamine), and photographed with a phototube. Confocal microscopy assistance was provided by Jeanette G. Killius at NEOUCOM.

Transfection Efficiency of Nanospheres:

To qualitatively examine the transfection efficiency of both 1% and 10% complexed pDNA loaded nanospheres, an X-Gal transfection assay was performed on primary human fibroblasts with the direct addition of nanospheres. First, human fibroblast cells were seeded onto 24 well tissue culture plates at a density of 25,333 cells/well and maintained overnight at 37°C with fibroblast feeding media. The next day, the fibroblast feeding media was replaced. Next, 2 mg of nanospheres were suspended in 500 μL of feeding medium. Then, 100 μL of the suspended nanospheres were added to the cells. Control fibroblasts were transfected with stock pDNA using a mixture of 200 ng (2 μL), 1.9 μL of FuGENE 6, and 96.1 μL of DMEM. After 3, 5, 7, 9, and 11 days of incubation, the fibroblast cells were fixed with 1% formaldehyde in PBS for 10 minutes. Next, an X-Gal transfection assay was performed according to the manufacturer's instructions. For each transfection result (n = 3 for each time point), three random fields were selected using a microscope (Axiovert 200, Carl Zeiss) with a 20X magnification lens, and bright field images were captured using a Cannon Power Shot G5 camera. The cells that appeared blue were successfully transfected and were expressing the β-gal enzyme. Blank nanospheres, 4 μg of complexed pDNA, pDNA with FuGENE 6, blank TE buffer, and 4 μg of pDNA were used controls.

Statistics:

All quantitative studies were performed in 6 replicates determined by power analysis with $\alpha = 0.05$. The non-parametric Kruskal-Wallis analysis of variance was used to determine statistical differences within each sample group. All results were considered significant when $p \leq 0.05$. If no significant differences were found within a sample group, then the sample was considered normally distributed. Tukey's analysis of variance was then performed among the normally distributed sample groups. All results were considered significant if $p \leq 0.05$.

Results:

Preliminary Results: Agarose Gel Electrophoresis Assay of Sonicated pDNA-LPEI Complex:

Studies in the past have shown that pDNA complexed with polycationic polymers results in a high molecular weight band in the loading wells of the gel. These bands were visible in the gel for the sonicated samples 1:1 pDNA-LPEI and for stock 1:1 pDNA-LPEI (Figure 3). No evidence of pDNA shearing was visible in the gel. If un-complexed pDNA is sonicated, shearing and destruction to the pDNA can be visualized as low molecular weight streaks in the gel. However, the electrophoresis gel showed the pDNA was complexed to the LPEI and pDNA was not sheared during sonication.

Transfection Efficiency and Cytotoxicity of pDNA-Polymer Complexes:

The in vivo addition of complexes of 1:1 pDNA-LPEI, 1:1 pDNA-PEG-g- CHN, and 1:10 pDNA-PEG-g-CHN yielded high cell viability, which was comparable to blank cells and cells transfected only with pDNA as shown in Figure 4. Cells that were exposed to 1:1 pDNA-LPEI, 1:1 pDNA-PEG-g-CHN, and 1:10 pDNA-PEG-g-CHN reached confluent populations that continued to proliferate and grow (Figure 4). The 1:1 pDNA-LPEI complex had nearly identical if not better cell viability to that of the established transfection reagent FuGENE 6 (Figure 4). However, cells that were exposed to 1:2, 1:4, 1:8, 1:10 pDNA-LPEI and 1:1, 1:2, 1:8 pDNA-BPEI did not continue to grow and cell death occurred (Figure 4).

Fibroblasts exposed to 1:1 pDNA-LPEI (4 μg of LPEI) produced the highest transfection percentage of approximately 30% (Figures 5 and 6), which was much higher than the accepted transfection reagent FuGENE 6 at approximately 12%. Complexes of pDNA with BPEI and PEG-g-CHN produced low transfection percentage. Hence, due to PEG-g-CHN's low transfection and BPEI's high cytotoxicity, they were not considered for future conjugation studies. X-Gal transfection assay revealed no significant difference ($p = 0.1019$) between transfection from stock pDNA-LPEI and 30 second and 1 minute sonicated pDNA-LPEI (Figures 5 and 6). Statistical insignificance was determined using Kruskal-Wallis Test. Transfection differences were considered significant when $p < 0.05$. Therefore, complexing pDNA with LPEI at a 1:1 mass ratio protects the pDNA from shearing during sonication.

Scanning Electron Microscopy of Nanospheres:

Scanning electron microscopy (SEM) is utilized to examine the nanospheres' morphology, size, and shape. The images obtained by the SEM reveal a smooth surface morphology of all nanosphere formulations (Figures 7 through 11). The diameter range of the 1% complexed pDNA nanospheres is between 100 to 500 nm (Figures 7 through 11). Nanospheres with diameters of 100 nm or less are difficult to visualize due to the limitations of the SEM. The shape of all the nanosphere formulations is spherical (Figures 7 through 11). The SEM images of the impeller formed 1% complexed nanospheres reveal a diameter range between 100 to 500 nm (Figure 10), which is comparable to the sonicator formed 1% complexed pDNA nanospheres. Similarly, the 10% complexed pDNA nanospheres are shown in the SEM images to have a diameter range between 100 to 500 nm. Nanospheres fusing together are also seen in the SEM images (Figures 7 through 11).

Laser Light Scattering of Nanospheres:

Dynamic laser light scattering is used as an additional method to quantitatively measure the nanosphere diameter range. The frequencies of the nanosphere diameters are reported as differential distribution values. Laser light scattering measures the blank nanosphere diameter range to be between 156 to 562 nm (Figure 12). Larger particles are also measured, which ranged from 7 to 10 μm. These larger particles could be either aggregates of nanospheres or actual microspheres. The 1% encapsulation of pDNA-LPEI complexes in the nanospheres appears to have little effect on the diameter of the nanospheres. Laser light scattering reveals a diameter range between 128 to 716 nm of the 1% complexed pDNA nanospheres (Figure 13). The impeller formed 1% complexed pDNA nanospheres have a slightly smaller and narrower diameter range between 92 to 349 nm (Figure 14). A population of smaller nanospheres ranges between 24 and 49 nm. Microspheres ranging between 1000 to 3000 nm are also measured from the impeller formed 1% complexed pDNA nanospheres (Figure 14). Unexpectedly, the 10% complexed pDNA nanospheres ranges in diameter from 14 to 594 nm (Figure 15). The smaller diameter measurements may be pDNA-LPEI complexes.

Degradation of Nanospheres:

Dynamic laser light scattering is further utilized to characterize the degradation of the nanospheres. The degradation is equated as the decrease in mean diameter of the nanospheres after 7 days. Blank nanospheres are completely degraded in PBS at 37°C after 7 days (Figure 16), which mirrors the degradation of LTP films. The blank nanospheres lose nearly 75% of it's diameter after 3 days. After 3 days, the mean nanosphere diameter went from 1.9 μm to 500 nm. After 7 days the mean diameter is 2 nm (Figure 16). The degradation profile of the 1% complexed pDNA nanospheres has an unexpected increase in diameter from day 0 to day 1 (Figure 17), which may be due to nanosphere aggregation. Furthermore, the diameter of 1% complexed pDNA nanospheres levels off to 40 nm on day 7, which may be the pDNA-LPEI complex or aggregates of the complex. A similar degradation profile for impeller formed 1% complexed pDNA is shown in Figure 18. A similar degradation profile is seen for the 10% complexed pDNA nanospheres (Figure 19). The 10% complexed pDNA nanosphere mean diameter increases from 421 nm to 711 nm from day 0 to day 1. However, the mean diameter begins to decrease down to 297 nm on day 2. On day 7 the mean diameter is measured at just 1 nm, and is determined to be fully degraded.

Cell Viability after Exposure to Nanospheres:

The viability of human dermal fibroblasts 24 hours after exposure to 1% pDNA nanospheres is determined using a LIVE/DEAD Cell Assay. In the LIVE/DEAD cell assay, metabolically active cells reduce C-resazurin to red fluorescent C-resorufin and dead or dying cells fluoresce green, since their plasma membranes are compromised and are permeable to the nucleic stain SYTOX. Nanospheres, which fluoresced green with the same filter as dead cell nuclei, were distinguished by their smaller and more spherical shape as opposed to the larger bean shaped nuclei. In Figures 20 through 29, the dead cells are circled in yellow to improve their visualization. Cell viability is the percentage of live cells, which is calculated by the following equation:

$$\text{Cell Viability} = [(\text{red cells})/(\text{red cells} + \text{green cells})] \times 100$$

The addition of the positive control buffer, 40 μL of TE buffer and 160 μL of $dH_2O$, to fibroblasts results in a 98% ± 1 % cell viability after 24 hours (Figure 20 and 29). In the fluorescence images, only several green nuclei are seen, but red-fluorescent resorufin can be seen in many metabolically active cells (Figure 20). The addition of 4 μg of pDNA (200 μL of 20 μg/μL) to fibroblasts also results in a high cell viability of 99% ± 1% (Figures 21 and 29). The negative control, fibroblasts exposed to 2 mM $H_2O$ for 3 hrs, has a 0% ± 0% viability (Figures 22 and 29). Every nucleus of the negative control fluoresces green. Fibroblasts exposed to 400 μg of 1% complexed pDNA nanospheres have a viability of 97% ± 2% (Figures 25 and 29). Similar results are seen for impeller formed 1% complexed pDNA nanospheres and PLGA nanospheres, which exhibit viabilities of 94% ± 1% and 98% ± 1% respectively (Figures 26, 27 and 29). However, viability decreases with the addition of 4 μg of pDNA complexed to LPEI and 4 μg of pDNA complexed to FuGENE 6 transfection reagent, which yields viabilities of 91% ± 3% and 90% ± 4% (Figure 23, 24 and 29). The lowest fibroblast viability is seen with the addition of 100 μg of 10% complexed pDNA nanospheres, which results in viability of 86% ± 2% (Figures 28 and 29).

Statistical analysis is performed to determine significant differences among the gene vectors and controls. First, a Kruskal Wallis nonparametric test of variance demonstrates that there is no significant difference within each group of gene vectors, which shows that the samples are normally distributed. Therefore, Tukey's analysis of variance is performed among the groups of gene vectors, since each group has a normally distributed sample. Tukey's comparison of means finds that after 1, 3, 7, and 11 days, the viability of 1% pDNA nanospheres is not significantly different ($p > 1.000$) to TE buffer, PLGA nanospheres, or pDNA. After 1 day, only 10% pDNA nanosphere viability was found to be significantly different ($p = 0.0003$) than TE buffer. After 3 and 7 days, TE Buffer viability is significantly different than pDNA-FuGENE 6 ($p < 0.0001$) and 10% pDNA nanospheres ($p = 0.0047$) and ($p = 0.0021$) respectively. After 11 days, TE buffer is significantly different than blank nanospheres ($p < 0.0001$), pDNA-FuGENE 6 ($p < 0.0001$), 10% pDNA nanospheres ($p < 0.0001$), and pDNA-LPEI ($p = 0.0006$).

<u>Loading of</u> pDNA <u>into Nanospheres:</u>

The loading efficiency of pDNA into the nanospheres is determined using a PicoGreen® DNA quantitation assay. A decrease in fluorescence is observed when pDNA is complexed with LPEI. Therefore, a standard curve for emission fluorescence and concentration is made using titrations of the pDNA-LPEI complexes. The loading of pDNA-LPEI is determined from the standard curve. Loading efficiency is defined by the following equation:

$$\text{Loading Efficiency} = [(\text{measured amount of pDNA from nanospheres})/(\text{amount of pDNA in nanospheres})] \times 100$$

The loading efficiencies for the sonicator formed 1% and 10% pDNA nanospheres are 40% ± 3% and 13% ± 1 % respectively (Figure 30). A 40% loading efficiency means that 40% of the pDNA-LPEI used to make the nanospheres is encapsulated in the nanospheres during the emulsion. The impeller formed 1% pDNA nanospheres yield a much higher loading efficiency of 89% ± 8% (Figure 30).

Statistical analysis is performed to determine significant differences among the loading efficiencies. Initially, a Kruskal Wallis nonparametric test of variance demonstrates that there is no significant difference within each group of nanospheres, which shows that the samples are normally distributed. Therefore, Tukey's analysis of variance is performed among the groups of nanospheres, since each nanosphere has a normally distributed sample. Tukey's test shows that the loading efficiencies of all nanosphere formulations are significantly different from each other ($p < 0.0001$).

<u>Agrose Gel Electrophoresis of</u> pDNA <u>Released from Nanospheres:</u>

In order to qualitatively characterize the release of nanospheres, an agrose gel electrophoresis is performed. In gel electrophoresis, pDNA migrates from a well through the gel due to a charge gradient. When pDNA is complexed with cationic polymer LPEI at a 1:1 mass ratio (7.7 N/P ratio), it loses its negative charge and does not migrate through the gel. Thus, bands that remain in the wells are determined to be pDNA-LPEI complexes. Agrose gel electrophoresis of blank nanospheres results in low molecular weight bands (fast migrating particles) below the 1 kb DNA ladder (Figure 31). These low molecular bands are determined to be degradation products of the nanospheres or smaller nanospheres.

Gel electrophoresis of the 1% complexed pDNA nanosphere release is sustained over 7 days (Figure 32). High to low molecular weight streaks in the gel are determined to be pDNA sheared from sonication. Sheared pDNA is released from 0.5 hrs to day 2 (Figure 32D through J). After 0.5 hrs the most sheared pDNA is released. Non-complexed super coiled dimmer pDNA and relaxed pDNA dimmer are seen in the release from 0.5 hrs to day 2 (Figure 32D through J). From 0.5 hrs to day 7, pDNA-LPEI complexes are released (Figure 32D through O). The greatest release of pDNA-LPEI

appears between 0.5 hrs to 1 day (Figure 32D through I). Bands of pDNA-LPEI are still visible between day 2 to day 7, but the UV fluorescence grows fainter with each passing time point (Figure 32J through O). Degradation products or small nanospheres are seen in the release from 0.5 hrs to day 7, with greatest intensity after 0.5 hrs (Figure 32D through O).

The electrophoresis gel of release from 10% complexed pDNA nanospheres resembles the release of the 1% complexed pDNA nanospheres, but with greater intensity of pDNA and pDNA-LPEI (Figure 33). Likewise, the 10% loaded nanospheres demonstrates a release over 7 days. Bright bands of pDNA-LPEI are seen in the wells from 0.5 hrs to day 4 (Figure 33D through K), and slightly fainter bands for day 6 and 7 (Figure 33 L through M). Sheared pDNA, relaxed and super coiled dimmer are also released from 0.5 hrs to day 7 (Figure 33 D through K). However, there is less intensity of degradation products (or small nanospheres) seen in the 10% complexed pDNA nanosphere release than in the 1% loaded nanospheres.

The impeller formed 1% complexed pDNA nanospheres yields a similar release to the sonicator formed nanospheres. However, the impeller nanospheres demonstrate their largest release after 0.5 and 1.5 hrs of pDNA-LPEI, sheared pDNA, relaxed and super coiled dimmer pDNA, and degradation products (Figure 34 D through E}. Fainter bands of pDNA-LPEI are seen between 3 hrs and 7 days (Figure 34 F through O).

The release of the pDNA from the 1% non-complexed pDNA nanospheres was visible in the agrose gel electrophoresis assay for the first 2 days of the release only as shown in Figure 35. The pDNA released on days 1 and 2 (Figure 35 C through D) was visibly sheared from the sonication during the nanosphere formulation. However, condensation of the pDNA by either LPEI or PEG-g-CHN on the release of day 2 (Figure 35 D) was denoted by bright band still in the well. This condensation could be explained by leeching LPEI and PEG-g-CHN as the nanosphere degrades. The pDNA could then have formed a complex with either of these polymers. This agrose gel release study further demonstrated the importance of complexing pDNA before its encapsulation in nanospheres by emulsification through sonication.

<u>Release Profile of Nanospheres Based from Transfection:</u>

The release profile of various nanosphere formulations is quantified using transfection percentages obtained from the release samples. However, insufficient transfection is obtained from the 1% pDNA nanosphere release samples. Transfection is only obtained from the day 2 release samples of 1% pDNA nanospheres (Figures 36 and 37). Transfection cannot be obtained from any of the other release time points. Release profiles were obtained from impeller formed 1 % pDNA nanospheres and the 10% pDNA nanospheres (Figures 36 and 37). The profiles from both 10% pDNA nanospheres and impeller formed 1% nanospheres demonstrates a fast release initially and then a leveling off after 7 days.

<u>Cellular Uptake of Nanospheres:</u>

Cellular uptake of nanospheres is confirmed with confocal microscopy of human dermal fibroblasts exposed to FITC loaded nanospheres. First, a preliminary study is performed using fluorescent microscopy with 3 channels of fluorescence (Axiovert 200, Carl Zeiss). The fibroblasts' nuclei are stained with Hoechst nuclear stain and fluoresce blue. The cytoskeleton is stained with rhodamine phalloidin and fluoresces red. These cellular stains can be seen for both control fibroblasts (Figure 39) and fibroblasts 58 exposed to 100 μg of FITC nanospheres (Figure 38). The FITC labeled nanospheres fluoresced green (Figure 38). However, these fluorescent images only suggest nanosphere uptake due to their 2-dimensionality.

Therefore, a 3-dimensional analysis of the uptake is achieved using confocal microscopy on fibroblasts exposed to 100 μg of FITC loaded nanospheres. Confocal microscopy produced 0.5 μm slices of the fibroblast samples. These slices revealed nanospheres at various depths within the fibroblasts' cytoskeleton (Figure 40). Slice depths are reported in the upper left hand corner of each image in Figure 40. The locations where nanospheres appeared were circled in yellow (Figure 40). At the depth 1 um, the cytoskeleton was visible, but few nanospheres were seen. However, at 2 and 3 μm depths, a number of nanospheres appear (Figure 40). These nanospheres later disappear at depths of 3.5 and 4.0 μm. The appearance and disappearance of the nanospheres within the cytoskeleton verify their uptake in the fibroblasts.

<u>Transfection Efficiency of Nanospheres:</u>

The controllable and sustained transfection from nanospheres is demonstrated by X-Gal staining of human dermal fibroblasts exposed to 400 μg of 1% complexed pDNA nanospheres. X-Gal staining is used to determine the percentage of cells transfected with pDNA expressing lacZ. The product of the lacZ gene, β-galactosidase, catalyzes the hydrolysis of X-gal, producing a blue color within the cell. Transfection percentage is used to determine the transfection efficiency of the nanospheres. The transfection percentage is calculated by the following equation:

$$\text{Transfection Percentage} = [(\text{blue cells})/(\text{total cells})] \times 100$$

Fibroblasts exposed to 4 μg of pDNA (200 μL of 20 μg/ml) after 3, 5, 7, 9 and 11 days demonstrate no transfection, which is comparable to buffers alone (Figure 41). Exposing fibroblasts to 4 μg of pDNA complexed at a 1: 1 mass ratio with LPEI achieves a high transfection percentage of 25% ± 5% after 3 days (Figure 41). However, the transfection percentage starts to diminish after 5, 7, 9, and 11 days to 5% ±1 %, 2% ± 1%, 2% ± 1%, and 2% ± 1% respectively (Figures 41 through 47). A diminishing transfection percentage is also observed for pDNA complexed with FuGENE 6. The greatest transfection efficiency using FuGENE 6 occurs at day 3 with a 15% ± 2% transfection percentage. Similar to LPEI, transfection efficiency decreases after day 5, 7, 9, and 11 to 5% ± 2%, 2% ± 1%, 3% ± 1%, 3% ± 1% (Figures 41 through 45, 48 and 49).

Unlike pDNA complexes with LPEI and FuGENE 6, nanospheres demonstrate a controllable and sustained transfection (Figures 41 through 45). The daily transfection profile using 1% complexed pDNA nanospheres demonstrates an initial delay until day 5, peaks after 7 days, and is sustained through day 11 (Figure 46 through 49). The transfection efficiency of 1% complexed pDNA nanospheres on days 3, 5, 7, 9, and 11 are 1% ± 1%, 4% ± 1%, 10% ± 1%, 8% ± 1%, and 6% ± 1% (Figures 41 through 49). An exploratory study using impeller formed 1% complexed pDNA nanospheres finds similar transfection results to the sonicator formed 1% complexed pDNA nanospheres. The transfection profile is delayed until day 7 using the impeller formed nanospheres. However, transfection from the impeller formed nanospheres is lower than from the sonicator formed nanospheres. Impeller formed 1% complexed pDNA nanospheres reach transfection efficiencies of 0%, 0%, 3%, 3%, and 2% on days 3, 5, 7, 9, and 11 respectively. An additional exploratory transfection study using 10% complexed pDNA nanospheres is unable to achieve any transfection. No fibroblasts are transfected using the 10% complexed pDNA nanospheres.

Statistical analysis is performed in order to establish significant differences in transfection efficiencies among the gene vectors. First, a Kruskal Wallis nonparametric test for variance demonstrates that there is no significant difference within each group of vectors, which shows that the vector samples are normally distributed. Then, Tukey's analysis of variance is performed among the groups of gene vectors, since each group has a normally distributed sample. On day 3, the transfection percentage for 1% pDNA nanospheres was found to be significantly different ($p < 0.0001$) than pDNA-LPEI and pDNA-FuGENE 6. On day 5, the transfection percentage of 1 % pDNA nanospheres was not found to be significantly different than either pDNA-LPEI ($p = 0.9989$) or pDNAFuGENE 6 ($p = 0.9996$). However on day 7, a significant difference exists between 1% pDNA nanospheres and pDNA-LPEI ($p = 0.0146$) and pDNA-FuGENE 6 ($p = 0.0121$). In addition, no significant differences were observed between transfection from nanospheres on days 7, 9, or 11 ($p = 0.9949$, $p = 0.8292$, $p > 1.00$ respectively). No significant differences in transfection percentage were found between 1% pDNA nanospheres and pDNA-LPEI for days 5, 9, and 11 ($p = 0.9989$, $p = 0.1769$, and $p = 0.2863$) and pDNA-FuGENE 6 for days 5, 9, and 11 ($p = 0.9996$, $p = 0.3176$, $p = 0.7329$).

Gel Electrophoresis of Sonicated pDNA-LPEI Complex:

Gel electrophoresis demonstrates that complexing pDNA with a polymer such as LPEI prevents large scale degradation of pDNA during the sonication step of nanosphere production. The complexing of pDNA by LPEI produces a condensed and more structurally stable package than pDNA alone. This smaller and more robust package can withstand the large amounts of energy and forces present in the emulsion created by sonication. Sonicating pDNA alone, degrades pDNA, and renders it inactive for transfection.

Transfection Efficiency and Cell Viability of pDNA-Polymer Complexes:

The complex of pDNA and LPEI is the most efficient pDNA-polymer complex at transfecting cells while maintaining acceptable cell viability. LPEI has better transfection efficiency than FuGENE 6, BPEI, and PEG-g-CHN, which is believed to be due to its ability to escape endosomes. In addition, LPEI demonstrates higher cell viability than FuGENE 6 and BPEI. The optimum mass ratio of pDNA to LPEI is 1 to 1, which corresponds to a 7.7 N/P. The 1:1 mass ratio of pDNA and LPEI produces neutrally charged complexes that have the highest transfection efficiency compared to other mass ratios, which is a result of the complete neutrality of charge. Future studies must explore the structure and size of the pDNA-LPEI complex, in order to better understand its mechanism for transfection.

Nanosphere Synthesis:

Nanospheres can be synthesized from L-tyrosine polyphosphate, PEG-g-CHN, and pDNA-LPEI using both the sonication and impeller methods to create water and oil emulsions. These nanosphere formulations represent the first

attempt to achieve a controlled and sustained intracellular delivery of pDNA for gene therapy. Both the sonication and impeller method are effective at producing pDNA loaded nanospheres.

Characterize the size, shape, morphology, degradation, and cytotoxicity of pDNA-LPEI loaded nanospheres:

SEM of nanospheres:

The nanospheres' shape, surface morphology, and size play a major role in biocompatibility and the ability to be internalized. Spherical and smooth particles demonstrate favorable transport in circulation and biological systems. Meanwhile, irregular and rough particles pose problems when navigating through microcirculation. Fibrous particles have been shown to stress cells and elicit immune responses. Previous studies show that cellular internalization of particles is size dependent. Eukaryotic cells can internalize nanoparticles with diameters ranging from 50 nm to 1 $\mu$m. Therefore, nanospheres must be produced with diameters smaller than 1 $\mu$m for intracellular delivery of genes. Scanning electron microscopy (SEM) reveals that our 1% pDNA nanospheres are spherical, smooth, and range in diameter between 200 to 700 nm (Figures 7 and 8). Ideally, these 1% pDNA nanospheres can be internalized by human cells and should have favorable navigation through the circulation. SEM also demonstrates that encapsulating pDNA-LPEI complexes into the nanospheres does not alter the shape, surface morphology, and size when compared to blank nanospheres (Figure 9). SEM images of the impeller formed nanospheres also demonstrate spherical shape, smooth surface morphology, and range in diameter between 200 to 700 nm. The impeller method is able to produce similar nanospheres compared to the sonication method. The 10% pDNA nanospheres appear slightly less spherical and smooth than the 1%, which could be attributed to pDNA-LPEI complex aggregation.

Laser Light Scattering of Nanospheres:

The results from the dynamic laser light scattering reaffirm nanosphere diameter range found with SEM. Laser light scattering shows that all the nanospheres formulations have a near normal distribution of diameters. The diameter distribution of impeller formed nanospheres is smaller and narrower (100 nm to 500 nm) than the sonicator formed, which could be attributed to greater energy created in the impeller emulsion. Furthermore, the impeller formed nanospheres are created in a water-in-oil-in-water emulsion, which may have greater emulsion stability than a sonication emulsion. Therefore, all pDNA nanosphere formulations are favorable in size for cellular internalization.

Aggregation of nanospheres is also observed using laser light scattering. The nanosphere aggregates appear as particles with 1 to 10 $\mu$m diameters (Figures 12 through 14). The aggregation could be due to the surfactant being washed away during the nanosphere washing step. SEM images also show aggregation of the nanospheres, which affirms that some nanospheres do aggregate after re-suspension. In addition, laser light scattering of the 10% pDNA loaded nanospheres shows nanoparticle populations between 14 to 42 nm in diameter (Figure 15). These smaller nanoparticles are hypothesized to be pDNA-LPEI complexes that were not encapsulated due to their high concentration. Complexes of pDNA and LPEI are typically 50 nm in size at a 1:1 mass ratio (N/P = 7.7). Sonication may have made the complexes smaller or broken them apart.

Degradation of Nanospheres:

Nanosphere degradation based off changes in mean diameter over time, shows that all pDNA nanosphere formulations are fully degraded after 7 days. This degradation profile is comparable to the 7 day degradation of LTP films incubated in PBS, which is expected since the nanospheres are about 90% LTP. All nanosphere formulations are approximately 75% degraded after 3 days (Figures 16 through 19), which is also comparable to LTP films incubated in PBS. LTP undergoes hydrolytic degradation, which is why PBS is used as the solvent in this study. The degradation results of nanospheres are a good indicator of how the nanospheres will degrade in the body, since PBS mimics the physiological salt concentration in the body. However, further investigation needs to be addressed on the degradation of the nanospheres in the presence of proteins. Future studies must measure the degradation of the nanospheres in serum or cell media. Although, the serum may cause problems when used with laser light scattering, since serum proteins can be visualized in laser light scattering.

The increase in mean nanosphere diameter from day 0 to day 1 with the sonicator formed pDNA nanospheres can be explained by increased aggregation (Figures 16 and 19). As the nanospheres start to degrade, much of the surfactant is removed, which allows aggregation to occur more readily. Furthermore, pDNA-LPEI complexes may be encapsulated on or close to the surface of the sonicator formed nanospheres. After 1 day of degradation, more pDNA-LPEI complexes will be exposed. The presence of pDNA-LPEI complexes on the surface of the nanospheres also increases aggregation due to charge interactions between the pDNA and LPEI. This diameter increase is not seen with impeller formed pDNA nanospheres and blank nanospheres (Figures 17 and 18). This lack of aggregation supports the

theory that pDNA-LPEI complexes on the surface of the nanospheres cause aggregation. Blank nanospheres contain no pDNA-LPEI complexes and impeller formed pDNA nanospheres have a better encapsulation due to the water-in-oil-in-water emulsion. The water-in-oil-in-water emulsion should encapsulate the pDNA-LPEI complexes deeper in the nanospheres.

Cell Viability after Exposure to Nanospheres:

The LIVE/DEAD cell assay demonstrates that 1% pDNA nanospheres have viabilities comparable to buffers, pDNA alone, and PLGA nanospheres. Other gene vectors such as LPEI and FuGENE 6 demonstrate increasing toxicity between 1 and 11 days of incubation with human dermal fibroblasts. The 1% pDNA nanospheres avoid these toxic effects by encapsulating the toxic LPEI and releasing it at a controlled and sustained rate. The nanospheres prevent the burst toxic effects of LPEI, which previous studies show to be toxic to cells at high concentrations. High cell viability is also due in part to the nontoxic polymers used to fabricate the nanospheres. LTP is a biocompatible polymer synthesized from the amino acid L-tyrosine and phosphate groups, which both are found naturally in the body. The hydrolytic degradation of LTP results in L-tyrosine and a phosphate that are both nontoxic. PEG and Chitosan have also been shown to be nontoxic. The 1% pDNA nanospheres are safe to use in vitro with fibroblasts at their effective concentrations, since they have comparable viabilities to buffers, pDNA, and PLGA nanospheres.

The LIVE/DEAD cell assay also demonstrates the degradation of the nanospheres as well. The fact that the nanospheres fluoresce green provides an opportunity to watch the nanospheres diminish in fluorescence as they degrade throughout the 11 days (Figures 25 through 29). Furthermore, the pDNA-LPEI and pDNA FuGENE 6 complexes also fluoresce green when using the LIVE/DEAD Cell assay. Future studies of quantifying these complexes can be pursued using a variation of this assay. This data could prove valuable since quantifying pDNA-LPEI complexes is impossible at low concentrations.

Quantify and Characterize Loading and Release pDNA-LPEI from Nanospheres: Loading of pDNA-LPEI in Nanospheres:

The PicoGreen assay shows that the loading efficiency varies among the various pDNA nanosphere formulations. The 10% pDNA nanospheres have the lowest loading efficiency at 13%, which can be attributed to the pDNA-LPEI concentration exceeding the nanospheres maximum loading. Micro and nanoparticles have a maximum loading, which cannot be increased with additional loading materials. Excess non-encapsulated pDNA-LPEI is washed away during the nanosphere collection and washing steps. The 1% pDNA nanospheres have the next highest loading efficiency at 40%. Further investigation is needed to determine if a 1% pDNA-LPEI concentration is the optimum concentration for loading in this nanosphere formulation. The sonication method for nanosphere formation relies on the random encapsulation of hydrophilic pDNA-LPEI in the inner water phase, due to the thermodynamic favorability. However, low encapsulation can occur when the hydrophilic pDNA-LPEI is caught in the outer water phase, which is also thermodynamically favorable. The impeller formed 1% nanospheres have the highest loading at 89%. The impeller method achieves a higher loading efficiency due to the nature of its formation, which includes and an initial water-in-oil emulsion. Unlike the sonication method where pDNA-LPEI can collect randomly in the outer or inner water phase, the initial water-in-oil emulsion forces nearly all the pDNA-LPEI in the inner water phase. Thermodynamics favor the pDNA-LPEI to exist only in the water phase. A common factor in both sonication and impeller systems that leads to a decrease in loading is the destruction of pDNA-LPEI during the emulsion. The emulsion generates a large amount of energy that can shear and destroy pDNA, which is demonstrated in the release of non-complexed pDNA nanospheres (Figure 35).

Agrose Gel Electrophoresis of pDNA Released from Nanospheres:

Gel electrophoresis shows a sustained release of PDNA-LPEI complexes from nanospheres throughout 7 days. The electrophoresis gel of the 1% pDNA nanospheres shows the greatest release is found during the first 2 days, which corresponds to the nanosphere degradation observed in Figure 17. The gel electrophoresis also reveals that the nanospheres are releasing non-complexed and degraded pDNA. The amount of non-complexed pDNA appears less than the complexed bands of pDNA-LPEI, which means that most of the pDNA-LPEI complexes are remaining intact. However, the sonication process is causes some of the pDNA-LPEI complexes to un-complex. Stock solutions of pDNA-LPEI complex at the same 1:1 mass ratio as used in the nanospheres, reveal no un-complexed pDNA in the gel. Some of the pDNA that becomes un-complexed is then degraded from the sonication and appears as faint trails in the gel. The faint trail is comprised of various pieces of pDNA at different lengths. The un-complexing and degradation of the pDNA and LPEI could be caused by the large amount of energy created during sonication. Previous studies show that Sonication can degrade pDNA. Release from non-complexed pDNA nanospheres, show that pDNA is completely degraded during nanosphere formation. The release from the 10% pDNA nanospheres is much greater and easier to visualize than the 1% pDNA nanospheres, due to the increase pDNA and pDNA-LPEI present. The impeller formed 1% pDNA nanospheres

show less un-complexed pDNA, which could mean there is less energy exerted into the emulsion.

The completely sheared pDNA found in the non-complexed pDNA nanosphere release further demonstrates the importance of complexing the pDNA before encapsulation in nanospheres. Encapsulating pDNA alone in nanospheres via the sonication method results in an ineffective gene vector, since the pDNA is no longer bioactive.

Release Profile of Nanospheres Based from Transfection:

Using transfection efficiency of nanosphere release in order to generate a release profile is unsuccessful, due to the inability of the release from 1% pDNA nanospheres to transfect cells. Only the release from day 2 of the 1% pDNA nanospheres achieved transfection. This lack of transfection could be attributed to loss of bioactivity of the pDNA during the experimental procedure of the release. The lyophilization and re-suspension of the release may destroy the bioactivity of the pDNA. In addition, the low transfection could be a result of the low loading efficiency found with the 1% pDNA nanosphere. Only 40% of the expected pDNA-LPEI is encapsulated in the nanospheres, which could lead to low transfection. This hypothesis is supported by the higher transfection obtained by the release from 10% pDNA nanospheres and impeller formed 1% nanospheres, which has higher loading than 1% pDNA nanospheres. There is more pDNA-LPEI present in these nanospheres, which leads to a greater release. Future release studies must be performed with a greater amount of 1% pDNA nanospheres in order to obtain transfection from the release. Other future studies could quantify the release of radioactively labeled pDNA-LPEI, since regulations and cost prevented such studies in this current research.

Verification that Nanospheres are Taken up by Cells and Achieve Higher Transfection Efficiency as Compared to pDNA Alone:

Cellular Uptake of Nanospheres:

Confocal fluorescent microscopy has verified the uptake of nanospheres by primary human dermal fibroblasts after 24 hours. Individual nanospheres can be seen appearing and disappearing within the cytoskeleton of the fibroblasts, which proves that they are inside the cell. In addition, nanospheres only require 24 hours to be taken up by the fibroblasts, which ensures that the nanospheres are not fully degraded before they can be internalized. The rapid uptake of these nanospheres is important for ensuring an internal release of pDNA-LPEI, which should improve transfection efficiency. The fluorescent images alone of fibroblasts with nanospheres only suggest an uptake of nanospheres. Although, the confocal images show that many of the nanospheres seen with the fibroblasts are likely inside the cell. Some nanospheres may also be still adhered to the membrane of the cell as well. The specific location of the nanospheres within the cell is unknown at this time. However, the nanospheres are hypothesized to exist within endosomes where they are being degraded. Studies by Leong show that internalized nanospheres are typically found in endosomes. Future studies are needed to verify the location of the nanospheres within endosomes. An assay for endosomal staining can demonstrate that the nanospheres are contained within endosomes. Furthermore, future studies can also verify how the nanospheres uptake the nanospheres. An enzymatic assay to determine receptor-mediated endocytosis must be performed to verify that endocytosis is indeed the method for nanosphere internalization.

Transfection Efficiency of Nanospheres:

X-Gal staining of human dermal fibroblasts exposed to 1% pDNA nanospheres shows a controlled and sustained transfection. The transfection is considered controlled, since transfection is not observed until 5 days after their exposure to cells. Unlike previously established gene vectors such as LPEI and FuGENE 6, there is a delay in transfection until day 5 when using the 1% pDNA nanospheres. This delay is a result of nanospheres taking time to reach the cell, be internalized, and degrade within the cell. Maximum transfection from the 1% pDNA nanospheres is reached on day 7 and is sustained through day 11. The fact that no significant differences were found between nanosphere induced transfection on days 7 through 11, demonstrates that transfection is sustained. The sustained transfection is achieved due to the nanosphere degradation time frame of 7 days (Figure 17). Corresponding to the 7 day release of pDNA-LPEI shown in gel electrophoresis, pDNA-LPEI is released internally in the cell to achieve the sustained transfection up to at least 11 days. The transfection study was terminated after 11 days, since fibroblasts in culture have reached the limits of the environment.

The 1% pDNA nanospheres' controlled and sustained transfection is in stark contrast to the initial burst and decaying transfection of the LPEI and FuGENE 6. The established gene vectors LPEI and FuGENE 6 achieve very high transfection initially. However, LPEI and FuGENE's transfection diminishes between 3 to 11 days due to their inability to control or sustain their transfection. Cationic polymers and lipoplexes are easily cleared by cells and biological systems. Gene vectors such as LPEI must be incorporated into a degradable system in order to obtain a controlled or sustained

transfection. The degradable nanospheres provide a means of controlling and sustaining transfection with LPEI.

Despite the transfection success of 1% pDNA nanospheres, poor results were found for 10% pDNA nanospheres and impeller formed nanospheres. Transfection could not be achieved using 10% pDNA nanospheres. Meanwhile, impeller formed 1% pDNA nanospheres demonstrate very low transfection, but still exhibit a controlled and sustained transfection. However, the sustained transfection obtained by impeller nanospheres is not significantly different from LPEI and FuGENE 6 on days 5 through 11. The low or lack of transfection is a likely a result of the size of the pDNALPEI complexes in the 10% pDNA nanospheres. Studies show that the smaller the pDNA-LPEI complex size, the greater the transfection. Furthermore, the studies show that the higher the concentration of pDNA and LPEI in solution, the larger the complexes that form. The concentration of both pDNA and LPEI is 0.3 mg/ml, 1 mg/ml, and 3 mg/ml when forming the complex for 1% pDNA nanospheres, 10% pDNA nanospheres, and impeller formed 1% pDNA nanospheres respectively. This increase in concentration likely increased the size of the pDNA-LPEI complex, which lowered the transfection. In addition, the cell viability studies show that the 10% pDNA nanospheres are more toxic to cells than the 1% pDNA nanospheres. An increase in cell toxicity leads to poor cell function and less gene expression.

Nanospheres formulated from a blend of LTP and PEG-g-CHN that encapsulate pDNA-LPEI can be used as a controllable and sustainable non-viral gene vector. An emulsion of water and oil produced by sonication and solvent evaporation creates the nanospheres and leads to the encapsulation of the pDNA-LPEI. This fabrication method produces nanospheres that are spherical, smooth, and approximately 100 to 700 nm in diameter. These nanospheres degrade in 7. days in PBS at 37°C. This degradation profile leads to a release of pDNA-LPEI over 7 days with most of the release occurring in the first 2 days. A formulation of 1% pDNA nanospheres exhibits higher cell viability than other established gene vectors such as LPEI and FuGENE 6. The cell viability of these nanospheres is comparable to TE buffer, pDNA, and PLGA nanospheres. The high viability of these nanospheres is due in part to the biocompatibility of the polymers used and the size of the nanospheres. The nanosphere size also provides a suitable scale for internalization by the cells. Uptake of the nanospheres by fibroblasts is achieved in 24 hours, which allows time for an intracellular delivery of pDNA-LPEI. This intracellular delivery leads to a controlled and sustained transfection of human dermal fibroblasts. These nanospheres achieve a controlled transfection by delaying prominent gene expression until 5 days after administration. Maximum transfection is reached on day 7 and sustained through day 11, which is unlike the initial burst transfection and then decay of gene vectors such as LPEI and FuGENE 6. Therefore, the nanospheres formulated from LTP, PEG-g-CHN, and pDNA-LPEI could be valuable vectors for intracellular delivery of therapeutic genes against diseases that require treatment for a couple weeks.

Polyurethane Embodiments:

The use of amino acids in the synthesis of polymers for different biomaterial applications is known (see, e.g., United States Patent No. 6,221,997 which discloses pendant chain amino acid-containing polyurethanes). L-tyrosine has been extensively used for the synthesis of biocompatible and/or biodegradable polymers for different biomaterial applications with particular emphasis on tissue engineering. In particular, L-tyrosine-based pseudo-poly (amino acids) has been investigated for biomaterial applications with desaminotyrosyl hexyl ester (DTH) as the building unit for the polymers. DTH based polycarbonate, polyimminocarbonate, polyphosphates and several other polymers are studied for biomaterial applications. However, the uses of these polymers are restricted due to several limitations regarding degradability, physical-chemical properties and processability as well.

Moreover, the difficulties in tuning the polymer structure and the related properties of the material have limited their chances of using these materials for wide range of applications.

Biocompatible polyurethanes are currently being investigated as an alternative for fabricating tissue engineering scaffolds. Several studies indicate that the ease of synthesizing and tuning the structure leads to wide range of properties that are pertinent to biomaterial applications. Polyurethanes are usually synthesized from three components, macrodiol (*i.e.*, polyol), diisocyanate, and diol or diamine based chain-extenders and has the general structure as shown below. This enables to constitute the soft segment and the hard segment of the polymer, which eventually can be exploited for various properties.

$$-M-(D(CD)_n-M)_m-$$

The above formula is a general schematic for the L-tyrosine-based polyurethane polymers where M = macrodiol, D = diisocyanate, and C = chain extender.

Amino acid based chain extenders are investigated for polyurethanes in very limited cases. Phenyl alanine based chain extender and lysine based isocyanate is used for the synthesis of polyurethanes.

In one embodiment of the present invention, the invention is focused on the synthesis and characterization of polyurethanes based on DTH as the chain extender, (see structure below):

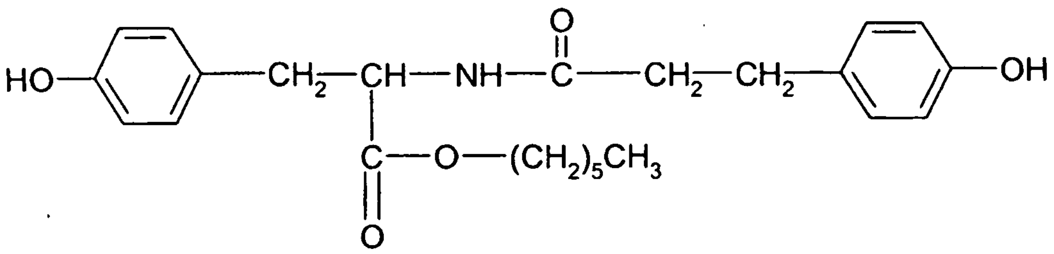

(DTH)

The presence of two hydroxyl groups enables DTH to be used as chain-extender in the synthesis of polyurethane. DTH being the dipeptide moiety synthesized from L-tyrosine and its deaminated metabolite desaminotyrosine, can be effectively used for chain extension for the prepolymer made from the conventional macrodiols and the diisocyanate. Phenyl alanine based chain-extenders are the diester formed by the coupling of the carboxylic acid (of the amino acid) and the hydroxyl group of the of a low molecular weight diol *(e.g.,* ethylene glycol). Whereas, DTH based chain extender is amide product, which makes the potentially degradable under enzymatic condition.

This invention uses the conventional two-step polyurethane synthesis process to synthesize the L-tyrosine based polyurethanes, In the first step macrodiols are reacted with diisocyanate in presence of catalyst with DMF (dimethyl formamide) as solvent at temperature 100°C to 120°C for 3 to 4 hours. In the second step the reaction mixture was cooled down to room temperature and the DTH chain-extender was added. The reaction was further allowed to continue for 10 to 12 hours at 70°C to 80°C. At the end, the reaction was quenched by pouring the reaction mixture in cold concentrated solution of sodium chloride. The product was either filtered or centrifuged according to the condition of the polymer.

The macrodiols (polyols) used for the synthesis of the polyurethanes are, in one embodiment, polyethylene glycol (PEG) and poly caprolactone (PCL) based diols. Potentially non-toxic aliphatic diisocyanate hexamethylene diisocyanate (HDI) was used as the diisocyanate and DTH was the chain-extender. Two polyurethanes were synthesized as is explained in detail below using this combination as shown in the Table 3 below.

Table 3

| Polyurethanes | Macrodiol | Diisocyanate | Chain Extender |
|---|---|---|---|
| Polymer A | PEG | HDI | DTH |
| Polymer B | PCL | HDI | DTH |

In one embodiment, the polyurethanes formed in accordance with the methods of the present invention are useful for various biomedical applications including, but not limited to, bio-scaffolding applications. Polyurethanes for biomaterial applications have been investigated for variety of applications. One criteria for such polyurethanes depends on the biocompatibility of the components used to form the polyurethane.

In one embodiment, the polyurethanes of the present invention are formed as mentioned above, and discussed in further detail below, using biocompatible polyols that include, but are not limited to, polyethylene glycol (PEG), polytetramethylene glycol (PTMG), polycaprolactone diol (PCL), or suitable combinations of two or more thereof. Several aromatic and aliphatic diisocyanates can be used in conjunction with the present invention. Such aromatic and aliphatic diisocyanates include, but are not limited to, 4,4'-diphenylmethane diisocyanate (MDI), toluene diisocyanate (TDI), hexamethylene diisocyanate (HDI), and suitable combinations of two or more thereof. Suitable chain extenders are 1,4-butanediol (BD), ethylenediamine (EA), desaminotyrosyl hexyl ester (DTH), or combinations thereof.

In one embodiment, as is mentioned above, the polyurethanes of the present invention are formed using desaminotyrosyl hexyl ester (DTH) as the chain extender. This permits the incorporation of an amino acid, or amino acid functionality, into the polyurethanes of the present invention. In another embodiment, the amino acid portion can be incorporated into the polyurethane structure as diisocyanate or the chain extender.

The synthesis of segmented polyurethanes involves two steps: (i) first the reaction of polydiol with diisocyanate in a stoichiometric ratio such that isocyanate terminated prepolymer is formed; and (ii) the reaction of the isocyanate terminated prepolymer with a low molecular weight diol or diamine compound to extend the chain.

Two different polyols are used in the present invention: polyethylene glycol ($M_w$ 1000) (PEG) and polycaprolactone diol ($M_w$ 1250) (PCL) because of the biocompatible characteristics of the segments formed therefrom. It should be noted that the present invention is not limited to just the compounds, or the molecular weights, given above. Instead a wide range of PEG and PCL molecular weights can be used in conjunction with the present invention to for a desired polyurethane.

In one embodiment, the diisocyanate used is aliphatic hexamethylene diisocyanate (HDI) due to its potential biocompatibility. The chain extender is desaminotyrosyl hexyl ester (DTH) is a diphenolic, dipeptide molecule based on L-tyrosine and its metabolite, desaminotyrosine (DAT).

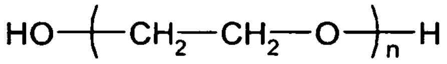
(PEG)

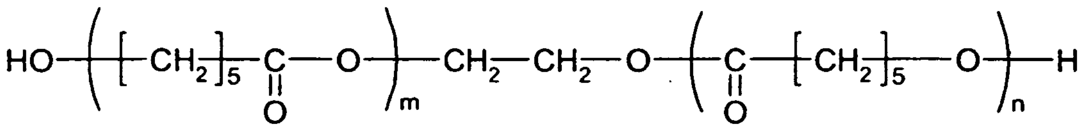
(PCL)

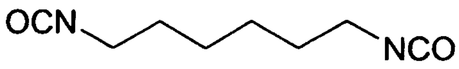
(HDI)

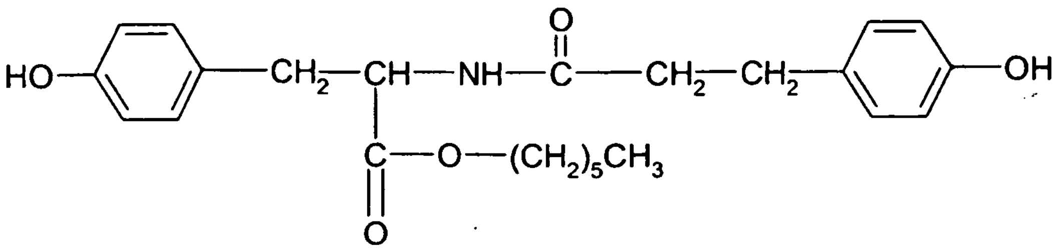
(DTH)

Synthesis of Polymer:

The synthesis of polymer involves two steps: (i) synthesis of the chain extender DTH and (ii) synthesis of the polyurethane. All the chemicals and solvents were used as received, unless otherwise stated and were purchased from Sigma Aldrich. Distilled water was used for all purposes.

DTH Synthesis:

The synthesis of DTH is known to those of ordinary skill in the art and is described in various literature sources. Briefly, DTH is synthesized from hexyl ester of L-tyrosine (TH) and desaminotyrosine through carbodiimide coupling reaction. The reaction steps are summarized below and scheme of the reaction is shown below.

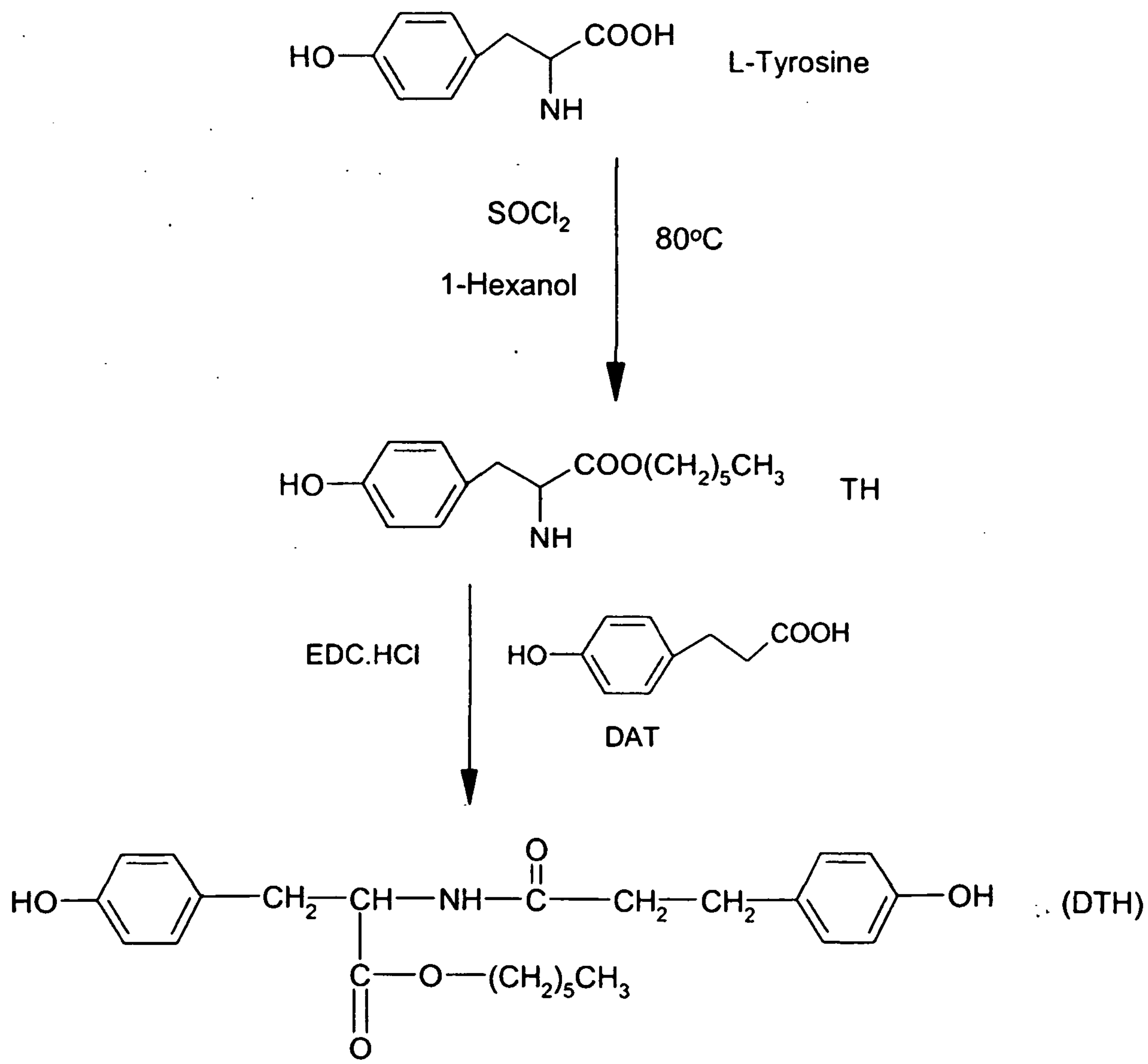


Step (i) The carboxylic acid group of the L-tyrosine (0.05 mole) is esterified by 1-hexanol (50 mL) in presence of thionyl chloride (0.05 mole) at 0°C initially, followed by reaction at 80°C for 12 hours. The reaction product obtained after cooling down the reaction to room temperature was completely precipitated in cold ethyl ether. The product was then filtered and washed with cold ether to obtain white solid, which is the chloride salt of hexyl ester of L-tyrosine.

Step (ii): The white solid was re-dissolved in distilled water and subsequently neutralized by 0.5 M sodium bicarbonate solution till the pH of the solution is slightly basic (pH-7.5). At this point solution turns turbid due to formation of tyrosine hexyl ester (TH). Tyrosine hexyl ester (TH) was extracted in ether, and the ether was evaporated to complete dryness to obtain tyrosine hexyl ester (TH) as an off-white solid.

Step (iii): Coupling of TH with DAT was mediated through hydrochloride salt of N-ethyl-N'-dimethylaminopropyl carbodiimide (EDC.HCl). Typically TH, DAT and EDC.HCl were added in equimolar proportion in 99% pure tetrahydrofuran (THF) as solvent at 0°C. After that, the reaction was allowed to continue at room temperature for 12 hours. At the end of 12 hours, the reaction mixture was poured into four times its volume of distilled water and was extracted in the organic phase by dichloromethane (DCM).

Step (iv): The organic DCM phase was washed with 0.1 N HCl solution, 0.1 N sodium carbonate solution and concentrated sodium chloride solution to remove the by products. The organic DCM phase was dried, and the solvent was evaporated under vacuum to obtain desaminotyrosyl tyrosine hexyl ester (DTH) as yellow, viscous oil.

Synthesis of Polyurethanes:

The synthesis of polyurethane is a condensation type polymerization typically involving the reaction of isocyanate

(-NCO) and hydroxyl (-OH) to form the carbamate (-NHCO) linkages. The polymerization is usually a two-step process leading to the formation of segmented polyurethane: (i) reaction of polyol with diisocyanate to form isocyanate terminated prepolymer and (ii) chain extension through the reaction of prepolymer and chain extender. Two different polyurethanes were synthesized using PEG and PCL as the polyol with HDI (diisocyanate) and DTH (chain extender). The reactions were carried out in a completely dry and moisture-free environment under inert (completely dry nitrogen, $N_2$) atmosphere. Both PEG and PCL were dried under vacuum for 48 hours at 40°C to remove entrapped water. N,N'-Dimethyl formamide (DMF) used as solvent, was dried over calcium hydride ($CaH_2$) followed by molecular sieve.

Diisocyanate of high (>99%) purity grade was used. The detailed protocol for the synthesis of polyurethane is summarized below:

Step (i): The polyol (PEG or PCL) was reacted with HDI at a 1 2 molar ratio in DMF as solvent and 0.1% stannous octoate catalyst to form the prepolymer. Typically, 5 mmol of polyol was added into 40 ml of DMF and 10 mmol of HDI and 2 to 3 drops of stannous octoate was added to the reaction mixture under dry and inert atmosphere with continuous stirring.

Step (ii): The temperature was increased to 110°C and the reaction was allowed for 3 hours at this temperature. After 3 hours, the reaction cooled down to room temperature (-25°C) with continuous stirring. The temperature of reaction was carefully maintained within the range of ±3°C.

Step (iii): DTH was added in the second step at a 1:1 molar ratio with the prepolymer. Typically, 5 mmol of DTH in 10 mL of DMF was added.

Step (iv): The temperature of reaction was then gradually increased to 80°C and the reaction was allowed to continue for 12 hours. The temperature of reaction was controlled within the range of ±3°C. After 12 hours the reaction was quenched by pouring the reaction into cold concentrated aqueous solution of sodium chloride. At this point, solid polyurethane polymer precipitates out from the reaction mixture.

Step (v): For PEG based polyurethanes, the polymer is suspended in the form of gel in the water. The final polymer is centrifuged out and re-suspended in water and then centrifuged. This process is repeated for at least three times to remove the impurities and unreacted materials. The final polymer is then dried in vacuum at 40°C for 48 hours. The polymer is yellowish white sticky solid. The nomenclature used for the PEG based polyurethane is PEG-HDI-DTH.

Step (iv): For PCL based polyurethanes, the polymer is suspended as solid polymer. The final polymer is filtered out and washed with water. This washing is repeated for at least three times to remove the impurities and unreacted materials. The final polymer is then dried in vacuum at 40°C for 48 hours. The polymer is yellowish white solid. The nomenclature used for the PCL based polyurethane is PCL-HDI-DTH.

The polyurethanes synthesized were stored is desiccators for the purpose of characterization and future experiments. The structure of the two polyurethanes is shown below.

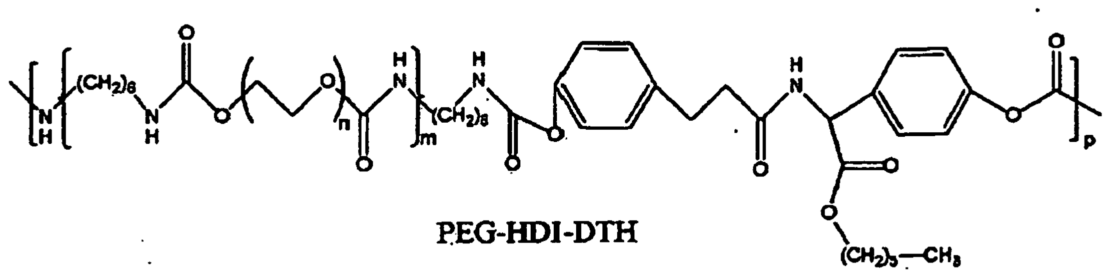

PEG-HDI-DTH

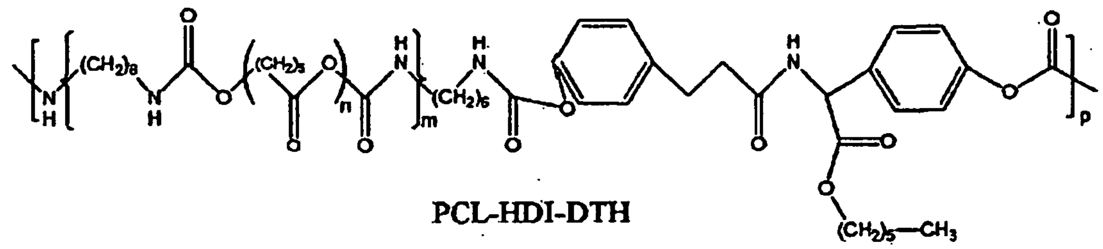

PCL-HDI-DTH

where n is an integer in the range of 5 to 25, m is an integer in the range of 1 to 4, and p is an integer in the range of 20 to 200. In another embodiment, m is equal to 1. In still another embodiment, n is an integer in the range of 7 to 22, or an integer from 10 to 20, or even an integer from 12 to 17. In still another embodiment, p is an integer in the range of 30 to 180, or an integer in the range of 50 to 175, or an integer from 75 to 150, or even an integer from 100 to 125. Here,

as well elsewhere in the specification and claims, individual range limits can be combined to form additional non-disclosed ranges.

In still yet another embodiment, m, n and p are selected so that the molecular weight of the above polyurethane compounds is in the range of 4,000 Da to 1,000,000 Da, or from 5,000 Da to 900,000 Da, or from 10,000 Da to 800,000 Da, or from 30,000 Da to 750,000 Da, or from 50,000 Da to 600,000 Da, or from 75,000 Da to 500,000 Da, or from 100,000 Da to 400,000 Da, or from 150,000 Da to 350,000 Da, or from 200,000 Da to 300,000, or even from 225,000 Da to 250,000 Da. Here, as well elsewhere in the specification and claims, individual range limits can be combined to form additional non-disclosed ranges. In another embodiment, m, n and p are selected so that the molecular weight of the PEG-HDI-DTH is about 98,000 Da. In another embodiment, m, n and p are selected so that the molecular weight of the PCL-HDI-DTH is 246,000 Da.

Characterization of Polymer:

The polymerization and the polyurethanes were characterized by various techniques to determine the structure and understand the basic properties of the polymers. The preliminary characterization studies include structural, thermal and mechanical characterization.\

Structural Characterizations:

The structural characterizations were done by $^{1}$H-NMR, $^{13}$C-NMR and FT-IR study. NMR was carried out in 300 MHz Varian Gemini instrument with d-dimethyl sulfoxide ($\delta$ = 2.50 ppm for $^{1}$H NMR and 39.7 ppm for $^{13}$C NMR as internal reference) solvent for PEG-HDI-DTH and &chloroform ($\delta$ = 7.27 ppm for $^{1}$H NMR and 77.0 ppm for $^{13}$C NMR as internal reference) for PCL-HDI-DTH. FT-IR analysis was performed with a Nicolet NEXUS 870 FT spectrometer for neat samples with 16 scans. FT-IR analysis was also used to study the progress of polymerization reaction. The molecular weights of polymers were determined by gel permeation chromatography (GPC) using tetrahydrofuran (THF) as solvent and polystyrene as internal standard. The solubility of the polymers was checked in a variety of solvents by dissolving 10 mg of solid polymer in 10 mL of the solvent at room temperature.

Thermal Characterizations:

The thermal behaviors of the polyurethanes were characterized by differential scanning calorimetry (DSC) and thermo gravimetric analysis (TGA). DSC was performed with a DSC Q100V7.0 Build 244 (Universal V3. 7A TA) instrument at a scanning rate of 10°C/min from -80°C to 250°C. TGA was performed with a TGA Q50V5.0 Build 164 (Universal V3. 7A TA) instrument from 0°C to 600°C under nitrogen atmosphere at a rate of 20°C/min. An average of 10 mg of solid sample was used for both the experiments.

Mechanical Characterizations:

The tensile properties of the polyurethanes films were measured by Instron Tensile Testing Machine with a load cell of 100 N and cross-head speed of 100 mm/min at room temperature. The films were cast from 10% wt solution of polymers (DMF for PEG-HDI-DTH and chloroform for PCL-HDI-DTH) and solvent was allowed to evaporate at room temperature and then subsequently dried in vacuum oven at 50°C for 48 hours to remove the residual solvent. The sample dimension was 20 mm x 6 mm x - 0.3 mm with a free length of 10 mm. The average of five measured values was taken for each sample.

Polymerization Reaction:

Table 4 summarizes the composition of the two polymers with the relative contribution of hard and soft segment. The yield for the synthesis of DTH was about 85% and for the polyurethanes was about 70 to 80%. The results were reproducible within a range of $\pm$5% with reasonable purity of the polyurethanes.

Table 4

| Polymer | Soft Segment Molecular Weight ($M_w$) | Soft Segment Content (Weight Percent) | Hard Segment Content (Weight Percent) | |
|---|---|---|---|---|
| | | | Diisocyanate (HDI) | Chain Extender (DTH) |
| PEF-HDI-DTH | 1000 | 57.5 | 19.3 | 23.2 |
| PCL-HDI-DTH | 1250 | 62.6 | 16.9 | 20.5 |

NMR Characterization:

The $^1$H NMR (along with the peak assignments) of PEG-HDI-DTH and PCL-HDI-DTH is shown in Figures 51 and 52, respectively. PEG-HDI-DTH: δ 0.8 ($CH_3$-in hexyl group, DTH), 1.2 (-$CH_2$ in hexyl chain in DTH), 1.3 (-$CH_2$- in hexyl chain in HDI), 1.4 (-NH-$CH_2$-$CH_2$- in HDI), 2.7 (-$CH_2$-$CH_2$-CO- in DTH), 2.9 (-NH-$CH_2$- in HDI and -$C_6H_4$-$CH_2$-$CH_2$- in DTH), 3.0 (-$C_6H_4$-$CH_2$-CH in DTH), 3.5 (-O-$CH_2$-$CH_2$-O- in PEG), 3.6 (-$CH_2$-$CH_2$-O-CO- in PEG), 4.0 (-CO-O-$CH_2$-$CH_2$- in DTH), 4.4 (-NH-CH- (CO)-$CH_2$- in DTH), 6.9 and 7.1 (two -$C_6H_4$- in DTH).

PCL-HDI-DTH: δ 0.8 ($CH_3$- in hexyl group, DTH), 1.2 to 1.7 ($CH_2$ in DTH, HDI and PCL), 2.3 (-CO-$CH_2$- in PCL), 2.8 (-$CH_2$-$CH_2$-CO- in DTH), 2.9 (-NH-$CH_2$-in HDI and -$C_6H_4$-$CH_2$- in DTH), 3.1 (-$C_6H_4$-$CH_2$-CH in DTH), 4.0 (-CO-O-$CH_2$-$CH_2$- in DTH and PCL), 4.8 (-NH-CH-(CO)-$CH_2$- in DTH), 6.7 and 6.9 (two -$C_6H_4$-in DTW).

The $^{13}$C NMR (along with the peak assignments) of PEG-HDI-DTH and PCL-HDI-DTH is shown in Figures 53 and 54, respectively. PEG-HDI-DTH: δ 13.9 (-CH3 in hexyl group, DTH), 21.9 to 27.9 ($CH_2$ in hexyl chain in DTH, HDI), 29.2 to 30.8 ($CH_2$ in hexyl chain in DTH, HDI), 36.0 (-$CH_2$-$CH_2$-CO in DTH), 37.5 (-$C_6H_4$-$CH_2$-CH in DTH), 54.6 (-NH-CH- (CO) -$CH_2$- in DTH), 62.9 (-$CH_2$-$CH_2$-O-CO-NH- in PEG), 64.8 (-$CH_2$-$CH_2$-CO- in DTH), 68.9 (-$CH_2$-$CH_2$-O-CO-NH- in PEG), 69.8 (-O-$CH_2$-$CH_2$-O- in PEG), 121.5 and 128.8 (two -$C_6H_4$- in DTH), 156.0 to 158.1 (-NH-CO-O- in urethane carbonyl), 171.6 (ester and amide carbonyls in DTH).

PCL-HDI-DTH: δ 14.2 ($CH_3$- in hexyl group, DTH), 22.7 to 28.6 ($CH_2$ in hexyl chain in DTH, HDI, PCL), 29.9 to 31.5 ($CH_2$ in hexyl chain in DTH, HDI), 34.1 (-$CH_2$-$CH_2$-CO in DTH), 34.3 (-$CH_2$-$CH_2$-CO-O in PCL), 40.5 (-$C_6H_4$-$CH_2$-CH in DTH), 53.5 (-NH-CH-(CO)-$CH_2$- in DTH), 64.3 (-CO-O-$CH_2$-$CH_2$- in PCL), 129.5 and 130.4 (two -$C_6H_4$- in DTH), 156.0 (-NH-CO-O- in urethane carbonyl) and 172.0 (ester and amide carbonyls in DTH), 173.7 (ester carbonyls in PCL).

The peak assignment from $^1$H and $^{13}$C NMR show that all the three components are present in the polymer chains. However due to the presence of similar chemical environments for certain protons and carbons, there is considerable overlap of the peaks which makes the assignment a difficult task. In general, for both the PEG- and PCL-based polyurethanes the presence of the characteristic peaks indicate that the polymers are composed of the corresponding soft segments along with HDI and DTH. Most important is the presence of urethane link indicated by the 2.9 ppm in $^1$H NMR and 156 ppm in $^{13}$C NMR for both in PEG- and PCL-based polyurethanes. This clearly shows that urethane linkages are formed by the condensation polymerization. However some unassigned peaks in the spectra correspond to materials formed by possible side reactions and from of unreacted materials/solvent. But the intensity of such peaks are considerably lower than the assigned peaks which indicates that polymers are of reasonable purity.

FT-IR Characterizations:

The FT-IR spectra of the polyurethanes are shown in Figure 55. The spectra of both the polymers show the characteristic peaks for the polyurethane. For PEG-HDI-DTH, the characteristic 1100 $cm^{-1}$ represents aliphatic ether linkage of the PEG segment and the peak around 1540 $cm^{-1}$ represent N-H bending/C-N stretching of urethane linkages and the amide linkage of DTH segment. Moreover, 1620 $cm^{-1}$ represents the aromatic stretch of DTH segment. The characteristic peaks in the region of 1715 to 1730 $cm^{-1}$ represents the carbonyl of the urethane linkages. The distribution of the carbonyl peak indicates a degree of hydrogen-bonding of urethane carbonyl group indicating interactions between different segments. The broad shoulder around 3330 $cm^{-1}$ is indicative of hydrogen bonded N-H stretching. For PCL-HDI-DTH, similar peaks are observed but the peaks around the region of approximately 1730 $cm^{-1}$ is masked due to strong carbonyl absorption of caprolactone unit of PCL. The FT-IR analysis supports the structure of the polyurethanes.

The FT-IR of the starting materials, intermediate prepolymer and the final polymer is shown together in Figure 56. The immergence of peaks around 1500 to 1700 $cm^{-1}$ represents formation of urethane bonds in the prepolymers compared to PEG and PCL. The peak at approximately 1630 $cm^{-1}$ represents the stretching of C=O (amide I) and 1540 $cm^{-1}$ represents N-H bending vibrations (amide II) indicating the formation of urethane linkages. The peak at 2280 $cm^{-1}$ comparable to the isocyanate peak of HDI indicates that both the prepolymers are isocyanate terminated. The addition of DTH results in the complete disappearance of the isocyanate peaks at 2280 $cm^{-1}$ in the final polymer which indicates completion of reaction to the formation of final polyurethane. Moreover, the peak around approximately 1620 $cm^{-1}$ in the final polymer indicates C=C of aromatic ring structures of DTH. The peak at approximately 1715 $cm^{-1}$ represents combined free non hydrogen bonded C=O in amide I of urethane and amide (in DTH) and shoulder at 1740 $cm^{-1}$ represents ester C=O of DTH in PEG-HDI-DTH. Similarly, approximately 1715 $cm^{-1}$ represents combined free non hydrogen bonded C=O of amide I of urethane and amide (in DTH) and at 1730 $cm^{-1}$ represents ester C=O of caprolactone unit and DTH in PCL-HDI-DTH.

Table 5 summarizes the molecular weight of the polymers which shows that both the polyurethanes have significantly high molecular weight. Compared to the molecular weight of PEG and PCL as starting material, the molecular weight of the final polymers indicates the formation of polyurethanes. The low poly-dispersity indices of the polyurethanes indicate that the distribution of molecular weight is not broad and the polymerization is controlled. However, PEG based

polyurethanes are lower in molecular weight compared to PCL based polyurethanes. While not wishing to be bound to any one theory, this is probably due to presence of residual water in precursor PEG which inhibits high molecular weight of polymer by reacting away the diisocyanate. Considering different factors that contribute to the molecular weight of polymers in solution polymerization, these results were reproducible within range of ±10%.

Table 5

| Polymer | $M_n$ ($10^3$) | $M_w$ ($10^3$) | Poly Dispersity Index |
|---|---|---|---|
| PEG-HDI-DTH | 79 | 98 | 1.24 |
| PCL-HDI-DTH | 150 | 246 | 1.64 |

Solubility of the Polyurethanes:

Table 6 shows the solubility features of the polyurethanes in the common solvents.

Table 6

| Solvent/Polymer | PEG-HDI-DTH | PCL-HDI-DTH |
|---|---|---|
| Methylene Chloride | Almost Soluble | Almost Soluble |
| Chloroform | Almost Soluble | Soluble |
| DMF (Dimethyl Formamide) | Soluble | Almost Soluble |
| THF (Tetrahydrofuran) | Soluble | Soluble |
| Methanol | Insoluble | Insoluble |
| Ethanol | Insoluble | Insoluble |
| Ethyl Acetate | Insoluble | Insoluble |
| Acetone | Insoluble | Insoluble |

The solubility of the polymers shows that the polyurethanes are soluble in polar aprotic solvents and insoluble in water and protic solvents. The polyurethanes are also insoluble in acetone, ethyl acetate which is polar and aprotic, indicating that the different phases of the polyurethanes contribute differently towards solubility. But in general, the solubility features indicate that the polyurethanes are soluble for practical purposes.

Thermal Characterizations:

The DSC thermograms of the polyurethanes are shown in Figure 57. The differential scanning calorimetry (DSC) analysis of both the polymers indicates information regarding the morphology of the polyurethane structure. The biphasic morphology of the polyurethane is due to the presence of soft and hard segment. Considerable phase mixing or segregation occurs due to the difference in the compatibility of the segments. The compatibility of the segments arises from different interactions including, but not limited to, hydrogen bonding, dipolar interactions, van der Waals interaction, etc.

The DSC thermograms of the polyurethanes show distinct glass transition ($T_g$) at -40°C for PEG-HDI-DTH and at -35°C for PCL-HDI-DTH which correspond to the soft segment glass transition temperature. The shift from the $T_g$'s of the pure homopolymer Tg's (-67°C for PEG and -62°C for PCL) indicates some degree of phase mixing between the soft and hard segment of the polyurethanes. For PEG-HDI-DTH, three additional endotherms were observed: at 0, 50 and 162°C. Similar endotherms are also observed for PCL-HDI-DTH at 5, 52 and 173°C with an additional one at 31 °C. The absence of hard segment $T_g$ indicates that hard segments are relatively crystalline domains due to presence of aromatic ring structure in the back bone of polymer. It has been observed a hard segment $T_g$ that is probably due to amorphous hard segment with aromatic group as pendant groups from the backbone of the polymer.

Moreover, absence of melting endotherms for the phenyl alanine based polyurethanes indicates that the hard segment is largely amorphous. The endotherms at 162°C represent the melting of the microcrystalline hard segment domain while the other transitions at 0 and 50°C represents the dissociation of short range and long range order of the hard segment domain. Short range order of polyurethane actually represents the interaction between the soft segment and hard segment that actually contributes to the phase mixing behavior of the polyurethane. Long range order represents

'unspecified' interactions within the hard segment domain. Absence of soft segment melting endotherm for PEG-HDI-DTH indicate the amorphousness of the soft segment. The crystallinity of PEG is reduced due to the presence of hard segment at the PEG chain ends and due to partial dispersion of the hard segment within the soft segment of the polyurethane. The low molecular weight of PEG and high hard segment content in PEG-HDI-DTH favors this feature. Similar observations for PTMO based polyurethanes and phenyl alanine based polyurethanes are made. The similar endotherms for PCL-HDI-DTH at 173°C represent the melting of the microcrystalline hard segment domain while the other transitions at 0 and 52°C represents the dissociation of short range and long range order of the hard segment domain respectively.

The additional endotherm at 31 °C is probably due to the melting of soft segment. PCL being relatively more crystalline shows melting due to chain mobility at this temperature. The crystallinity of PCL soft segment is less affected in spite of phase mixing due to the dipolar interaction of ester bonds and relatively lower hard segment content. The phase mixing phenomenon is present in both the polyurethanes but PCL based polyurethane exhibits comparatively lesser degree of mixing than PEG-based polyurethane. The crystalline PCL soft segment is more cohesive in nature which prevents the mixing of hard and soft segment at the molecular level whereas relatively amorphous and non-polar PEG soft segment provides more integration in between the different segments. These characteristic features of the polyurethanes indicate that two phase morphology of the polyurethanes are present with variable degree of phase mixing/ segregation behavior. The relative crystallinity of the polymers is mainly contributed by the H-bonded hard segment. The DSC analysis of the polyurethanes provides significant information about phase morphology of the polyurethanes.

The thermogravimetric analysis (TGA) analysis of the polyurethanes is shown in Figure 58. The TGA analysis shows that these polymers are thermally stable as the onset of degradation for PEG-HDI-DTH is around 250°C and that for PCL-HDI-DTH is around 300°C. The earlier onset for PEG based polyurethane is probably due to associated water molecules of the PEG soft segment. Both the polyurethanes exhibit two stage degradation which is qualitatively in agreement with the two phase structure of the polyurethanes.

The melting of the polymers is at relatively lower temperature compared to pure poly-tyrosine indicates its applicability in the processing of the material for practical purposes of scaffolding in tissue engineering applications. The high degradation temperature indicates that the range of temperature within which the polymers are processible is sufficiently large.

Mechanical Characterizations:

The typical stress-strain curve of the polyurethanes is shown in Figure 59. The tensile properties of the polyurethanes are summarized in Table 7.

The mechanical properties of polyurethanes show that PEG based polyurethane is lower in mechanical strength compared to PCL based polyurethane. The mechanical properties of the polyurethanes are mainly controlled by the dominant soft segments. The lower tensile strength, modulus of elasticity and elongation (at break) of PEG-HDI-DTH is largely due to amorphous and flexible PEG soft segment compared to relatively more crystalline PCL. The contribution of hard segment is relatively less due to phase mixing of the hard segment with the soft segment. Thus, the mechanical properties of the polyurethanes are more controlled by the soft segment morphology. The difference in the mechanical properties of the polyurethanes can be directly correlated to structure and morphology of the polyurethanes. Polyurethanes with higher degree of phase separation exhibits better tensile properties than the phase mixed polyurethanes. This is probably due to disordering of hard segment domains. As indicated by DSC analysis, crystalline PCL soft segment inhibits phase mixing and therefore leads to more phase segregated morphology leading to higher tensile properties. In addition to this, the effect of molecular weight is directly related to the tensile property. PCL based polyurethane have significantly higher molecular weight which improves the tensile properties compared to the PEG based polyurethane. Moreover, the high hydrophilicity of PEG often leads to lower mechanical property of the polymer.

Results and Discussion:

Preliminary physical and chemical characterization indicates that polyurethanes can be synthesized using DTH as the chain extender. $^{1}H$ and $^{13}C$ NMR shows the presence of aromatic moieties which conclusively proves the inclusion of DTH as the chain extender. Moreover, the IR characterization shows appearance of urethane, and amide groups (1650 to 1700 $cm^{-1}$) for the polymer. The GPC analysis primarily concludes the polymerization process with sufficiently high molecular weight and relatively narrow molecular weight distribution. The solubility studies show that these polymers are partially to completely soluble in most the solvents.

The thermal characterization studies indicates both the polymers have melting temperature around 150°C (from DSC analysis), whereas the onset of the decomposition is around 300°C (from TGA analysis). These results indicate the wide thermal range for the processing of the material.

The hydrolytic degradation of the polymer at physiological pH 7.4 and body temperature 37°C shows that PEG

based polyurethanes are degradable under these conditions whereas PCL based polyurethanes are potentially less degradable under similar conditions. These results were further supported by the low water uptake of PCL based polyurethanes (approximately 5%) compared to their PEG (approximately 70%) based counterpart.

Conclusions:

The preliminary results of the L-tyrosine based polyurethanes indicate that these polymers can be synthesized easily by two-step methods. The characterization results indicate that these materials are suitable for biomaterial applications. Further and elaborate characterizations, including mechanical and biological properties of these polymers are currently under investigation for tissue engineering applications. This invention shows that L-tyrosine based DTH can be used as chain extender for the synthesis of polyurethanes. These polymers have the potential for biomaterial applications.

L-tyrosine-based phosphate polymers can be synthesized that degrade over shorter period of times, for example, in less than 20 days, less than 15 days, or even less than 7 to 10 days. On the other hand, the present invention also makes it possible to synthesize L-tyrosine-based urethane polymers that degrade over a period of several months to one year. In another embodiment, the present invention makes it possible to form copolymers of L-tyrosine-based phosphate polymers and L-tyrosine-based urethane polymers thereby permitting one to further control the degradation rate thereof.

Blends:

The present disclosure relates to a blended L-tyrosine polyphosphate polymer with a L-tyrosine polyurethane polymer. Depending upon the composition of each component in the blend it is possible to achieve a wide variety of degradation times. While not wishing to be bound to any one embodiment, as a general rule the L-tyrosine polyphosphate polymers of the present invention degrade in less time (e.g., usually less than about 2 weeks, less than about 7 days) that the L-tyrosine polyurethane polymers disclosed herein (degradation time of about 1 months to several months or longer). Thus, based upon the percentage of each in a polymer blend one could achieve a wide range of desired degradation times for a wide variety of biomedical applications.

## Claims

1. A composition for targeted delivery of nucleic acids to cells comprising:

   at least one synthetic polymeric micro- or nano-capsule, wherein the capsule comprises one or more materials selected from poly(lactide-co-glycolide), L-tyrosine polyphosphate, L-tyrosine polyurethane, or any combination thereof;
   at least one targeting moiety disposed on the surface of the capsule and available for binding to target molecules, wherein the targeting moiety comprises any moiety capable of specifically binding with target molecules selected from antibodies, antibody fragments, antigens, transmembrane proteins, glycoproteins, and any combination thereof;
   an additive for enhancing biocompatibility selected from one or more of PEG-g-chitosan and amphiphilic PEG species; and
   an additive for assisting in DNA transport across a cell membrane selected from one or more of linear polyethylenimine, PEG-g-chitosan, and any combination thereof.

2. The composition of claim 1, wherein the L-tyrosine polyurethane comprises at least one polymer composition having a formula shown below:

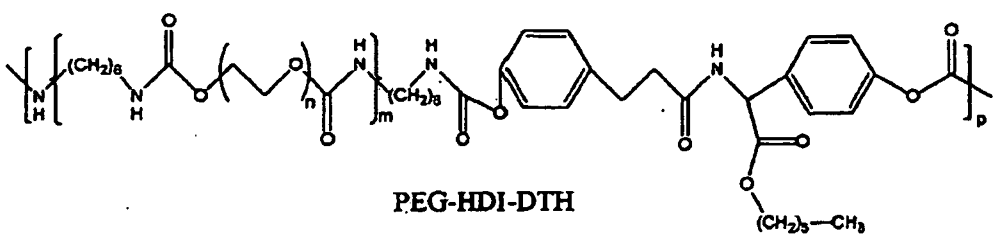

PEG-HDI-DTH

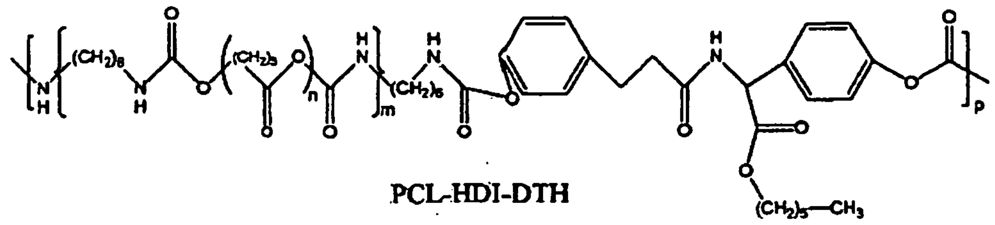

PCL-HDI-DTH

wherein n is an integer in the range of 5 to 25, m is an integer in the range of 1 to 4, and p is an integer in the range of 20 to 200.

**3.** The composition of claim 1, wherein the L-tyrosine polyurethane comprises at least one polymer composition having a formula shown below:

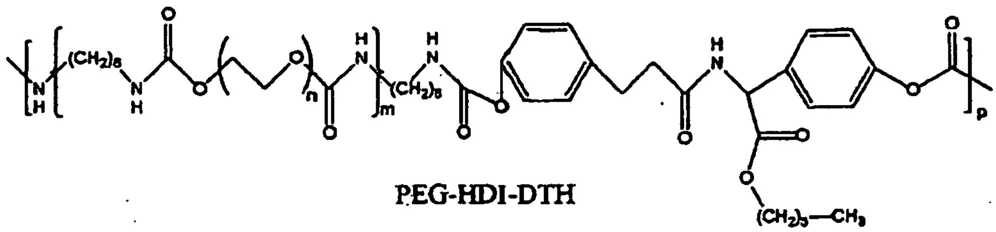

PEG-HDI-DTH

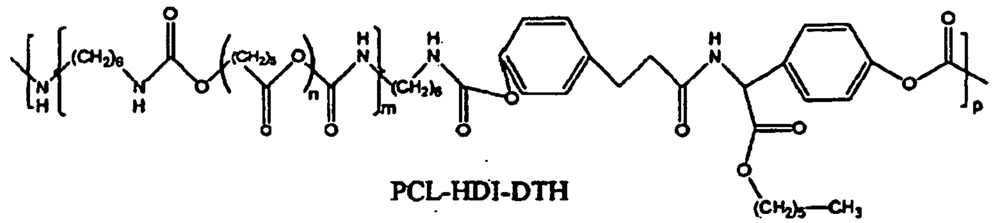

PCL-HDI-DTH

wherein the number of repeating units, m, n and p, are selected so that the molecular weight of the above polyurethane compounds are in the range of about 4,000 Da to 1,000,000 Da.

**Patentansprüche**

**1.** Zusammensetzung zur gezielten Abgabe von Nukleinsäuren an Zellen, umfassend:

wenigstens eine synthetische, polymerische Mikro- oder Nano-Kapsel, wobei die Kapsel ein oder mehrere Materialien umfasst, die ausgewählt sind aus Poly(lactid-co-glycolid), L-Tyrosinpolyphosphat, L-Tyrosinpolyurethan oder einer beliebigen Kombination davon;

wenigstens einen Zielrest, der an der Oberfläche der Kapsel angeordnet ist und zum Binden an Zielmoleküle zur Verfügung steht, wobei der Zielrest einen beliebigen Rest umfasst, der in der Lage ist, sich spezifisch mit Zielmolekülen zu verbinden, die ausgewählt sind aus Antikörpern, Antikörperfragmenten, Antigenen, Transmembranproteinen, Glycoproteinen und jeder beliebigen Kombination davon;

einen Zusatzstoff zur Verbesserung der Biokompatibilität, ausgewählt aus einem oder mehreren der PEG-g-Chitosan- und amphiphilen PEG-Spezies; und

einen Zusatzstoff zur Unterstützung beim DNA-Transport über die Zellmembran, ausgewählt aus einem oder mehreren von linearem Polyethylenimin, PEG-g-Chitosan sowie jeder beliebigen Kombination davon.

**2.** Zusammensetzung nach Anspruch 1, wobei das L-Tyrosinpolyurethan wenigstens eine Polymerzusammensetzung mit einer Formel umfasst, die nachfolgend dargestellt ist:

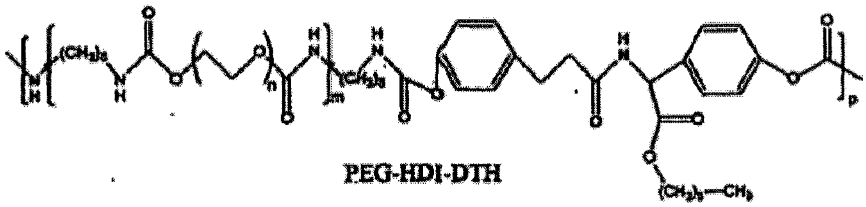

PEG-HDI-DTH

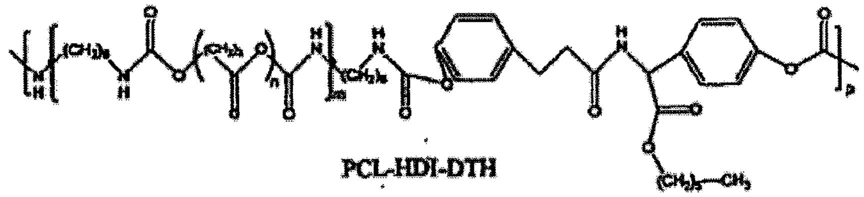

PCL-HDI-DTH

wobei n eine Ganzzahl im Bereich von 5 bis 25 ist, m eine Ganzzahl im Bereich von 1 bis 4 ist und p eine Ganzzahl im Bereich von 20 bis 200 ist.

3. Zusammensetzung nach Anspruch 1, wobei das L-Tyrosinpolyurethan wenigstens eine Polymerzusammensetzung mit einer Formel umfasst, die nachfolgend dargestellt ist:

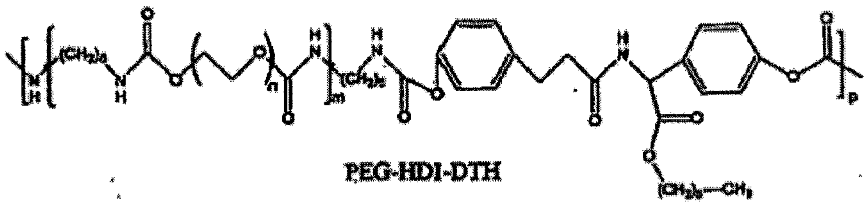

PEG-HDI-DTH

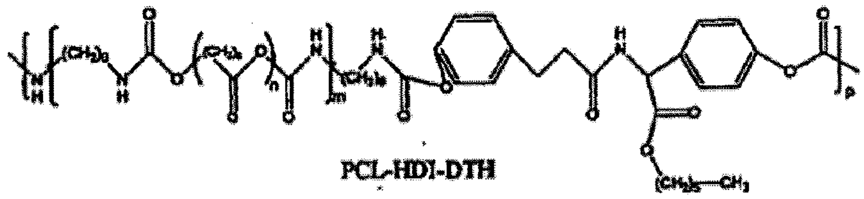

PCL-HDI-DTH

wobei die Anzahl von Wiederholungseinheiten, m, n und p derart ausgewählt ist, dass das Molekulargewicht der oben genannten Polyurethanverbindungen im Bereich von etwa 4.000 Da bis 1.000.000 Da liegt.

## Revendications

1. Composition pour la délivrance ciblée d'acides nucléiques à des cellules comprenant :

au moins une micro- ou nano-capsule polymère synthétique, dans laquelle la capsule comprend un ou plusieurs matériaux choisis parmi le poly(lactide-co-glycolide), le polyphosphate de L-tyrosine, le polyuréthane de L-tyrosine ou une quelconque combinaison de ceux-ci ;

au moins une fraction de ciblage disposée sur la surface de la capsule et disponible pour se lier à des molécules cibles, dans laquelle la fraction de ciblage comprend une quelconque fraction capable de se lier spécifiquement à des molécules cibles choisies parmi les anticorps, les fragments d'anticorps, les antigènes, les protéines transmembranaires, les glycoprotéines et une quelconque combinaison de ceux-ci ;

et un additif pour augmenter la biocompatibilité choisi parmi une ou plusieurs espèces de PEG-g-chitosan et de PEG amphiphile ; et

un additif pour aider au transport d'ADN à travers une membrane cellulaire choisie parmi un ou plusieurs de la polyéthylènimine linéaire, du PEG-g-chitosan et d'une quelconque combinaison de ceux-ci.

**2.** Composition selon la revendication 1, dans laquelle le polyuréthane de L-tyrosine comprend au moins une composition de polymère ayant une formule montrée ci-dessous :

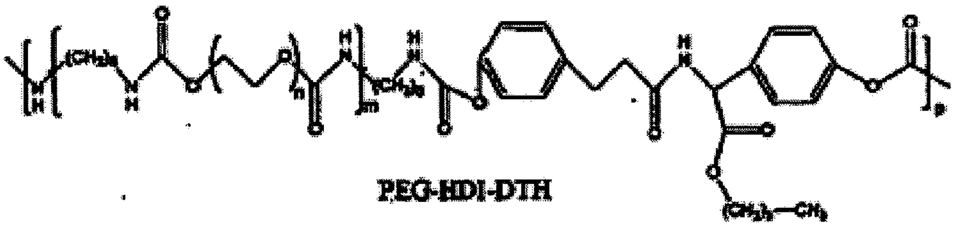

PEG-HDI-DTH

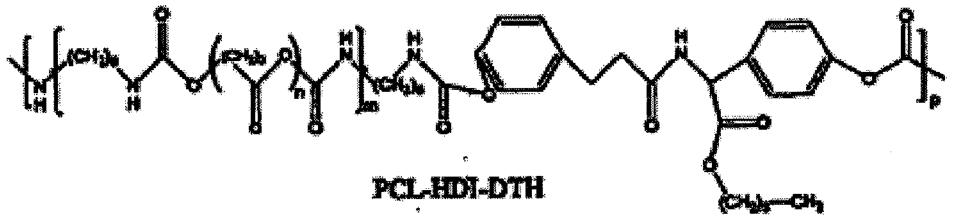

PCL-HDI-DTH

dans laquelle n est un nombre entier dans la plage de 5 à 25, m est un nombre entier dans la plage de 1 à 4 et p est un nombre entier dans la plage de 20 à 200.

**3.** Composition selon la revendication 1, dans laquelle le polyuréthane de L-tyrosine comprend au moins une composition de polymère ayant une formule montrée ci-dessous :

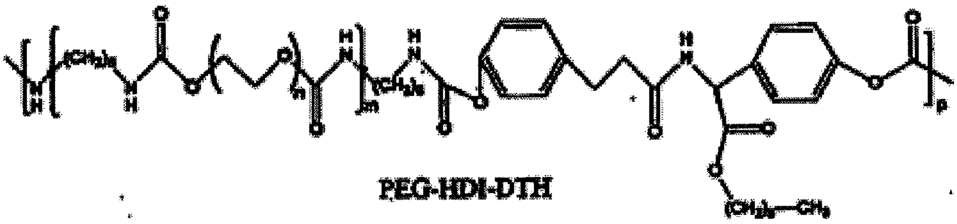

PEG-HDI-DTH

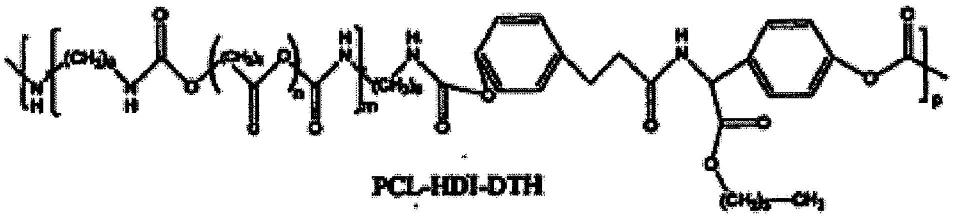

PCL-HDI-DTH

dans laquelle le nombre de motifs répétés m, n et p est choisi de telle manière que la masse moléculaire des composés de polyuréthane ci-dessus se situe dans la plage d'environ 4000 Da à 1000000 Da.

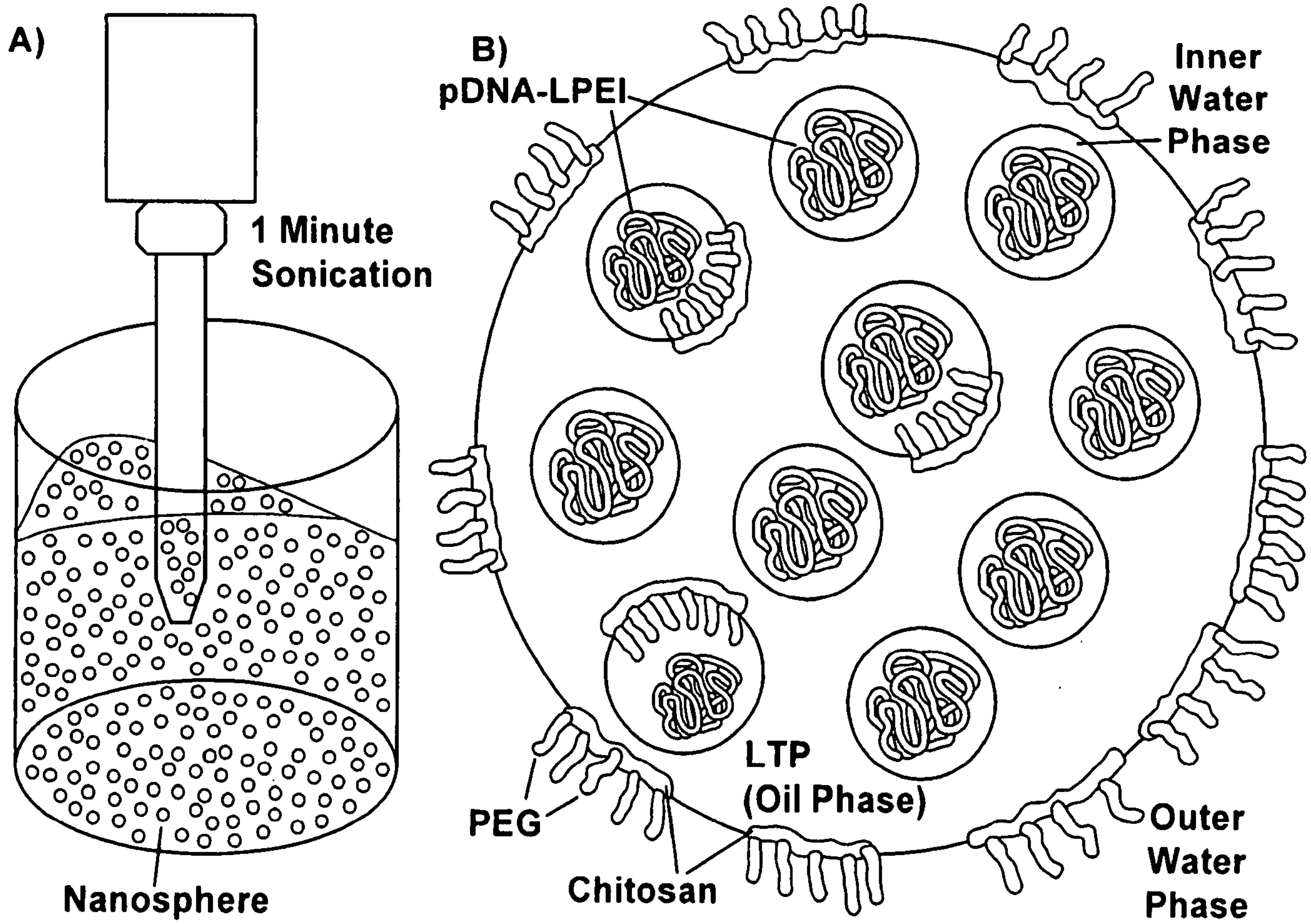


*A) Nanosphere formation by sonication emulsion,*
*B) Hypothesized Nanosphere Composition*

**FIG. 1**

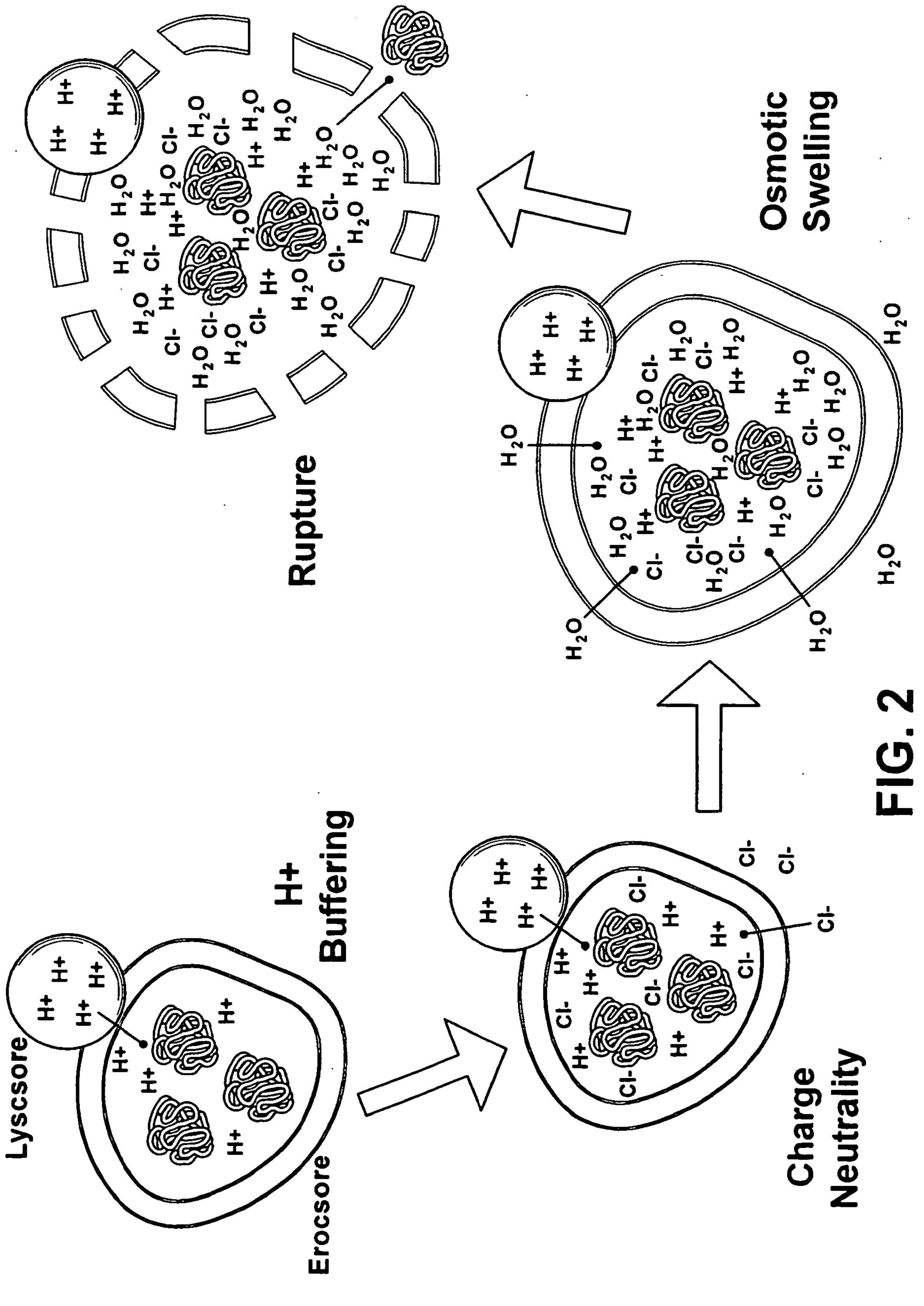
Lyscsore
Erocsore
H+ Buffering
Charge Neutrality
Osmotic Swelling
Rupture

FIG. 2

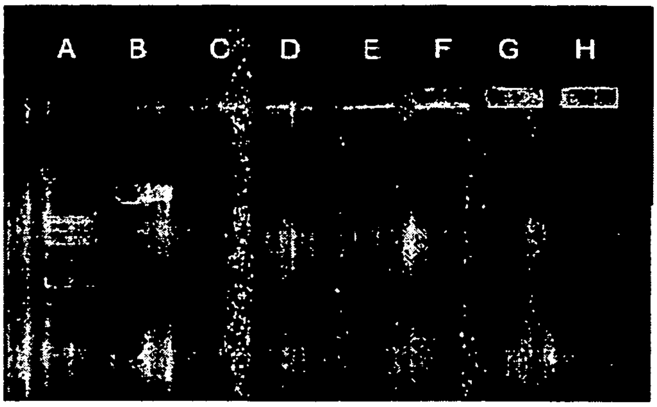


Figure 4.1.1.1: *Gel electrophoresis of A) 1 kb DNA ladder, B) stock pDNA, C-E) 1 minute sonicated pDNA-LPEI, F-H) stock pDNA-LPEI*

FIG. 3

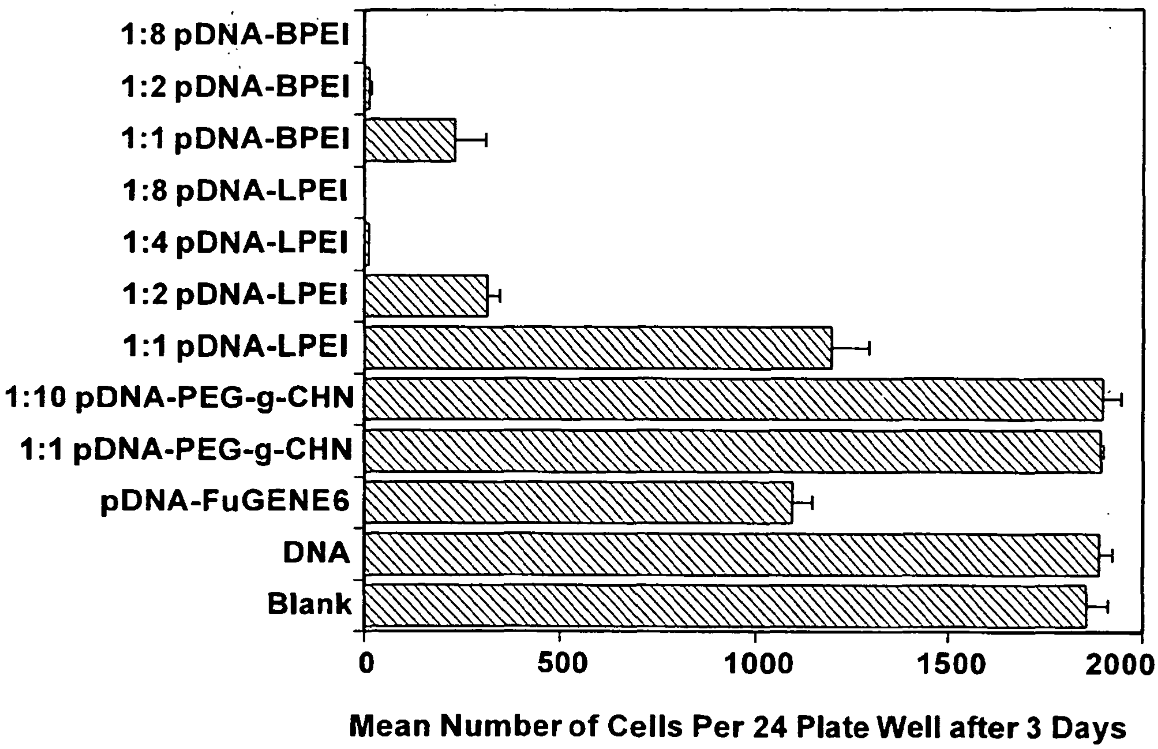


*Mean number of cells in 24 well culture plates transfected with various pDNA-LPEI, BPEI, and PEG-g-CHN complex mass ratios after 3 days.*

**FIG. 4**

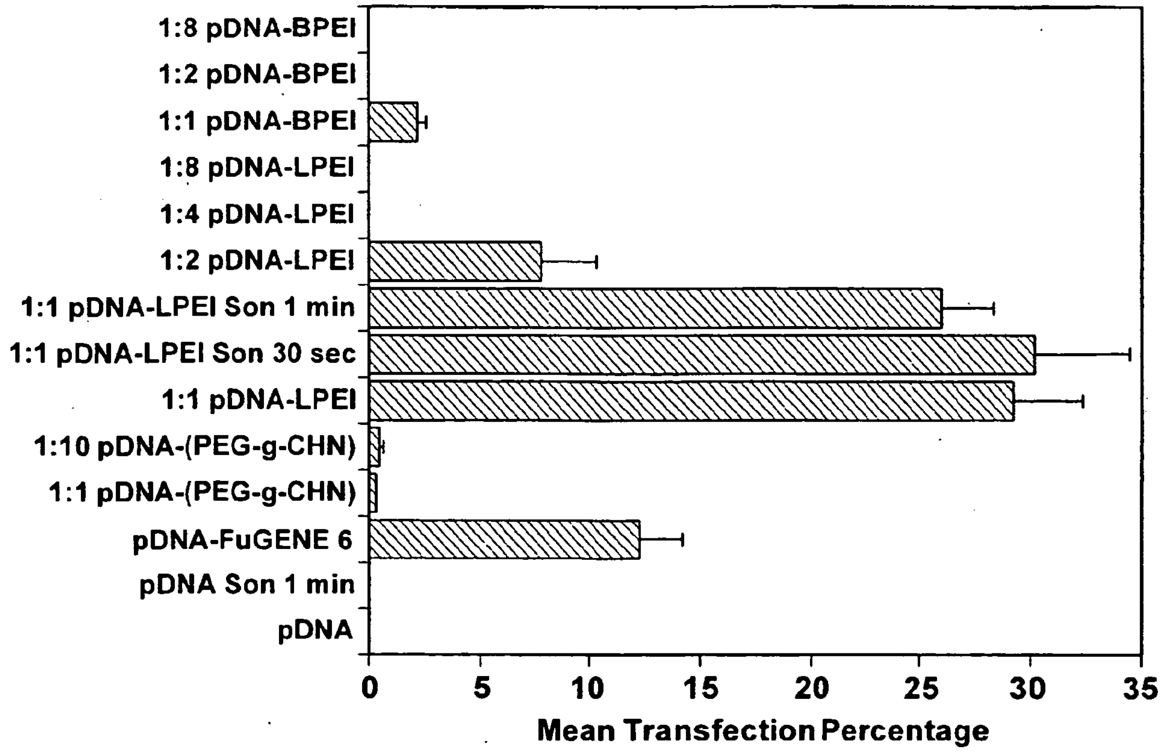


*Transfection percentage of human fibroblast cells with pDNA compexed with various mass ratios of polymers.*

# FIG. 5

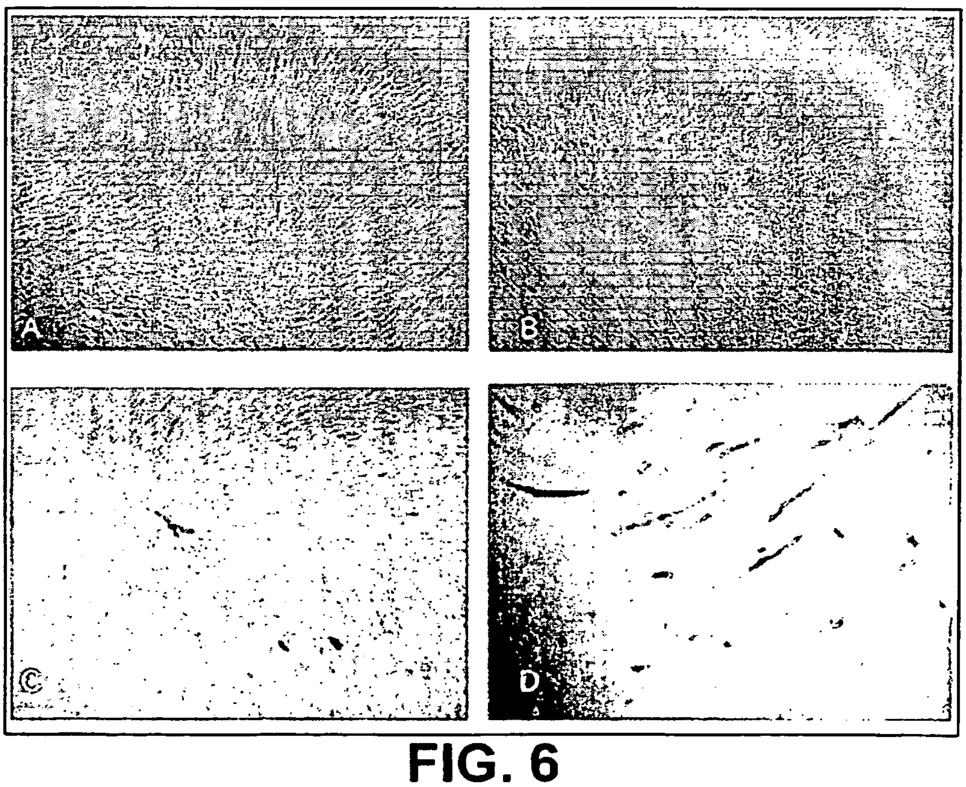


# FIG. 6

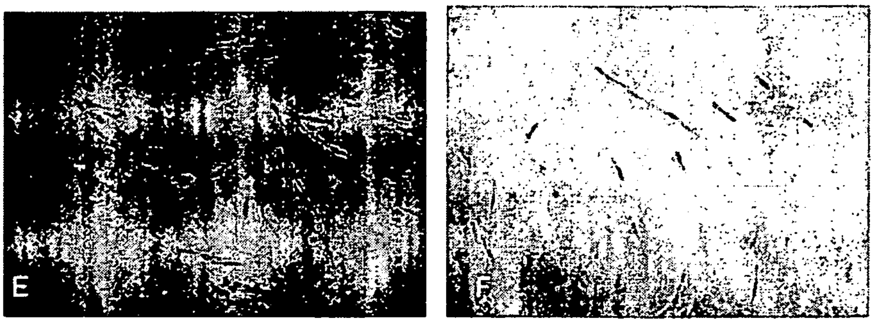


Figure 4.1.2.3: *Transfection of human fibroblast cells from A) Blank Cells, B) 1 minute sonicated pDNA, C) pDNA with FuGENE 6, D) PDNA-LPEI, E) 30 second sonicated pDNA-LPEI, F) 1 miute sonicated pDNA-LPEI*

**FIG. 6**

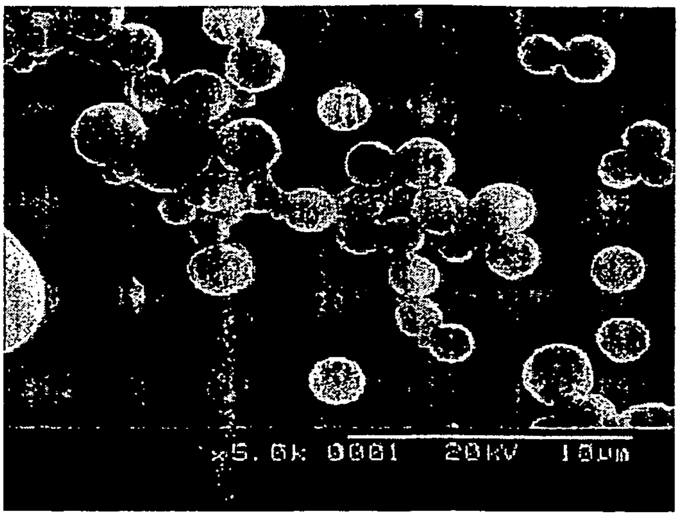


Figure 4.2.1: *SEM of 1% complexed pDNA nanospheres (5,000x magnification).*

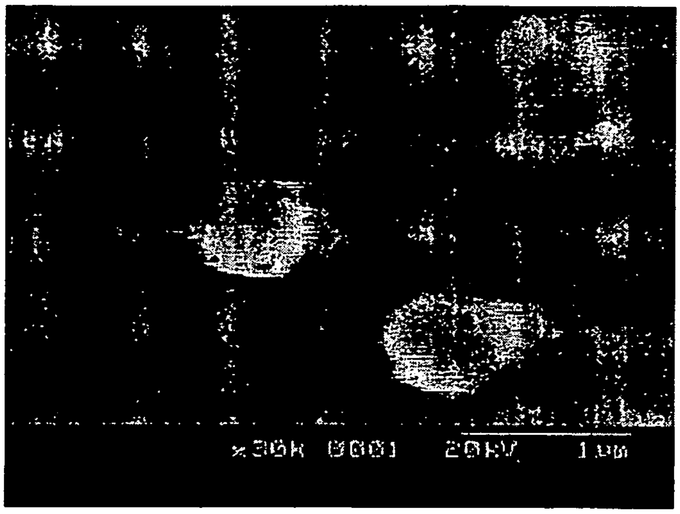


Figure 4.2.2: *SEM of 1% complexed pDNA nanospheres (30,000x magnification).*

**FIGS. 7 and 8**

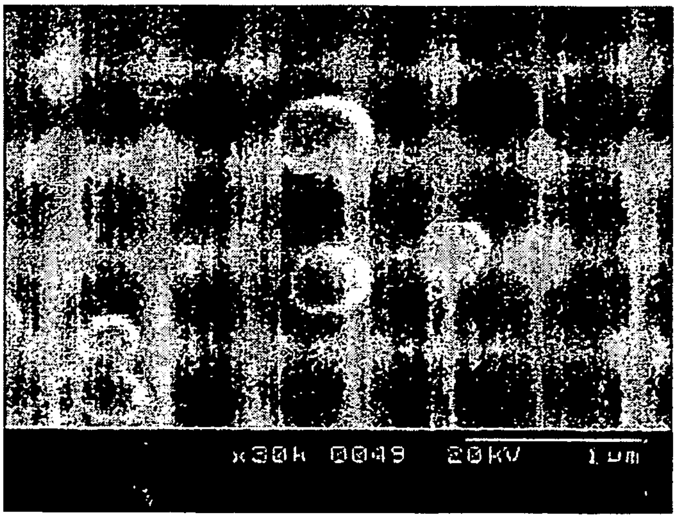


Figure 4.2.3: *SEM of blank nanospheres (30,000x magnification).*

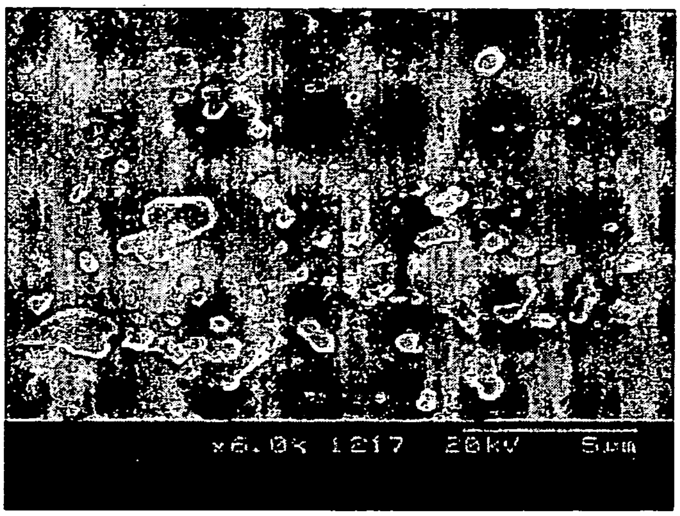


Figure 4.2.4: *SEM of impeller formed 1% complexed pDNA nanospheres (30,000x magnification).*

**FIGS. 9 and 10**

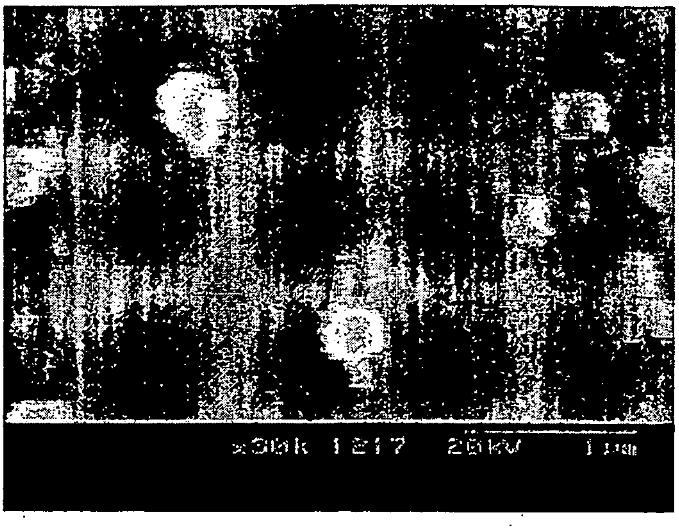


Figure 4.2.5: *SEM of 10% complexed pDNA nanospheres (30,000x magnification).*

FIG. 11

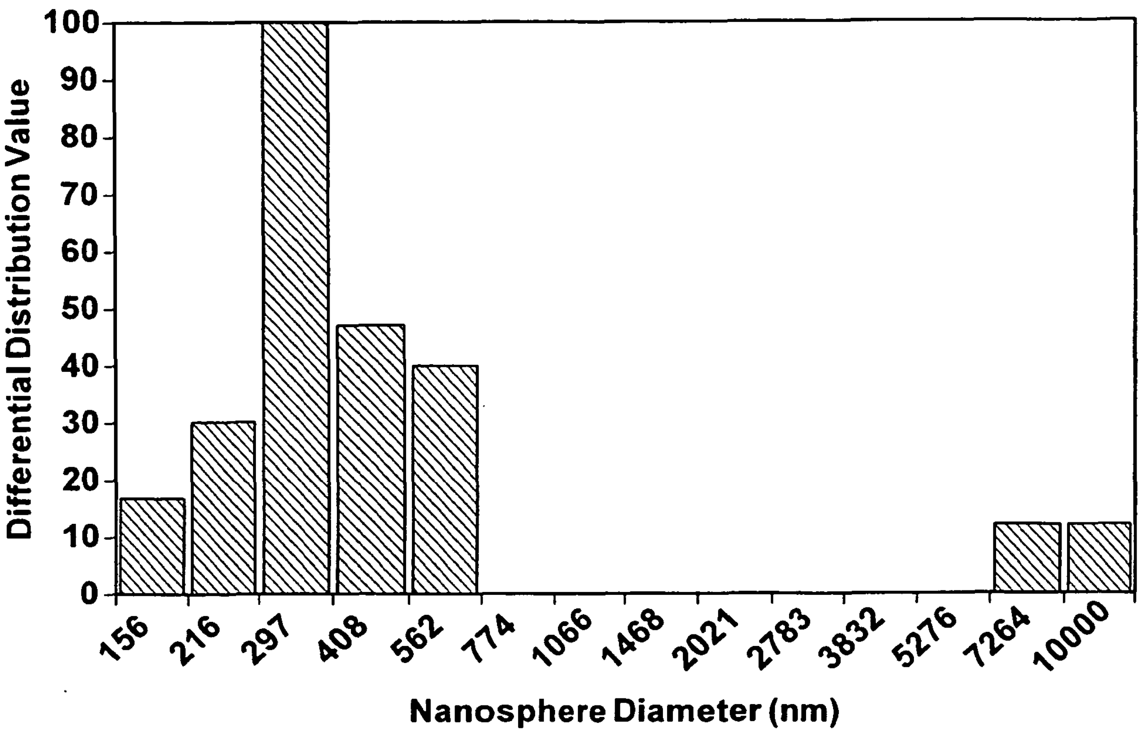


*Representative size distribution of blank pDNA nanospheres. Determined using Regularized Non-negatively Constrained Least Squares (Contin).*

# FIG. 12

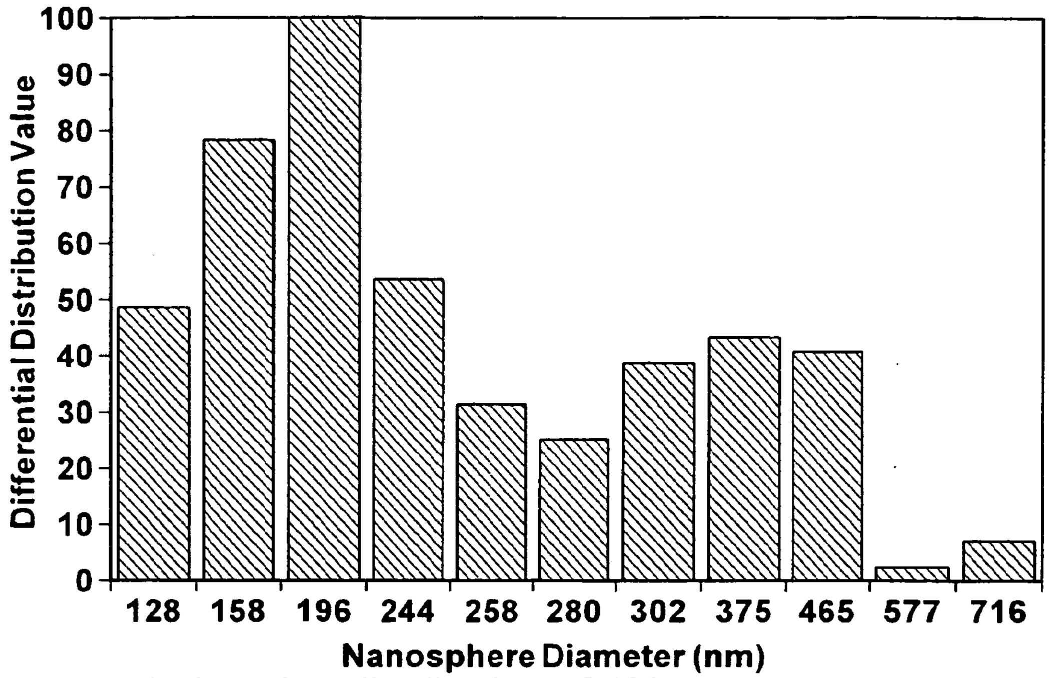


*Representative size distribution of 1% complexed pDNA nanospheres. Determined using Regularized Non-negatively Constrained Least Squares (Contin).*

**FIG. 13**

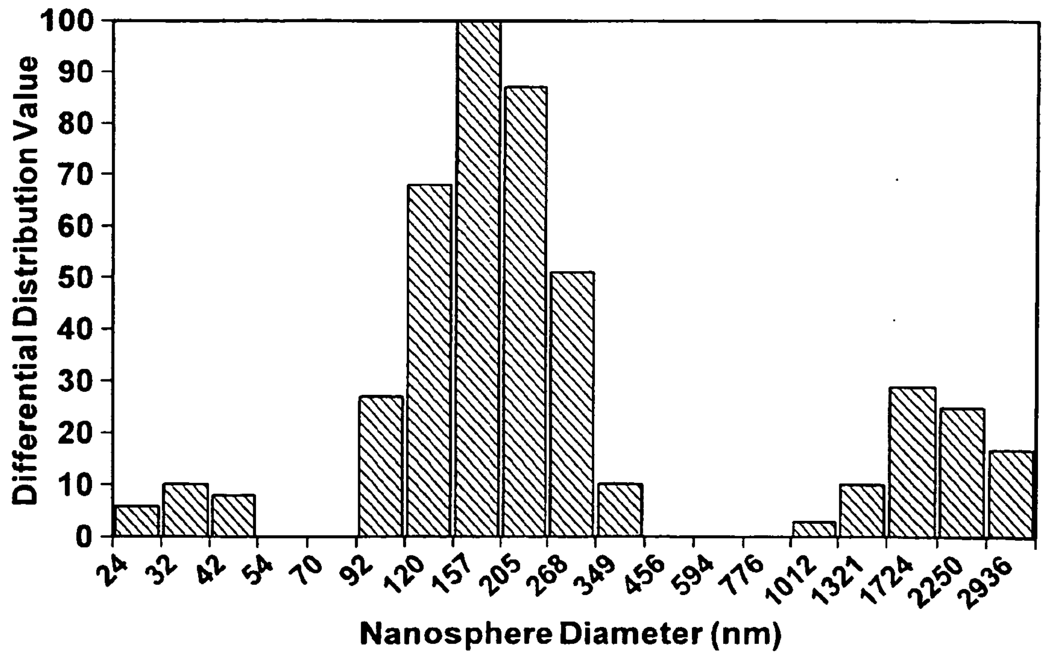


*Representative size distribution of impeller formed 1% complexed pDNA nanospheres. Determined using Regularized Non-negatively Constrained Least Squares (Contin).*

**FIG. 14**

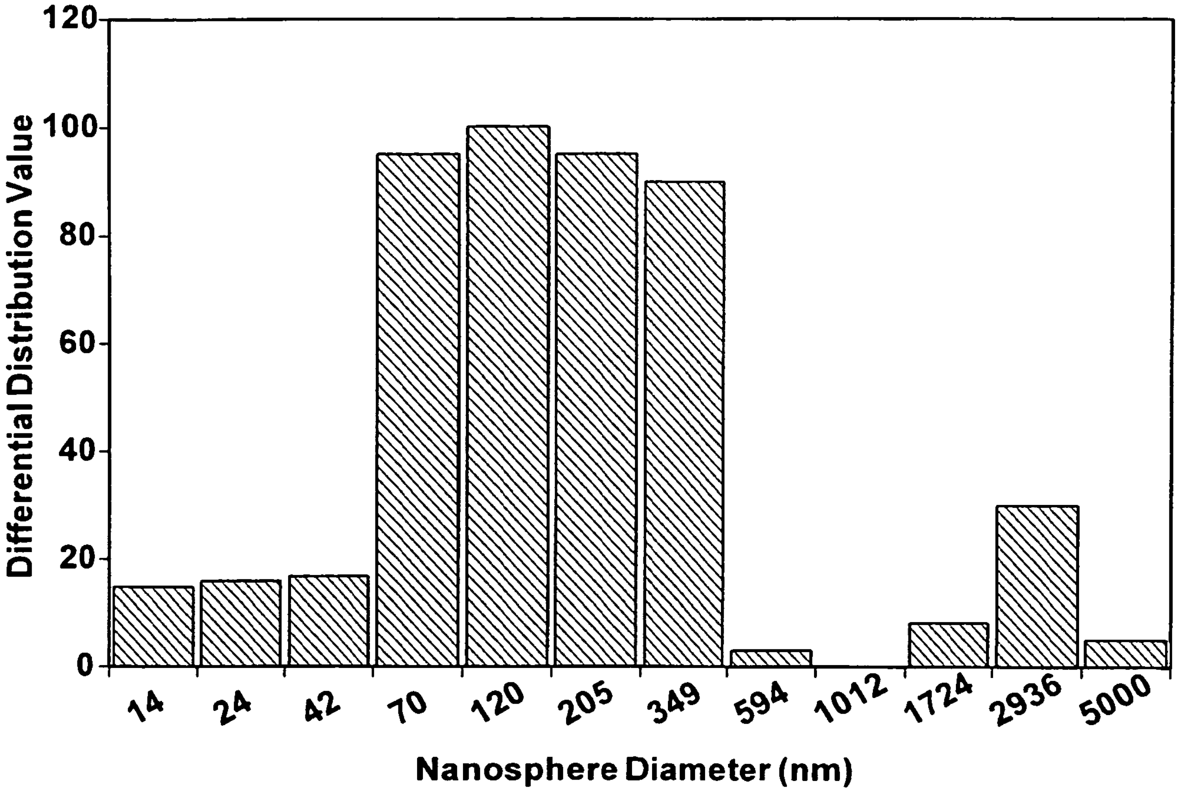


*Representative size distribution of 10% complexed pDNA nanospheres.*
*Determined using Regularized Non-negatively Constrained Least Squares (Contin).*

# FIG. 15

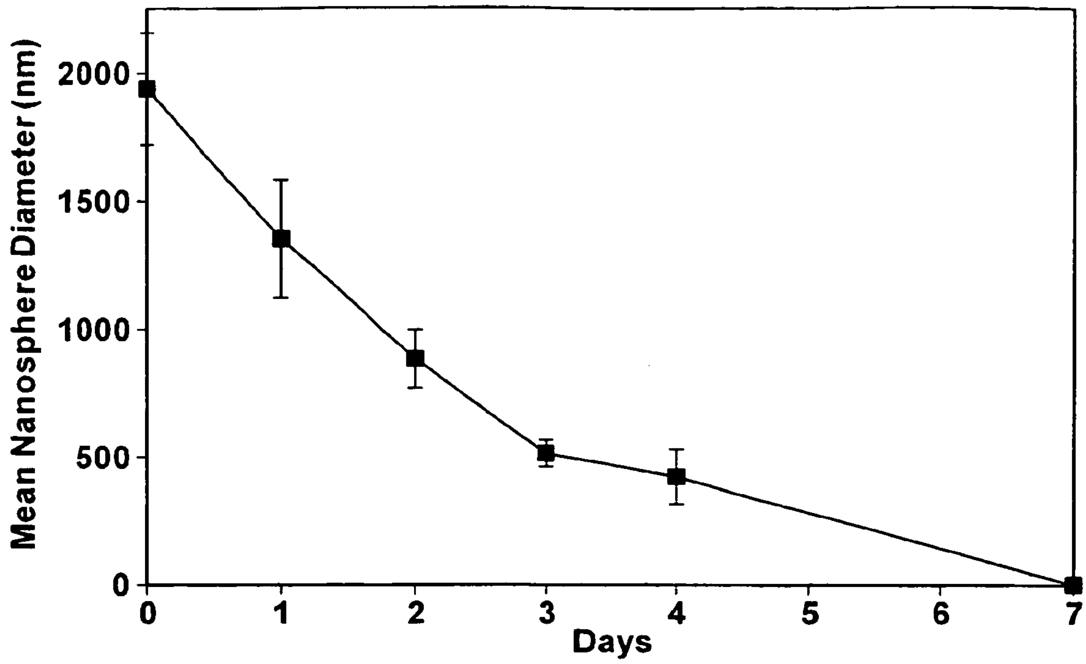


*Degradation of blank nanospheres mean diameter. Determined using Regularized Non-negatively Constrained Least Squares (Contin).*

**FIG. 16**

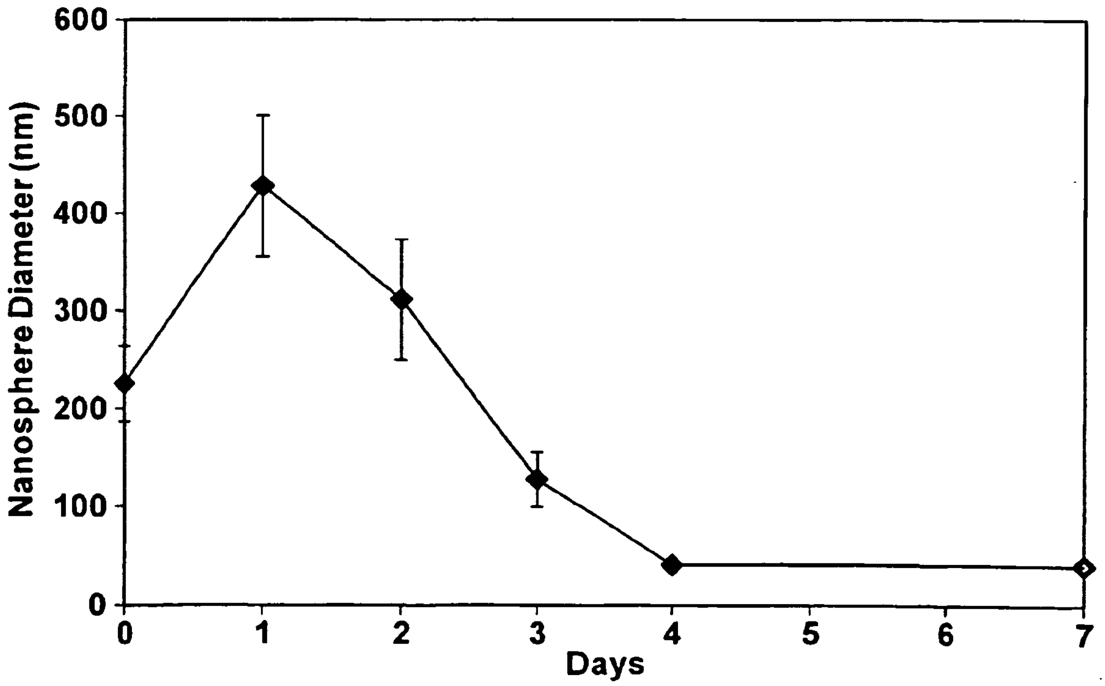


*Degradation of 1% complexed pDNA nanospheres mean diameter. Determined using Regularized Non-negatively Constrained Least Squares (Contin).*

**FIG. 17**

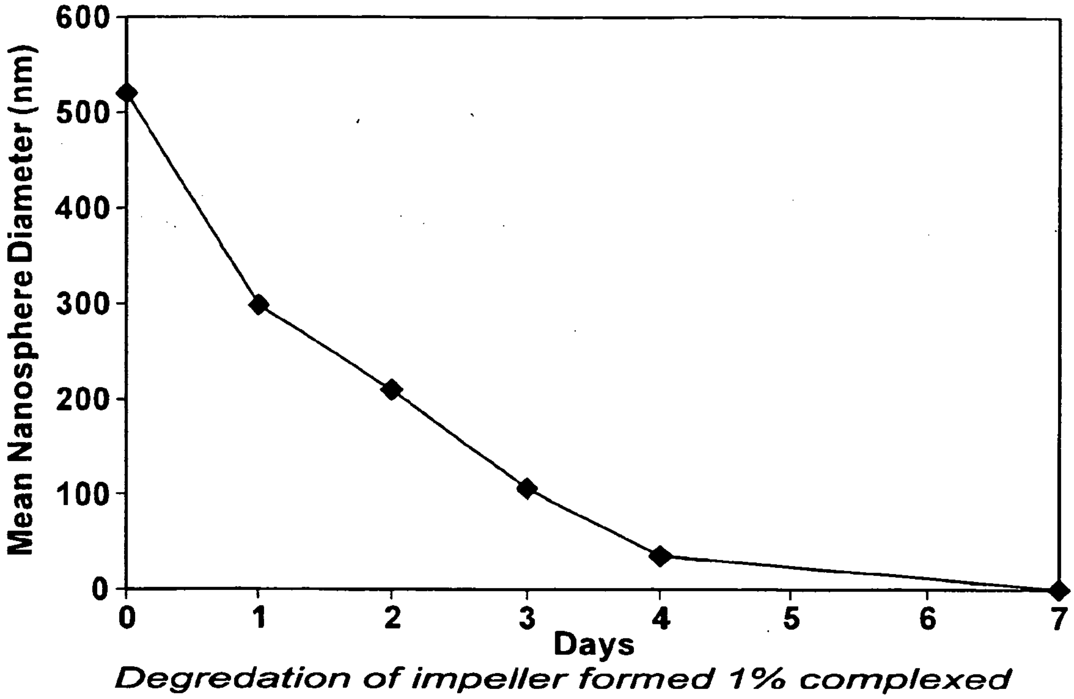


*Degredation of impeller formed 1% complexed pDNA nanosphere mean diameter. Determined using Regularized Non-negatively Constrained Least Squares (Contin).*

**FIG. 18**

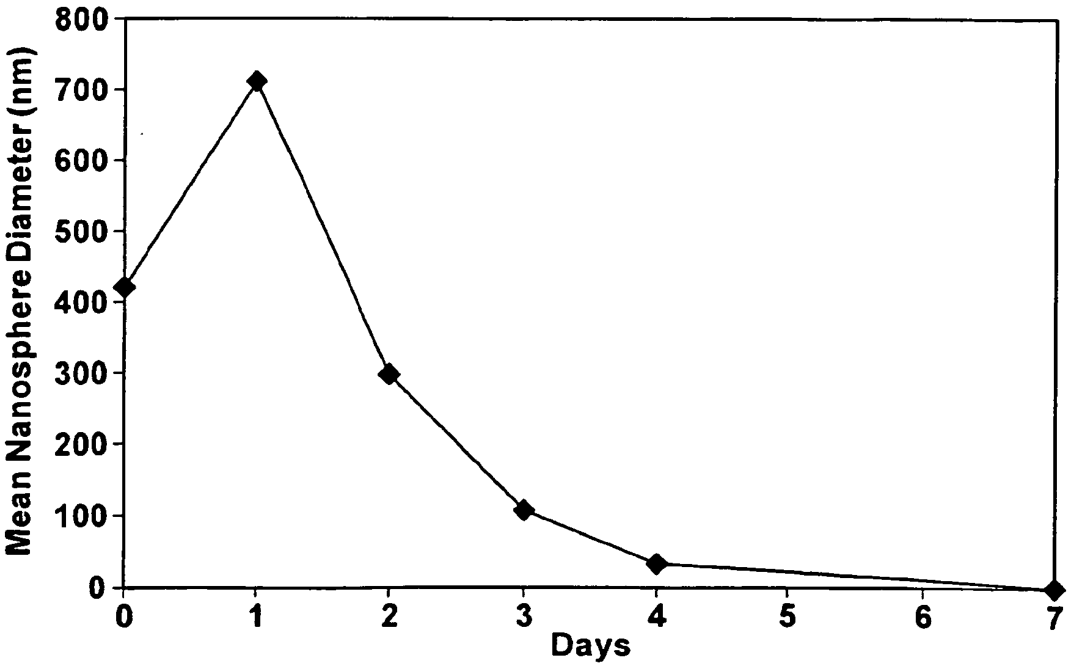


*Degredation of 10% complexed pDNA nanospheres mean diameter. Determined using Regularized Non-negatively Constrained Least Squares (Contin).*

**FIG. 19**

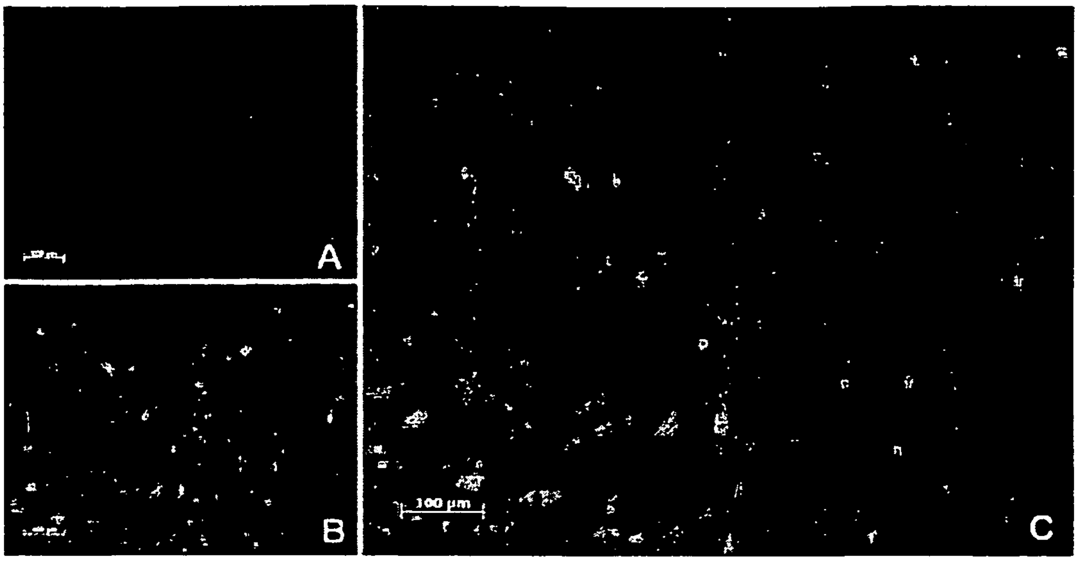


Figure 4.5.1: *LIVE/DEAD® Cell Assay of human dermal fibroblasts exposed to 200 µl of buffers. A) Dead cells (green), B) Live and metabolically active cells (red), C) Combined fluorescence channels.*

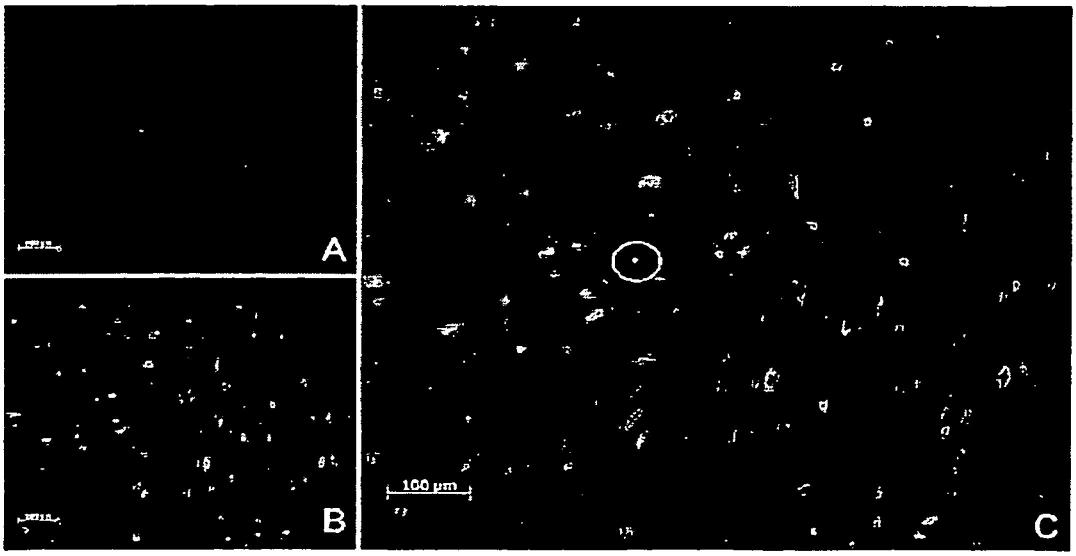


Figure 4.5.2: *LIVE/DEAD® Cell Assay of human dermal fibroblasts exposed to 4 µg of pDNA. A) Dead cells (green), B) Live and metabolically active cells (red), C) Combined fluorescence channels with dead cell circled in yellow.*

# FIGS. 20 and 21

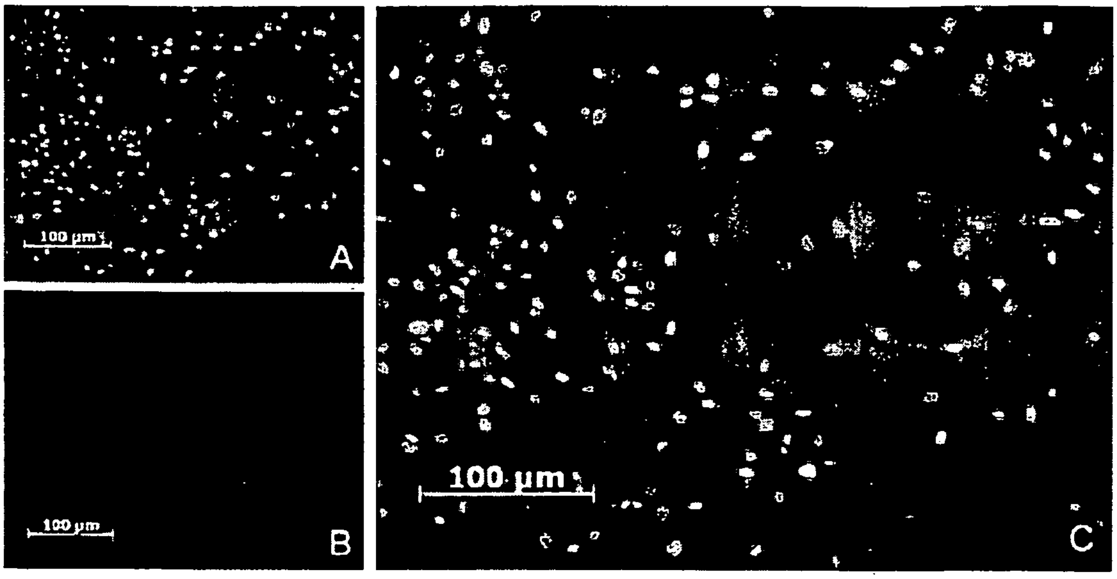


Figure 4.5.3: *LIVE/DEAD® Cell Assay of human dermal fibroblasts exposed to 2mM $H_2O_2$. A) Dead cells (green), B) Live and metabolically active cells (red), C) Combined fluorescence channels.*

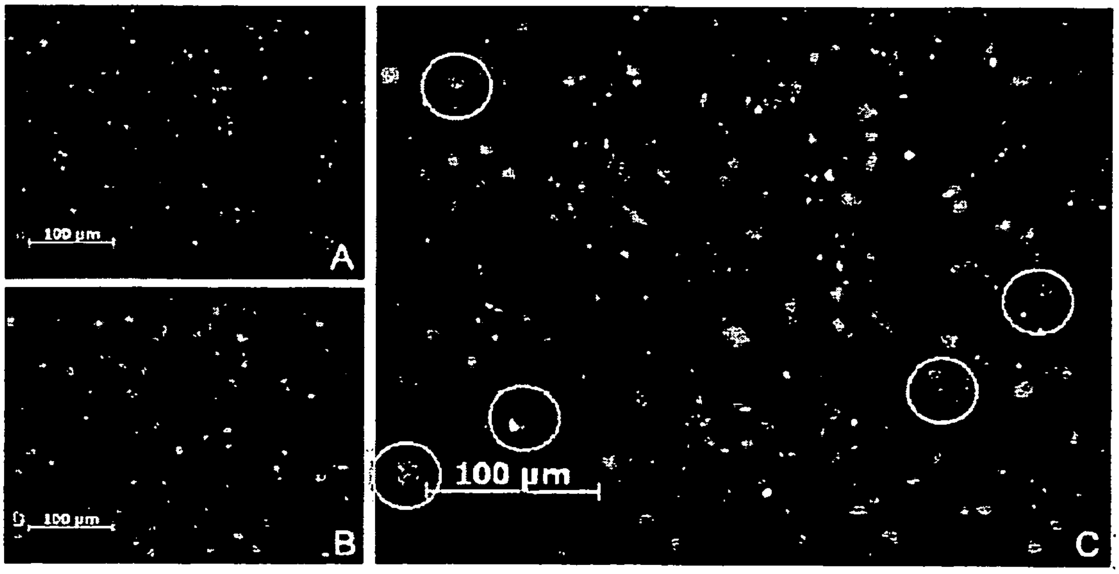


Figure 4.5.4: *LIVE/DEAD® Cell Assay of human dermal fibroblasts exposed to 4 µg of pDNA with12 µl of FuGENE 6. A) Dead cells (green), B) Live and metabolically active cells (red), C) Combined fluorescence channels with dead cell circled in yellow.*

## FIGS. 22 and 23

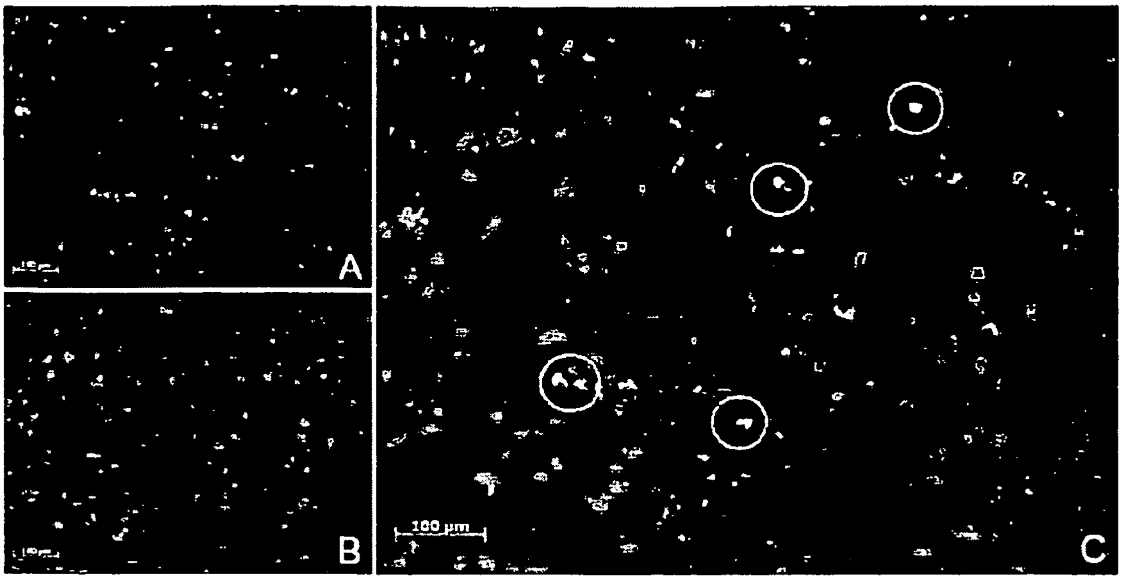


Figure 4.5.5: *LIVE/DEAD® Cell Assay of human dermal fibroblasts exposed to 4 µg of pDNA complexed with 4µg of LPEI. A) Dead cells (green), B) Live and metabolically active cells (red), C) Combined fluorescence channels with dead cell circled in yellow.*

FIG. 24

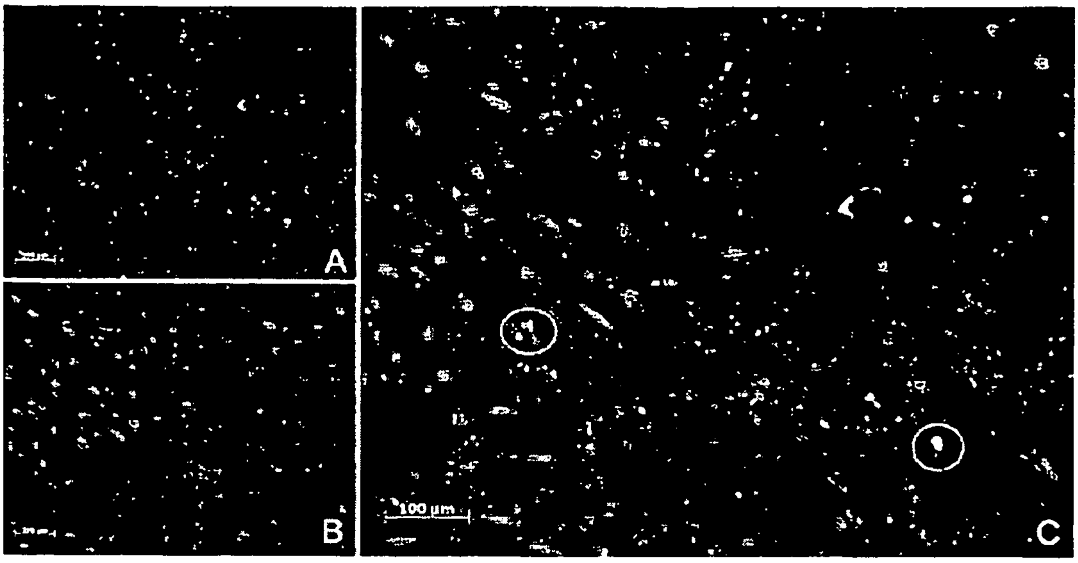


Figure 4.5.6: *LIVE/DEAD® Cell Assay of human dermal fibroblasts exposed to 400 µg of 1% complexed pDNA nanospheres. A) Dead cells (green), B) Live and metabolically active cells (red), C) Combined fluorescence channels with dead cell circled in yellow.*

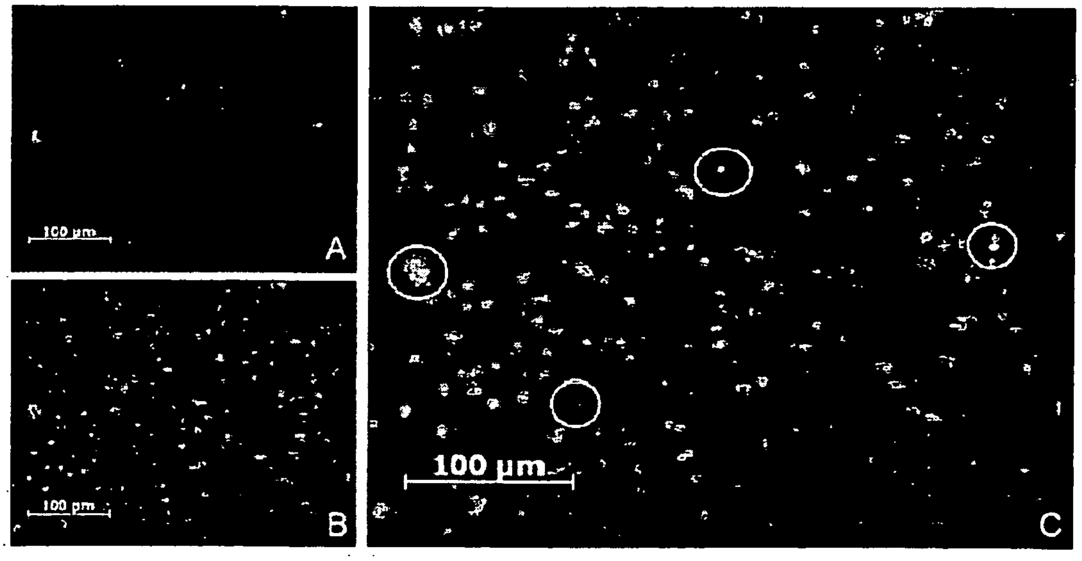


Figure 4.5.7: *LIVE/DEAD® Cell Assay of human dermal fibroblasts exposed to 400 µg of PLGA nanospheres. A) Dead cells (green), B) Live and metabolically active cells (red), C) Combined fluorescence channels with dead cell circled in yellow.*

## FIGS. 25 and 26

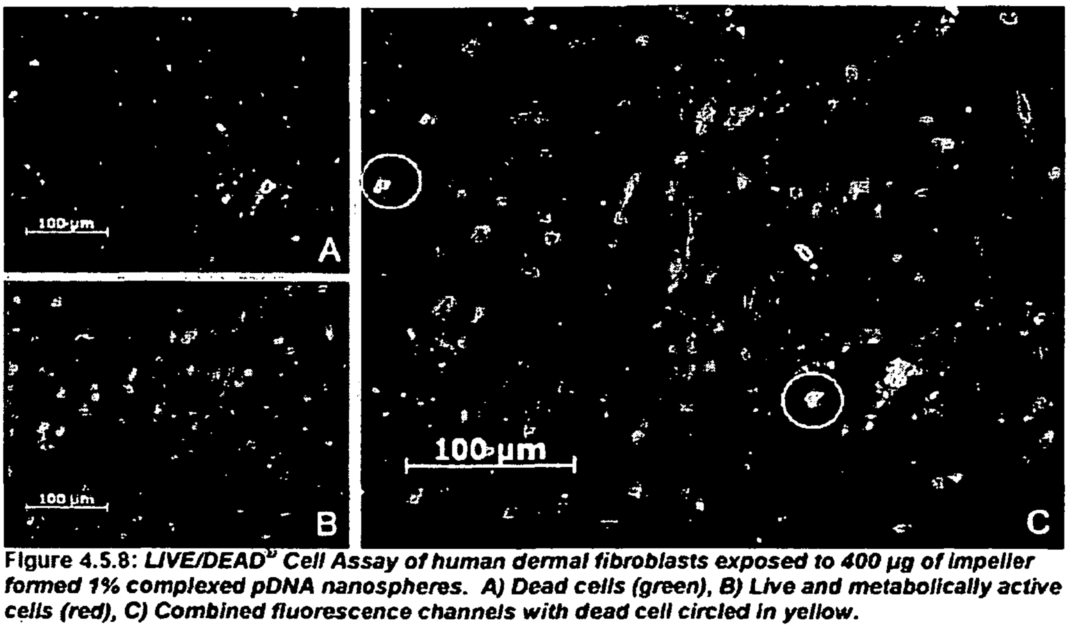


Figure 4.5.8: *LIVE/DEAD® Cell Assay of human dermal fibroblasts exposed to 400 µg of impeller formed 1% complexed pDNA nanospheres. A) Dead cells (green), B) Live and metabolically active cells (red), C) Combined fluorescence channels with dead cell circled in yellow.*

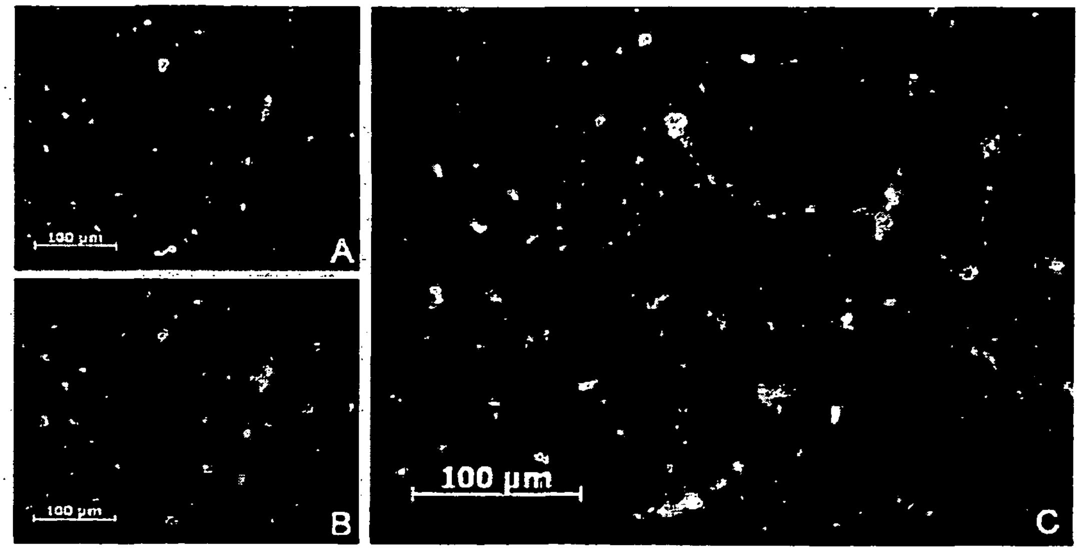


Figure 4.5.9: *LIVE/DEAD® Cell Assay of human dermal fibroblasts exposed to 100 µg of 10% complexed pDNA nanospheres. A) Dead cells (green), B) Live and metabolically active cells (red), C) Combined fluorescence channels with dead cell circled in yellow.*

FIGS. 27 and 28

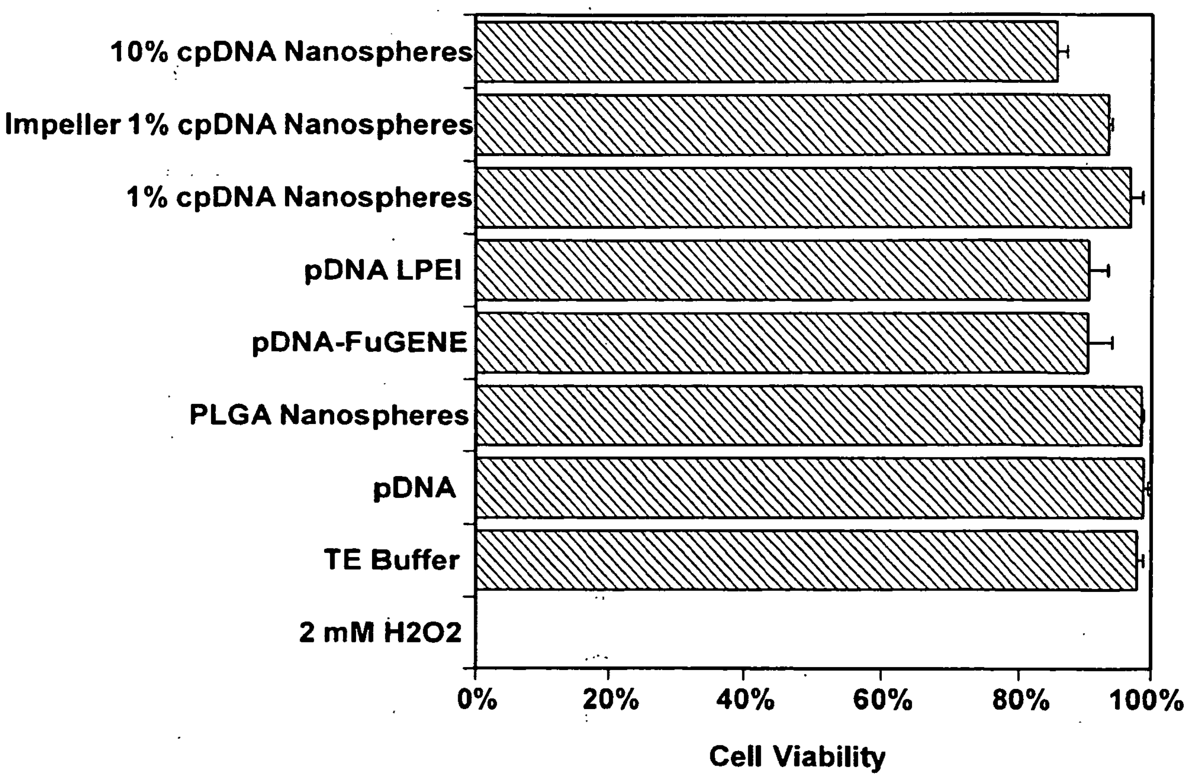


*Cell Viability of various gene vectors determined by LIVE/DEAD® Cell Assay.*

**FIG. 29**

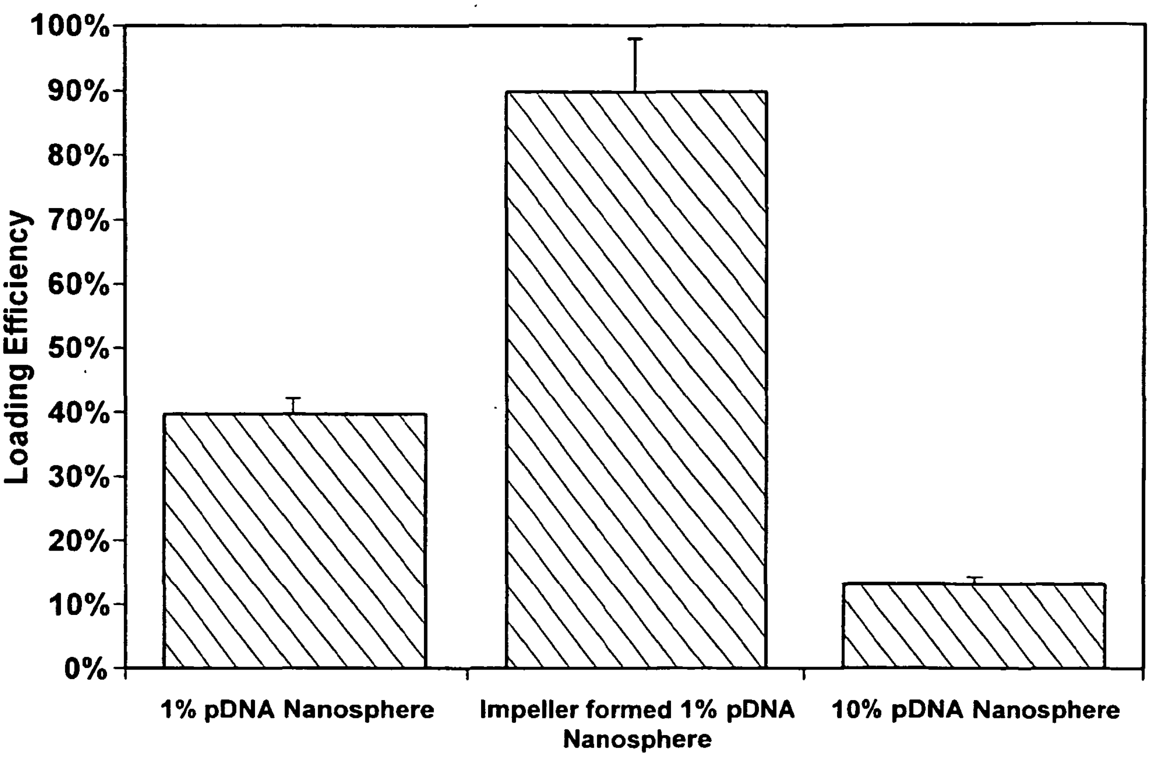


*Loading efficiency of various nanosphere formulations.*

**FIG. 30**

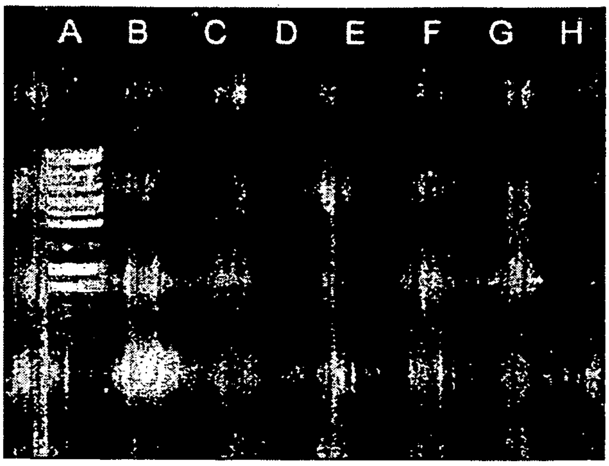


Figure 4.7.1: *Gel Electrophoresis of: A) 1 kb DNA ladder, blank nanosphere release after B) 1 day, C) 2 days, D) 3 days, E) 4 days, F) 5 days, G) 6 days, H) 7 days*

**FIG. 31**

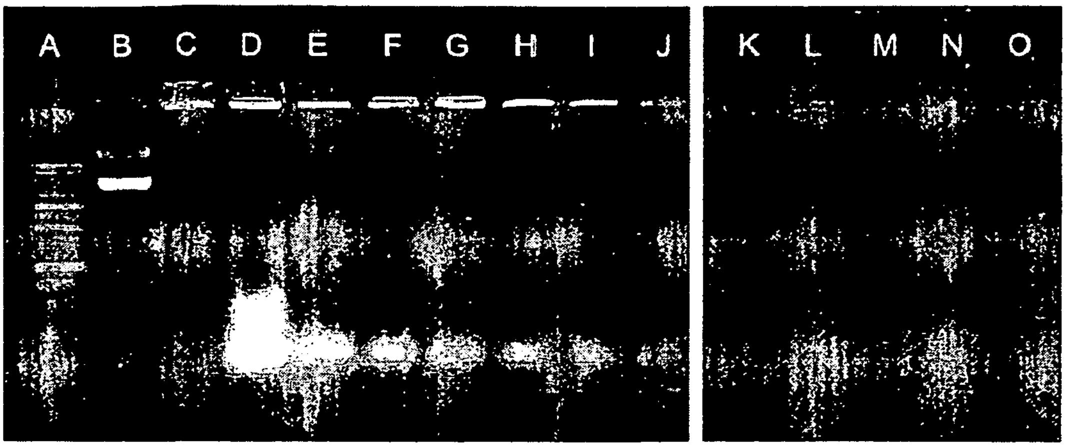


Figure 4.7.2: *Gel Electrophoresis of: A) 1 kb DNA ladder B) stock pDNA, C) stock pDNA-LPEI, 1% complexed pDNA nanosphere release after: D) 0.5 hrs, E) 1.5 hrs, F) 3.0 hrs, G) 6.0 hrs, H) 12.0 hrs, I) 24 hrs, J) 2 days, K) 3 days, L) 4 days, M) 5 days, N) 6 days, O) 7 days.*

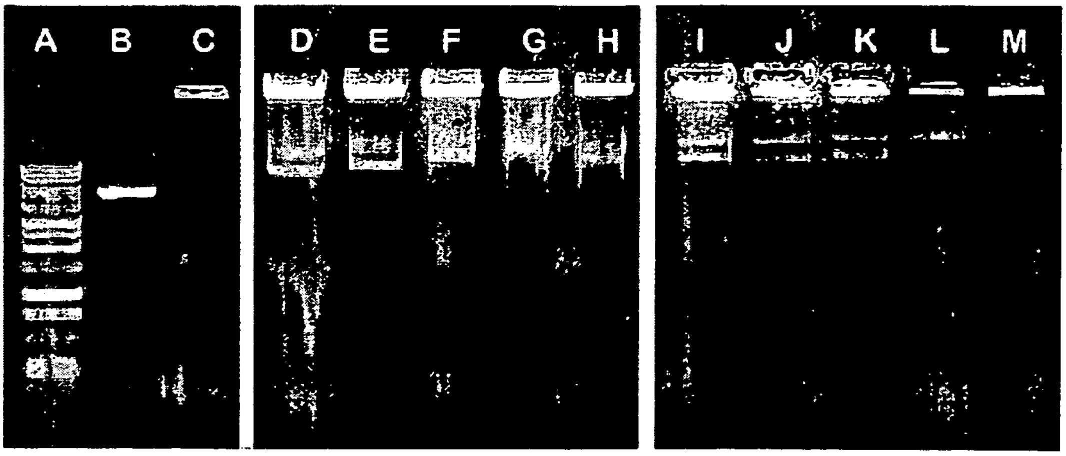


Figure 4.7.3: *Gel Electrophoresis of: A) 1 kb DNA ladder B) stock pDNA, C) stock pDNA-LPEI, 10% complexed pDNA nanosphere release after: D) 0.5 hrs, E) 1.5 hrs, F) 6.0 hrs, G) 12.0 hrs, H) 24.0 hrs, I) 2 days, J) 3 days, K) 4 days, L) 6 days, M) 7 days.*

**FIGS. 32 and 33**

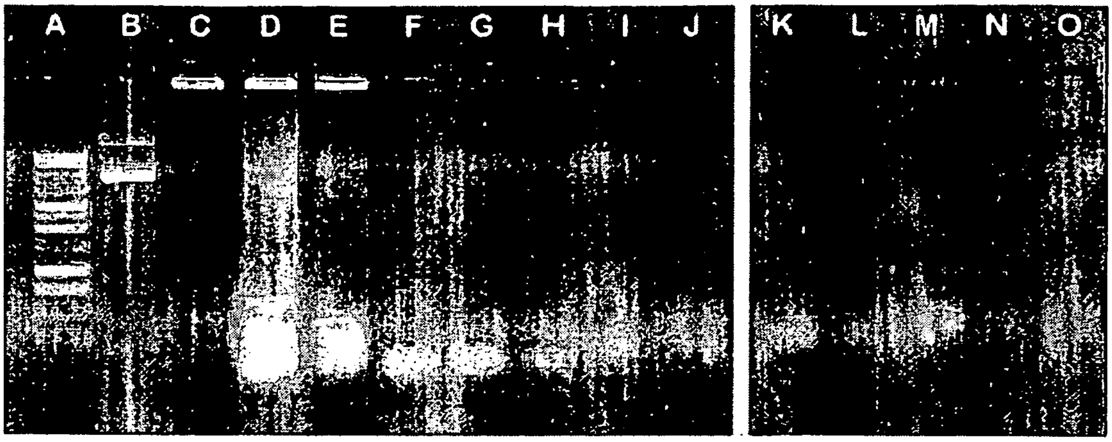


Figure 4.7.4: *Gel Electrophoresis of: A) 1 kb DNA ladder B) stock pDNA, C) stock pDNA-LPEI, impeller formed 1% complexed pDNA nanosphere release after: D) 0.5 hrs, E) 1.5 hrs, F) 3.0 hrs, G) 6.0 hrs, H) 12.0 hrs, I) 24 hrs, J) 2 days, K) 3 days, L) 4 days, M) 5 days, N) 6 days, O) 7 days.*

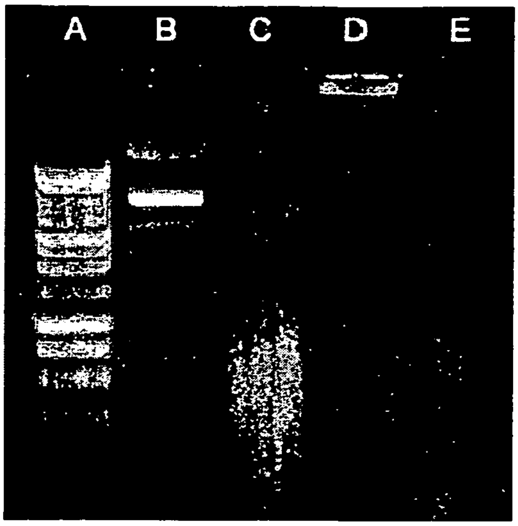


Figure 4.7.5: *Gel electrophoresis of A) ) 1 kb DNA ladder, B) stock pDNA, noncomplexed pDNA nanosphere release after: C) day 1, D) day 2, E) day 3*

# FIGS. 34 and 35

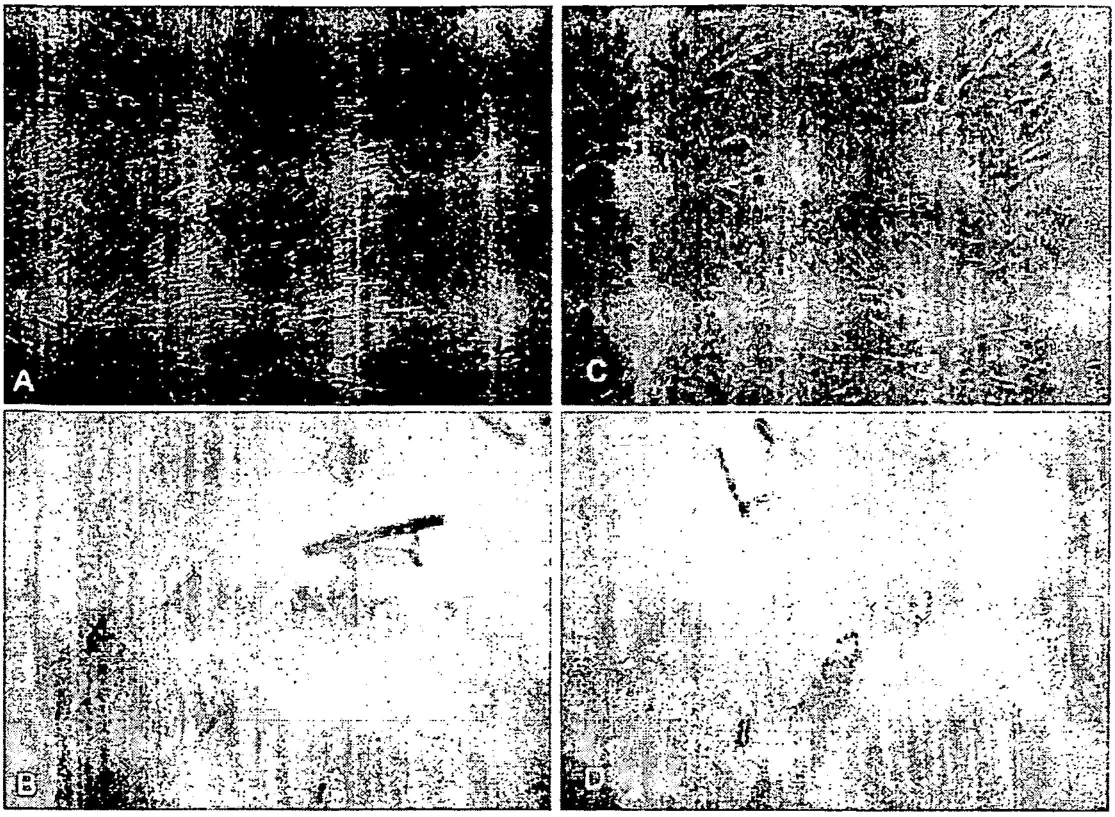


Figure 4.8.1: Representative transfection of primary human dermal fibroblasts obtained by day 2 release from A) blank nanospheres (100x phase contrast image), B) 1% pDNA nanospheres (100x phase contrast image), C) 10% pDNA nanospheres (200x bright field image), D) Impeller formed 1% pDNA nanospheres (200x bright field image).

FIG. 36

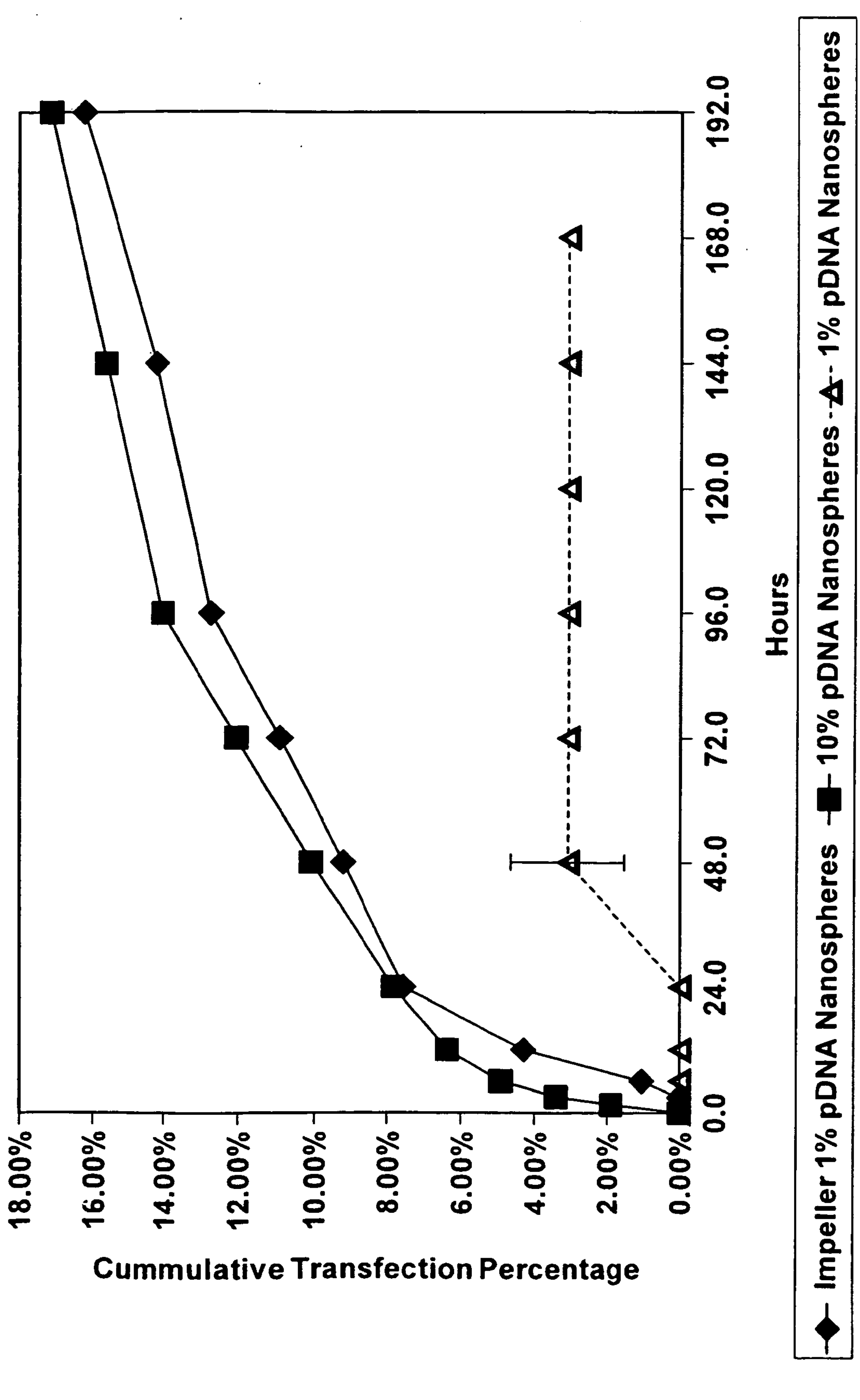


**FIG. 37**

*Cumulative release of pDNA-LPEI based on transfection percentage of primary human dermal fibroblasts.*

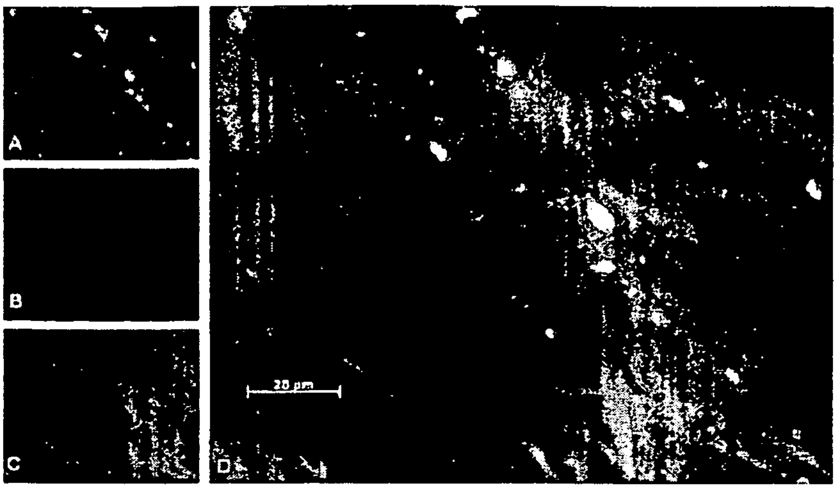


Figure 4.10.1: *A) FITC labeled nanospheres, B) DAPI stained nuclei, C) rhodamine phalloidin stained cytoskeleton, D) suggested human fibroblast cellular uptake of FITC labeled nanospheres*

# FIG. 38

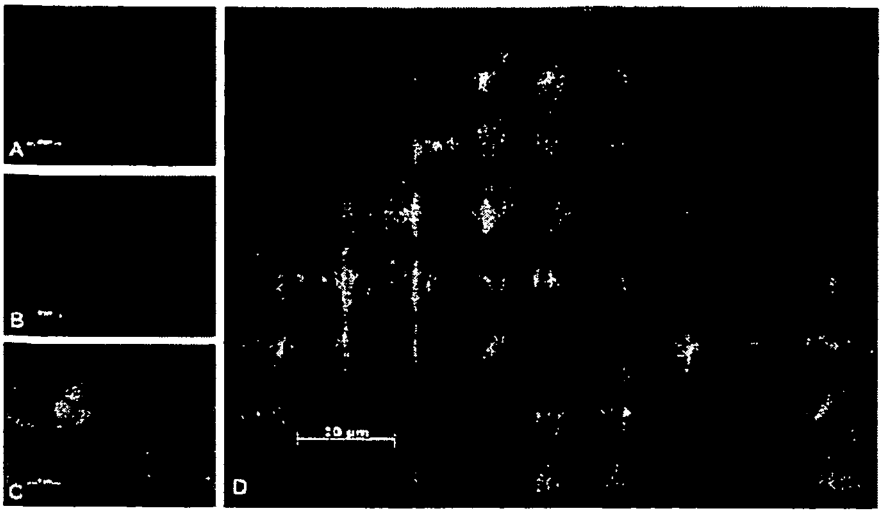


Figure 4.10.2: *A) FITC fluorescence filter on human fibroblasts, B) DAPI stained nuclei, C) rhodamine phalloidin stained cytoskeleton, D) human fibroblasts without addition of FITC labeled nanospheres*

FIG. 39

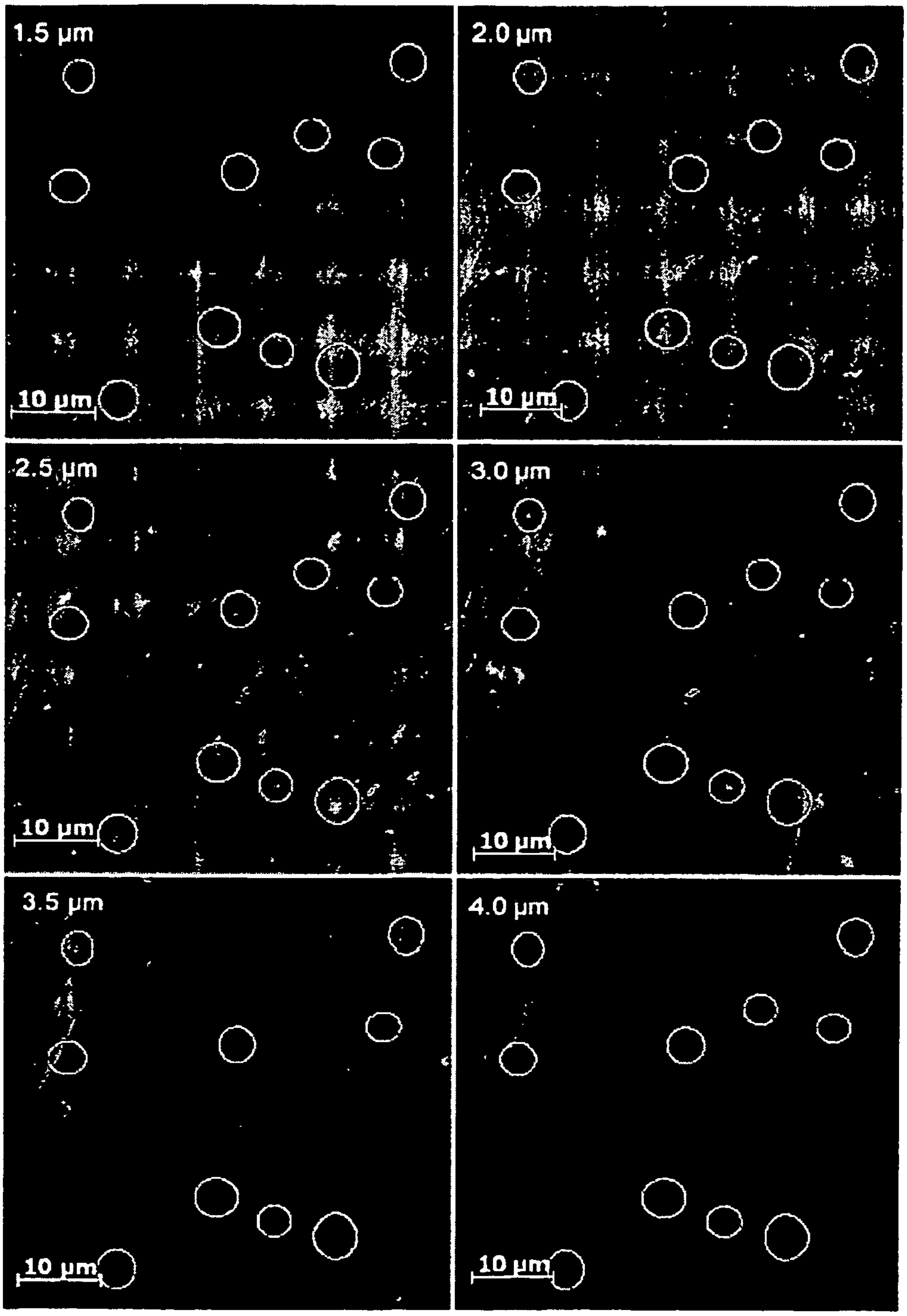


Figure 4.10.2: ***Confocal microscopy of fibroblast uptake of FITC labeled nanospheres.***

**FIG. 40**

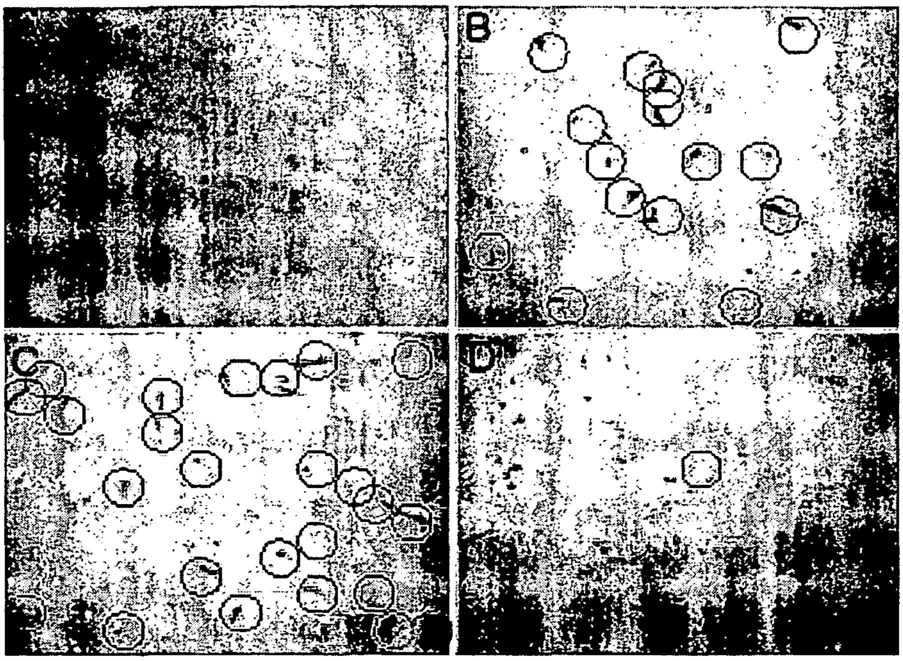


Figure 4.11.1: Day 3 of human dermal fibroblasts transfected with: A) 4 µg pDNA, B) 4 µg of pDNA with 12 µl of FuGENE 6 transfection reagent, C) 4 µg of pDNA complexed with 4 µg of LPEI, D) 400 µg of 1% pDNA nanospheres

FIG. 41

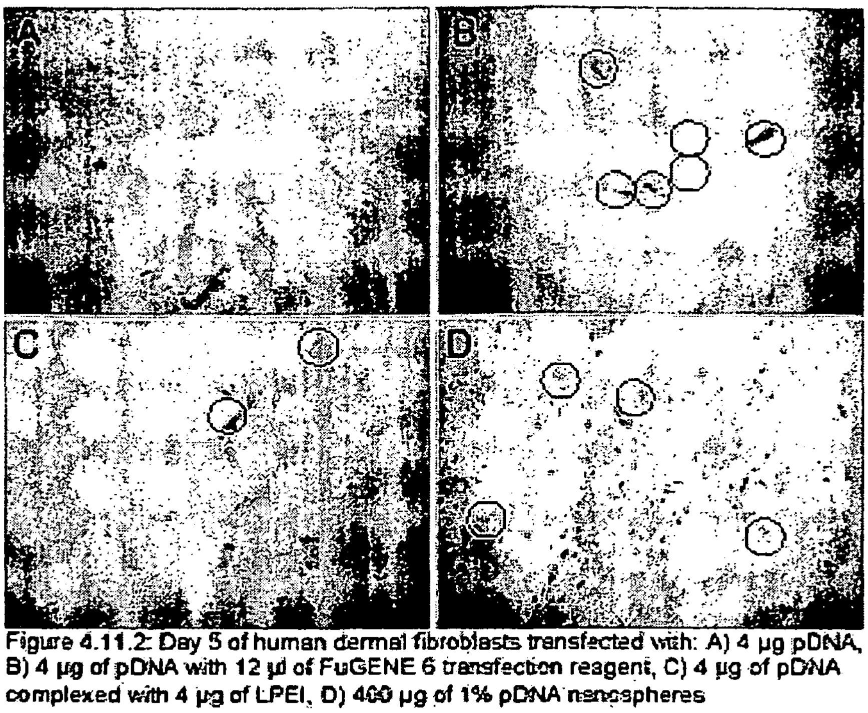


Figure 4.11.2: Day 5 of human dermal fibroblasts transfected with: A) 4 µg pDNA, B) 4 µg of pDNA with 12 µl of FuGENE 6 transfection reagent, C) 4 µg of pDNA complexed with 4 µg of LPEI, D) 400 µg of 1% pDNA nanospheres

FIG. 42

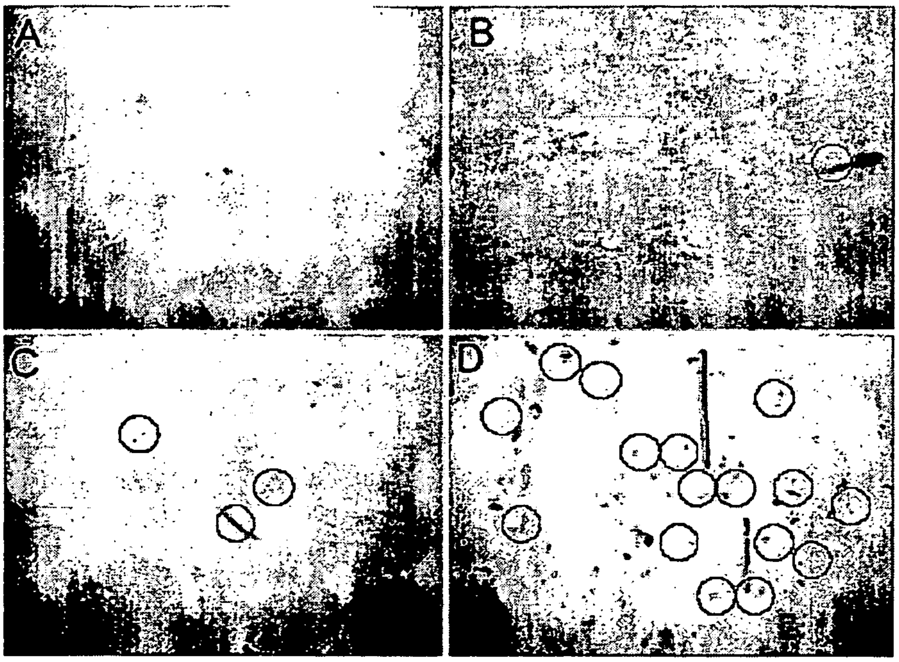


Figure 4.11.3: Day 7 of human dermal fibroblasts transfected with: A) 4 µg pDNA, B) 4 µg of pDNA with 12 µl of FuGENE 6 transfection reagent, C) 4 µg of pDNA complexed with 4 µg of LPEI, D) 400 µg of 1% pDNA nanospheres

FIG. 43

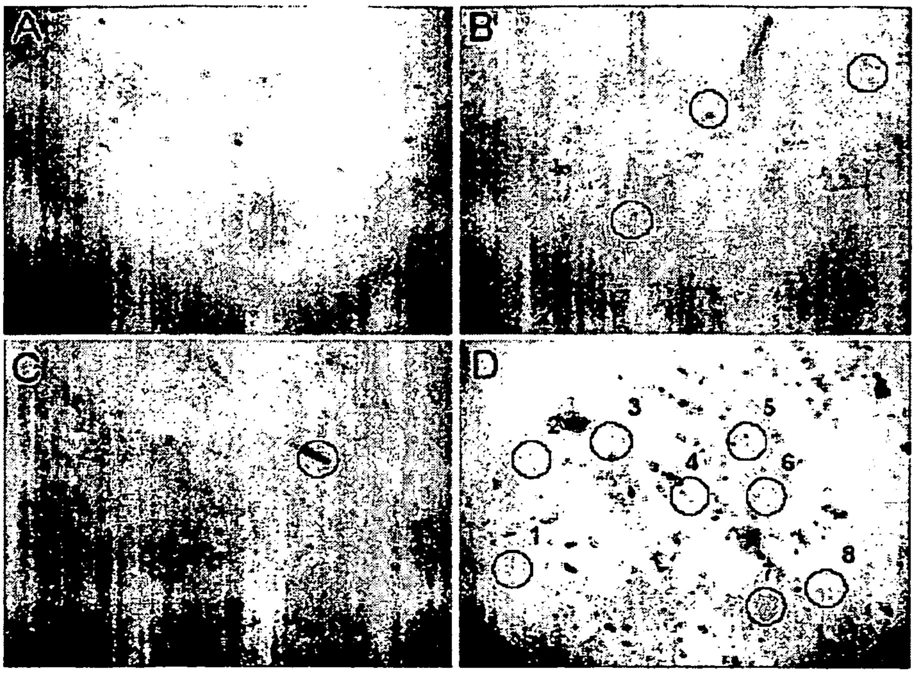


Figure 4.11.4: Day 9 of human dermal fibroblasts transfected with: A) 4 μg pDNA, B) 4 μg of pDNA with 12 μl of FuGENE 6 transfection reagent, C) 4 μg of pDNA complexed with 4 μg of LPEI, D) 400 μg of 1% pDNA nanospheres

FIG. 44

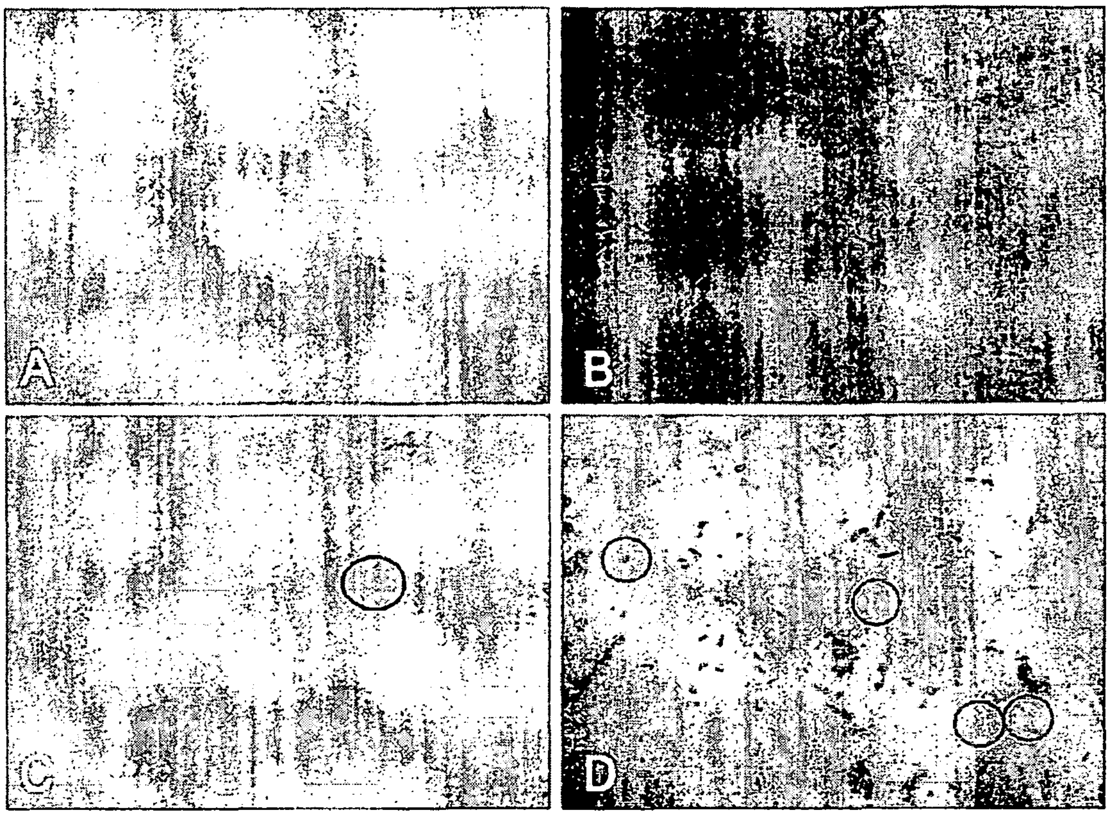


Figure 4.11.5: ***Day 11 of human dermal fibroblasts transfected with: A) 4 µg pDNA, B) 4 µg of pDNA with 12 µl of FuGENE 6 transfection reagent, C) 4 µg of pDNA complexed with 4 µg of LPEI, D) 400 µg of 1% complexed pDNA nanospheres.***

**FIG. 45**

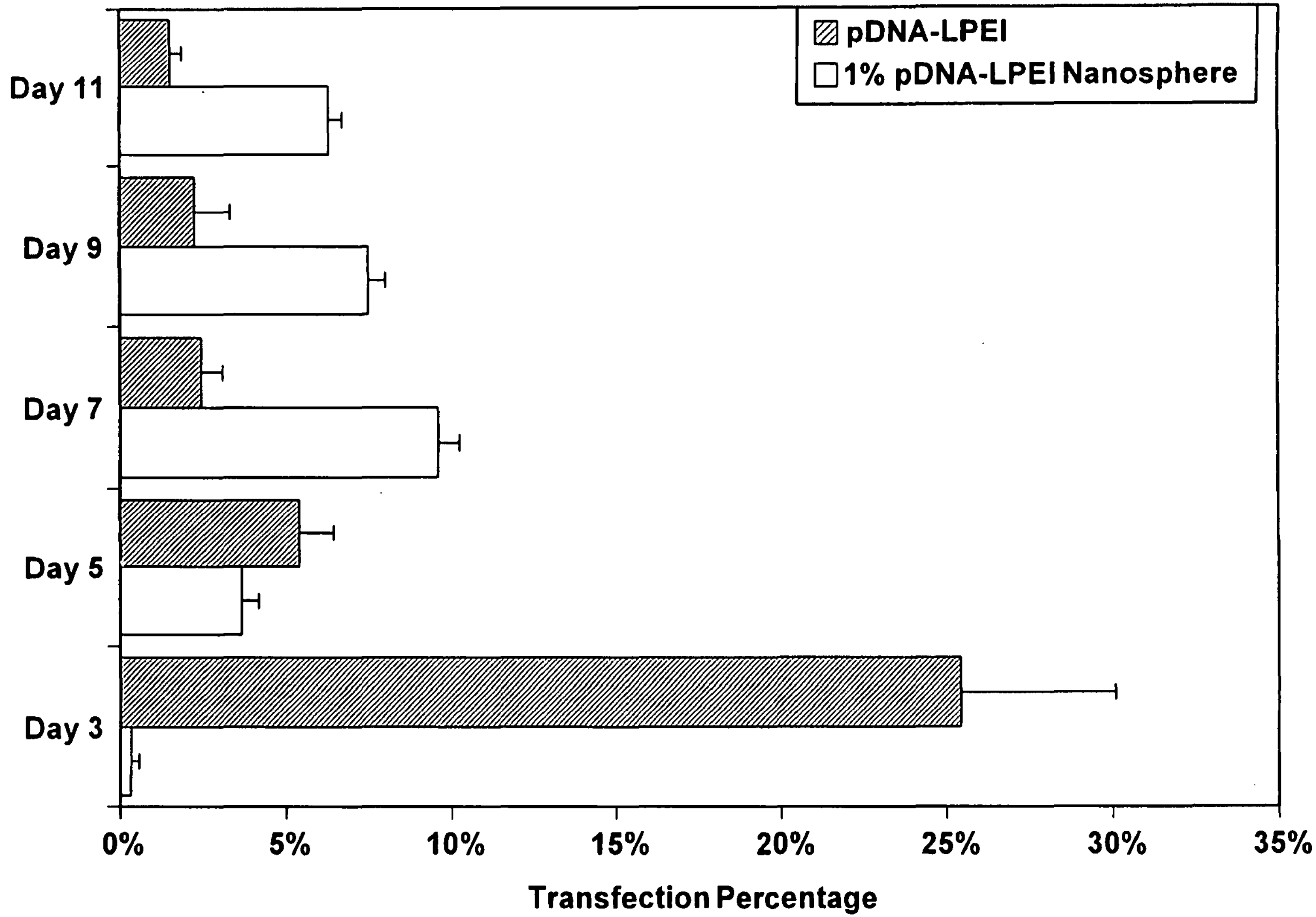


**FIG. 46** *Comparative transfection efficiency of pDNA-LPEI versus 1% pDNA-LPEI nanospheres over 11 days.*

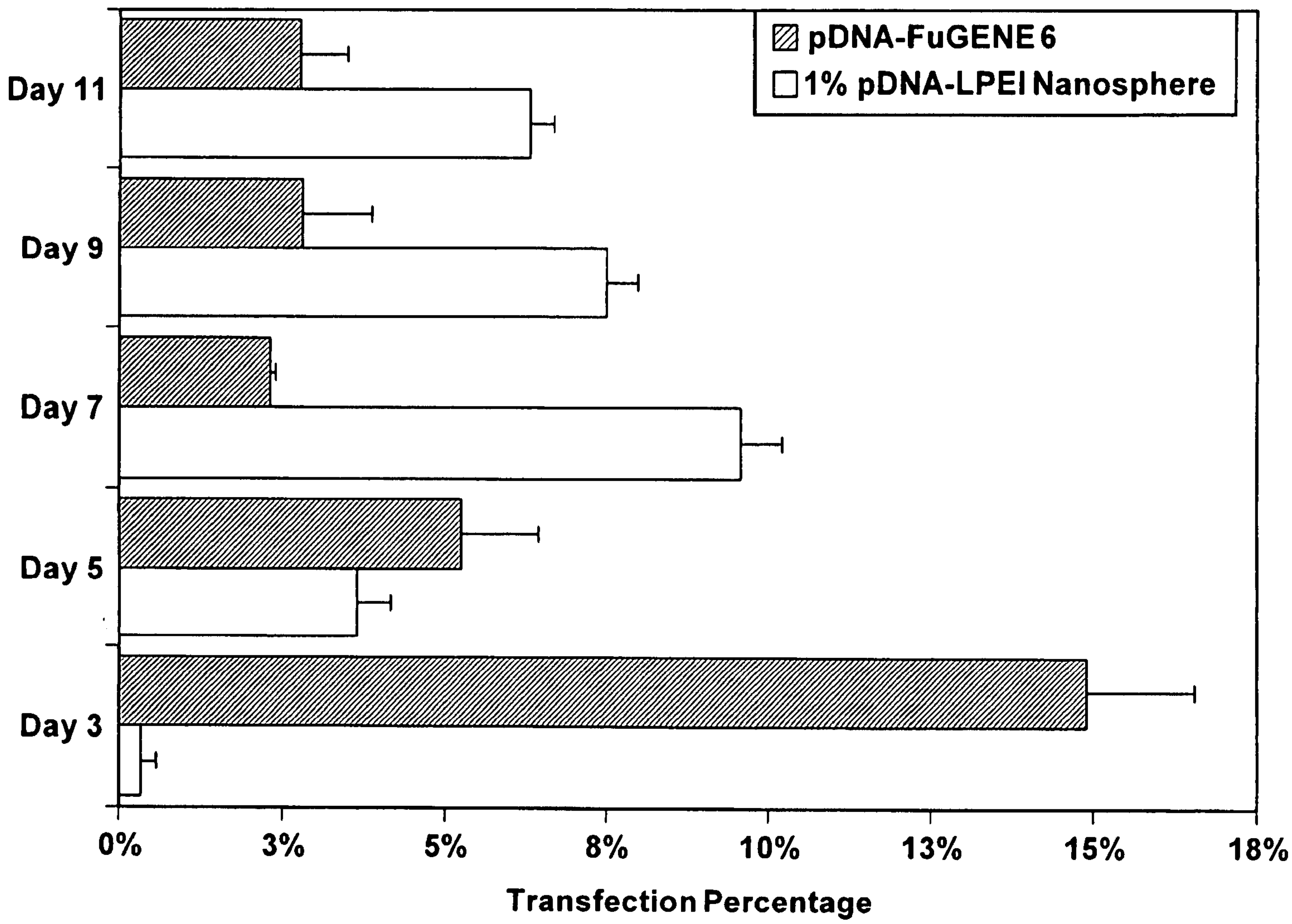


FIG. 47 *Comparative transfection efficiency of FuGENE 6 versus 1% pDNA-LPEI nanospheres over 11 days.*

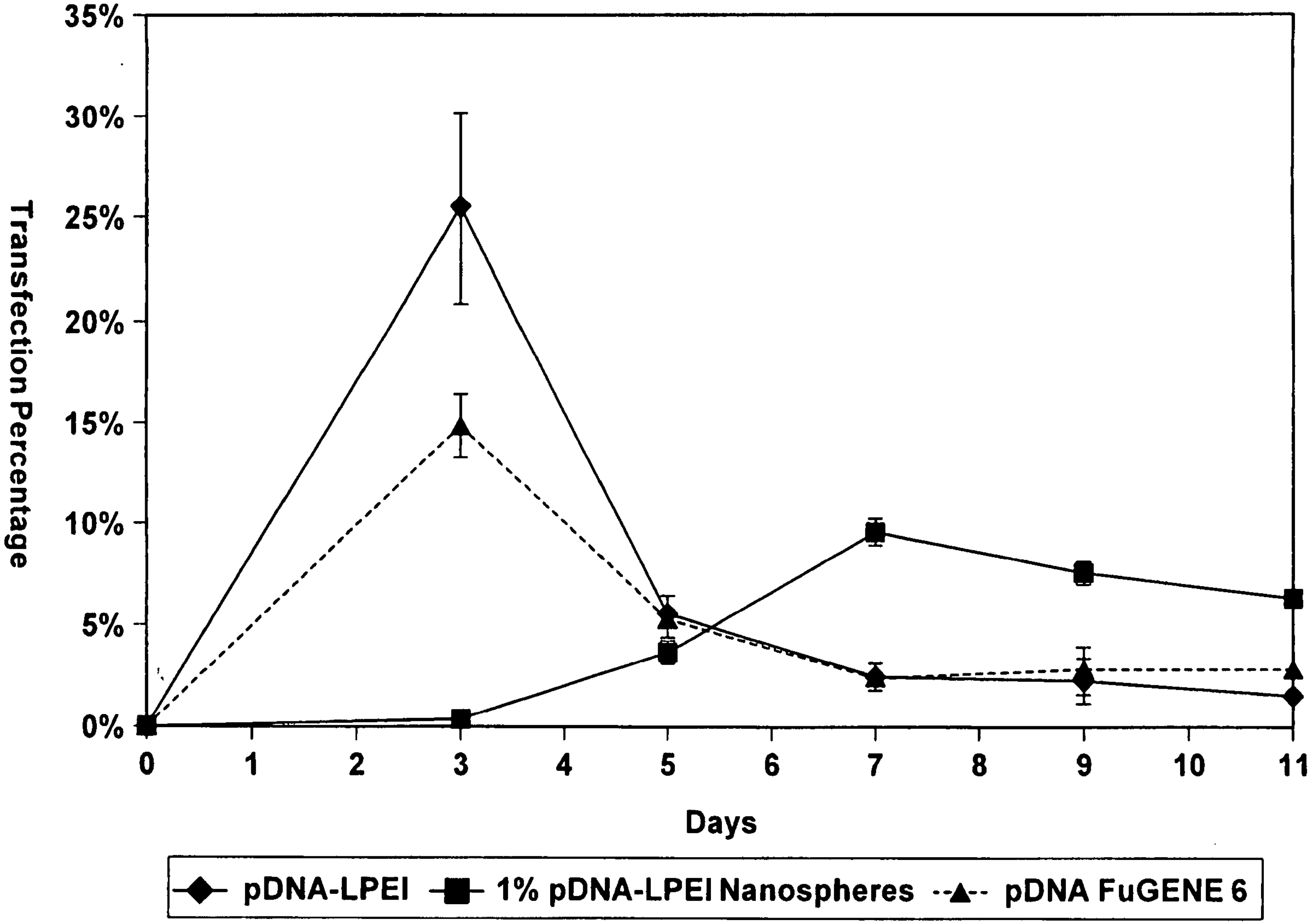


**FIG. 48** *Transfection efficiency profile of 1% pDNA-LPEI nanospheres versus pDNA-LPEI and pDNA-FuGENE 6 over 11 days.*

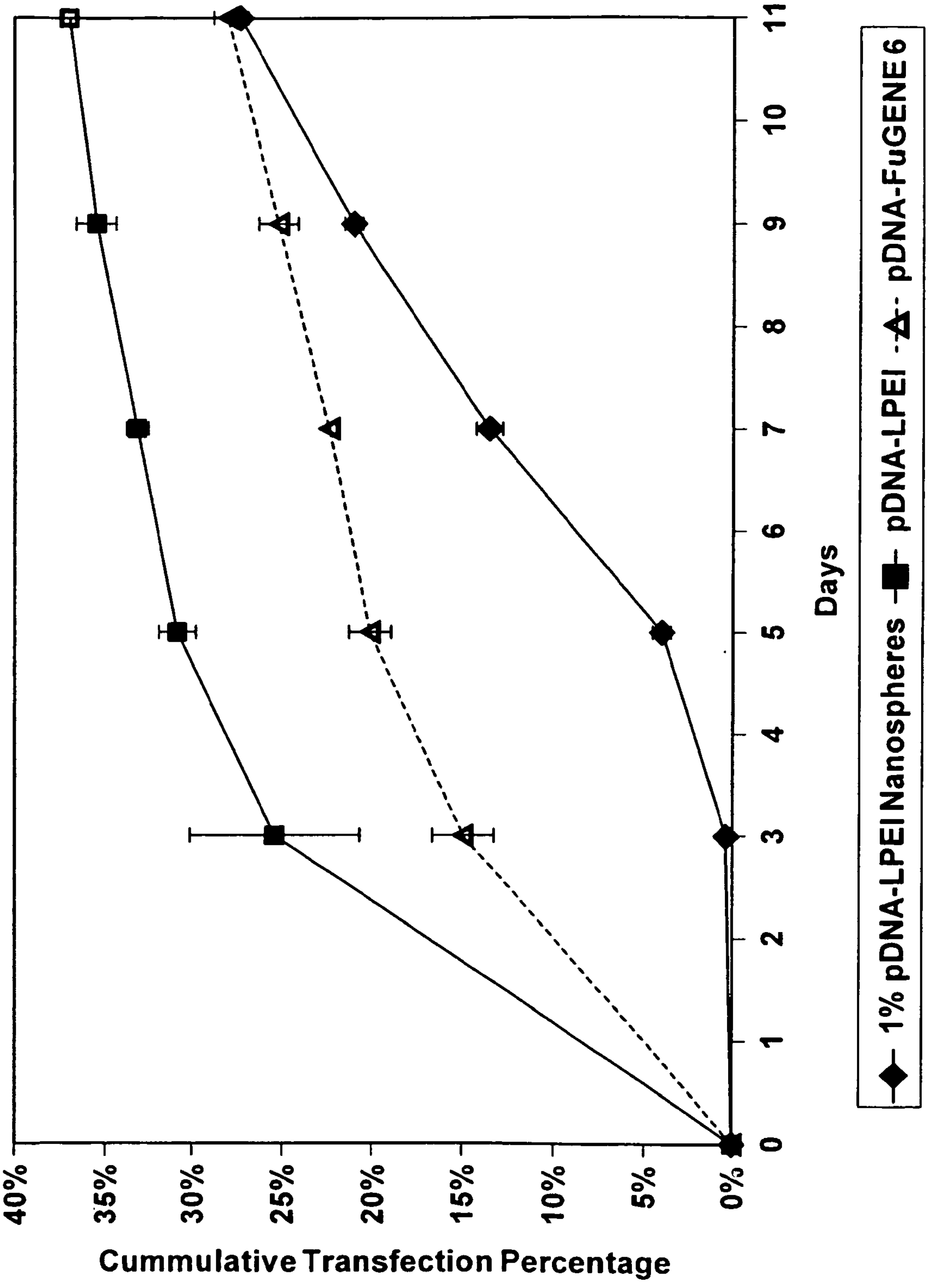


FIG. 49 *Cumulative transfection efficiency profile of 1% pDNA-LPEI nanospheres versus pDNA-LPEI and pDNA-FuGENE 6 over 11 days.*

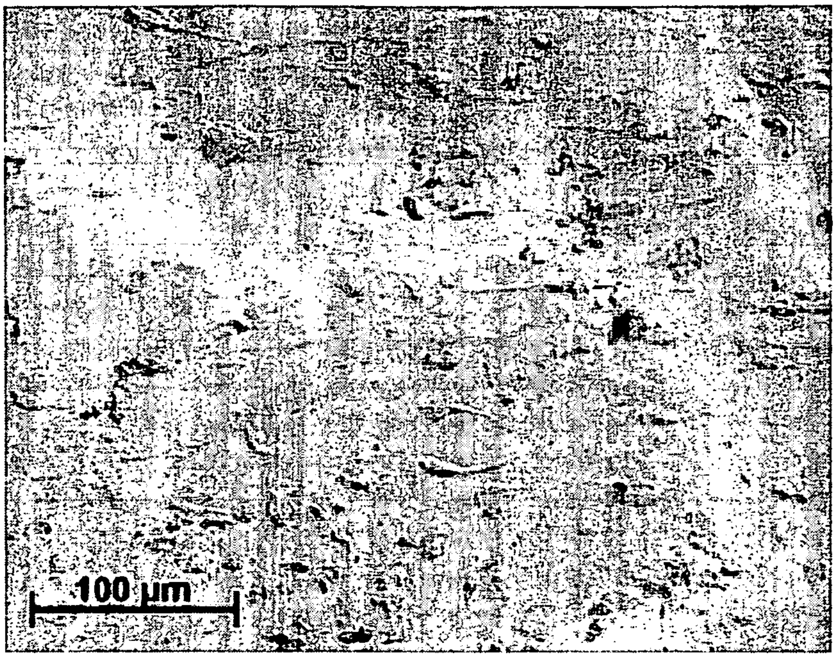


Figure 4.11.10: Brightfiled image of human dermal fibroblast expressing lacZ gene, which results in blue coloring from X-Gal™ staingings.

FIG. 50

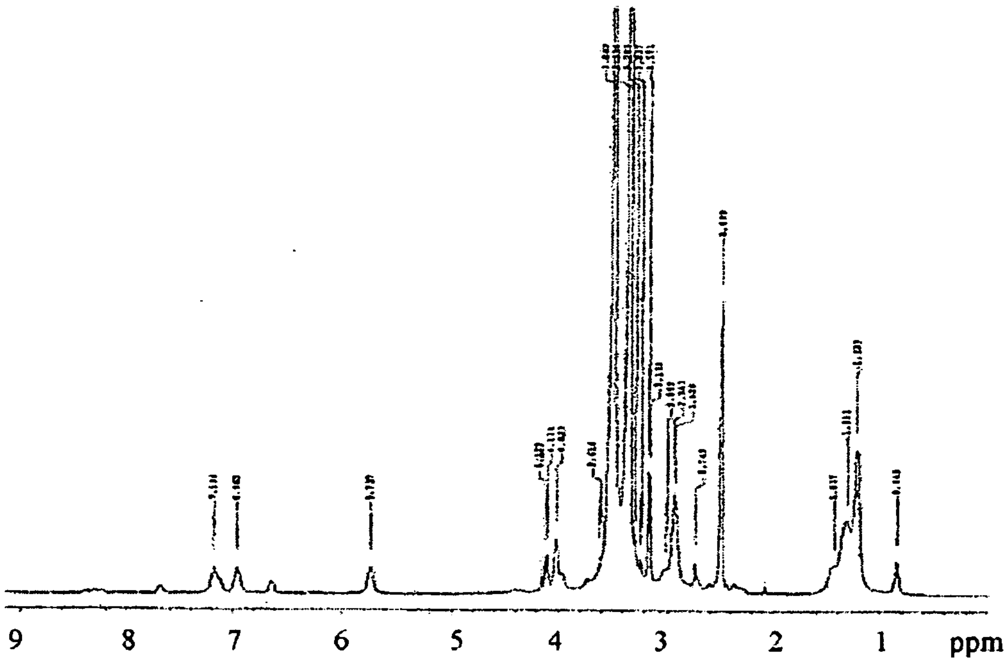


FIG. 51

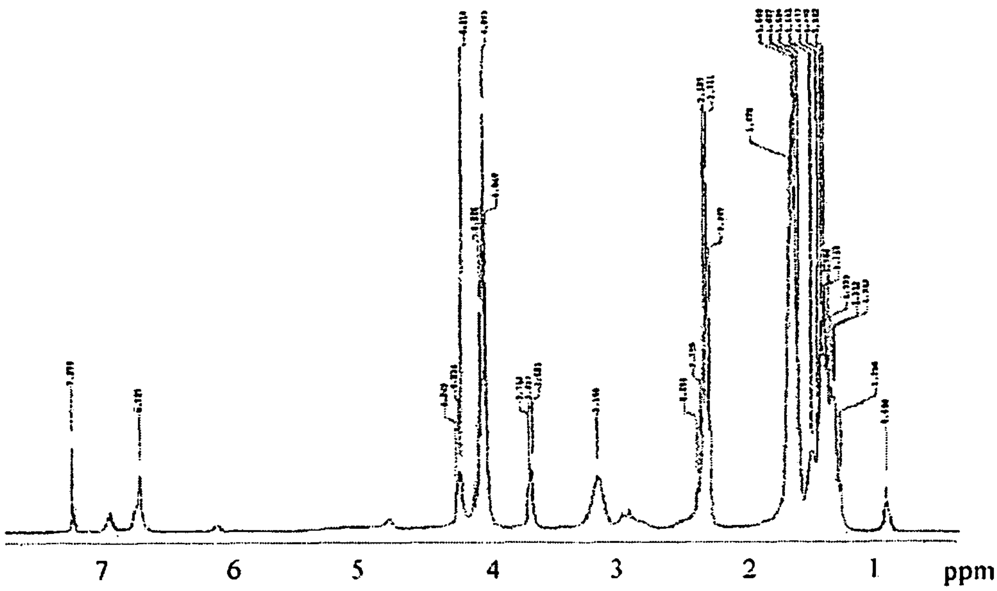


FIG. 52

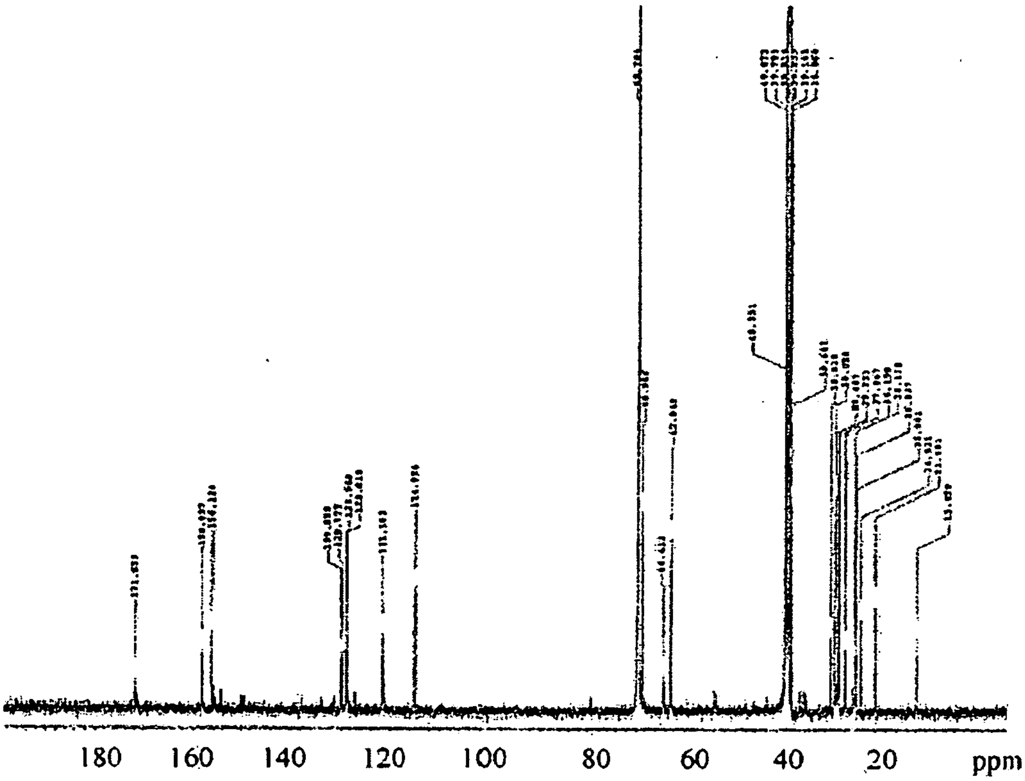


FIG. 53

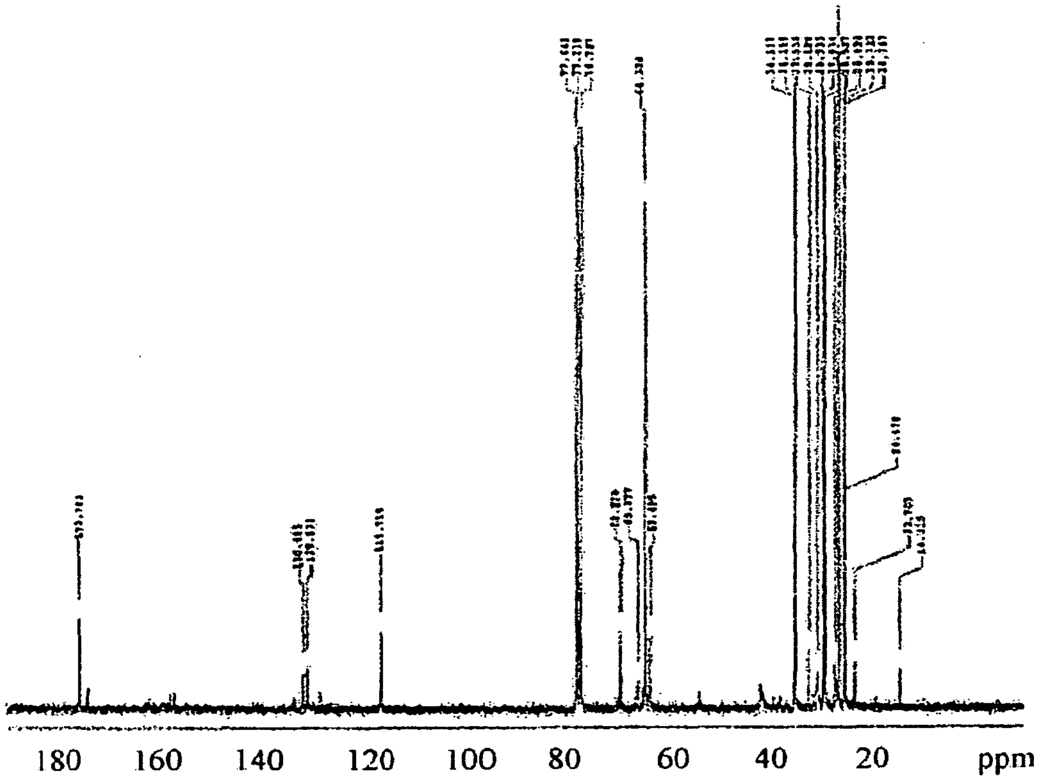


FIG. 54

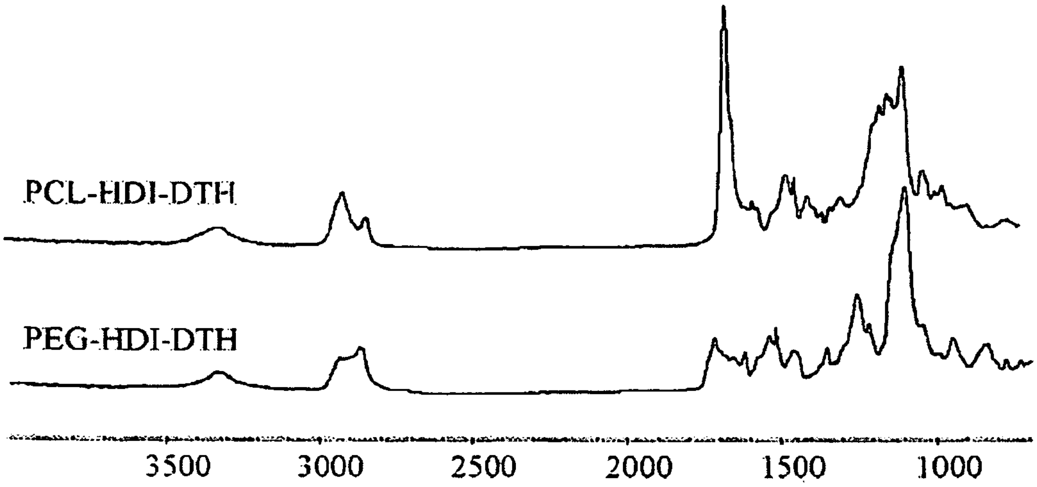


FIG. 55

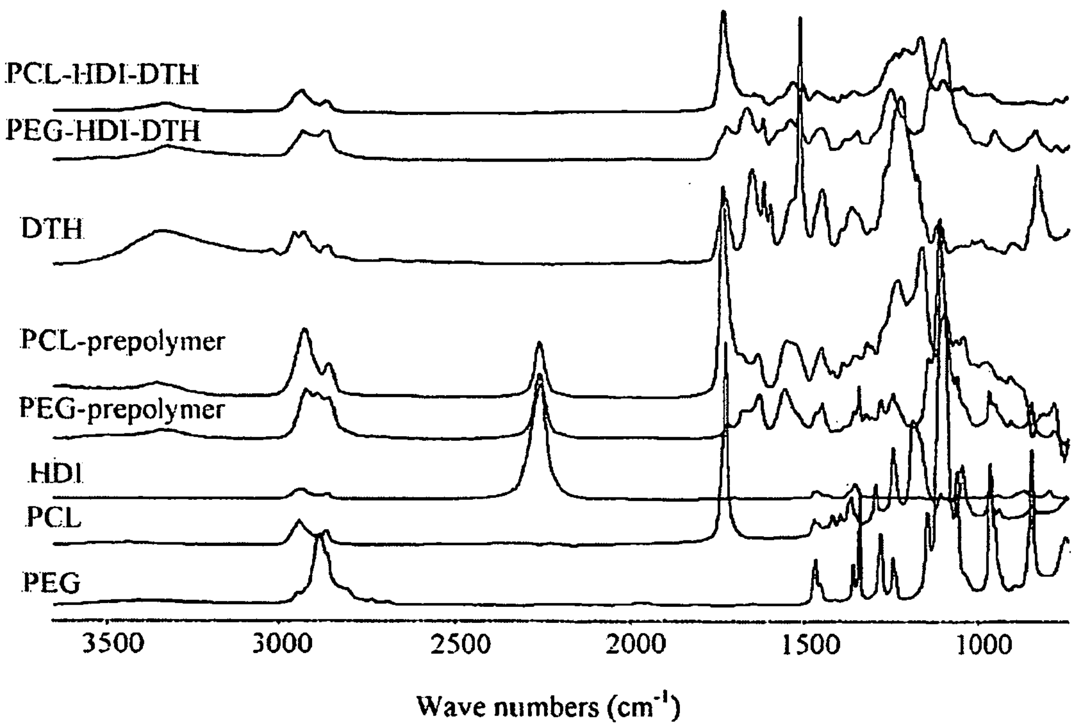
PCL-HDI-DTH
PEG-HDI-DTH
DTH
PCL-prepolymer
PEG-prepolymer
HDI
PCL
PEG
3500
3000
2500
2000
1500
1000
Wave numbers (cm⁻¹)

FIG. 56

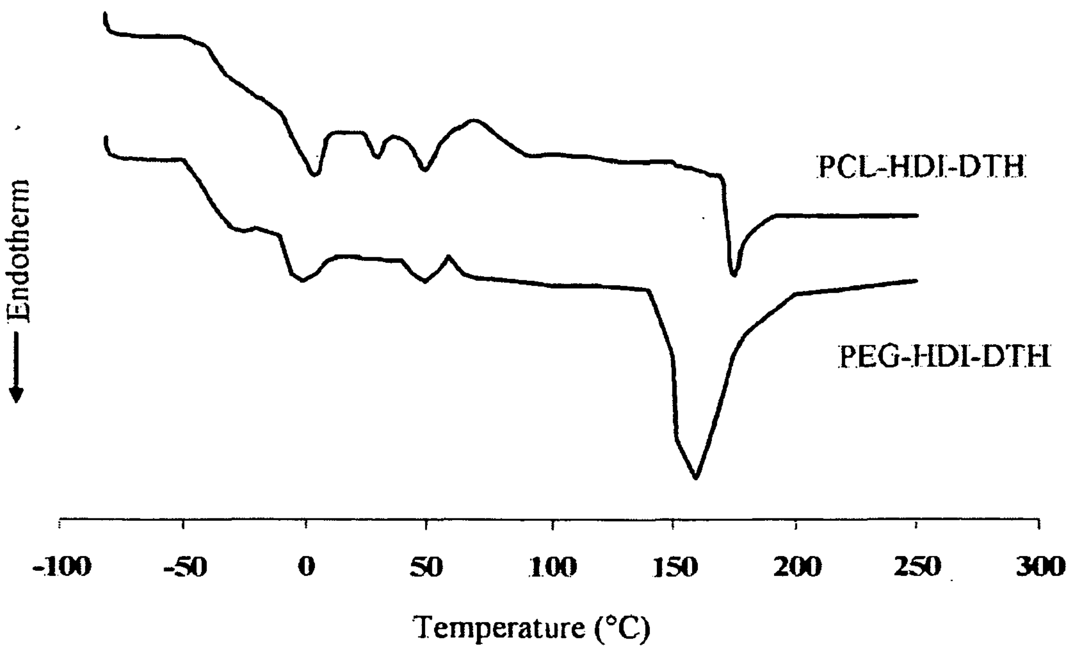
Endotherm
PCL-HDI-DTH
PEG-HDI-DTH
-100
-50
0
50
100
150
200
250
300
Temperature (°C)

FIG. 57

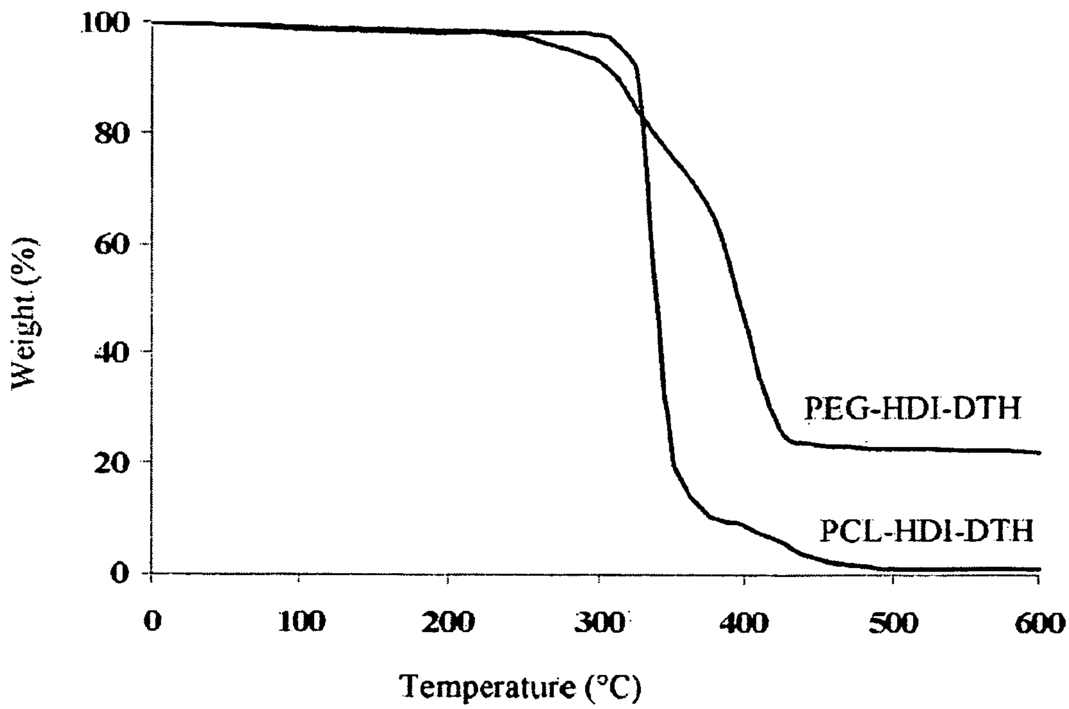
100
80
60
40
20
0
Weight (%)
0
100
200
300
400
500
600
Temperature (°C)
PEG-HDI-DTH
PCL-HDI-DTH

FIG. 58

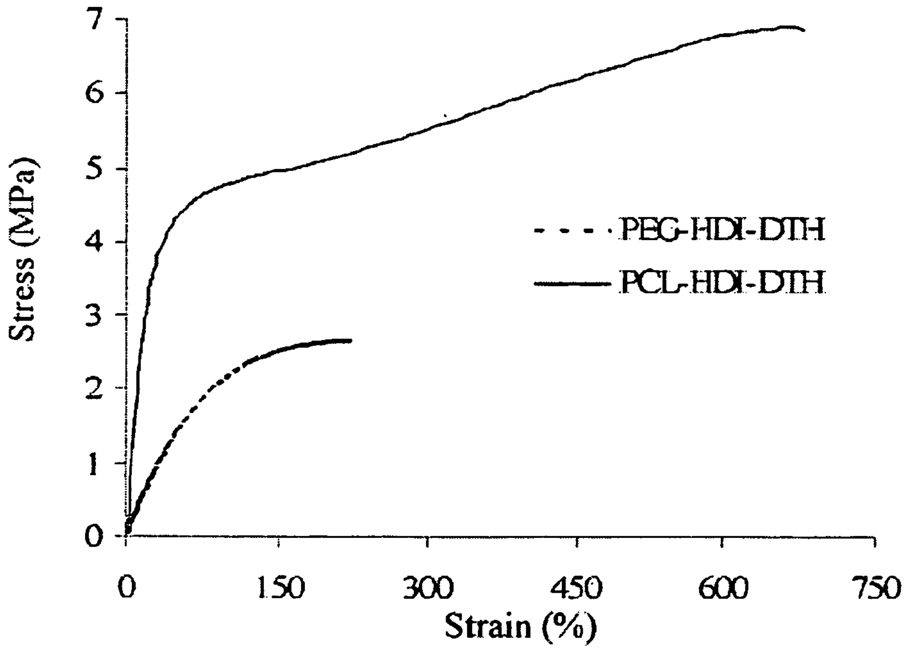
7
6
5
4
3
2
1
0
Stress (MPa)
0
150
300
450
600
750
Strain (%)
PEG-HDI-DTH
PCL-HDI-DTH

FIG. 59

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050025820 A
- US 20020131995 A
- US 20050037075 A
- US 6221997 B